# FASCICULE DE BREVET EUROPEEN

(11) **EP 1 437 122 B1**
(45) Date de publication et mention de la délivrance du brevet: **03.03.2010**
(21) Numéro de dépôt: 03104983.6
(22) Date de dépôt: 26.12.2003
(51) Int. Cl.: A61K 8/49, A61Q 5/10

(54) **Composition de teinture des fibres kératiniques comprenant un colorant direct dihétéroylarylméthane et procédé de teinture la mettant en oeuvre**
Einen Diheteroarylmethan-Direkfarbstoff enthaltende Zusammensetzung zur Färbung von Keratinfasern und Färbeverfahren mit dieser Zusammensetzung
Composition for dyeing keratinic fibers comprising diheteroarylmethane direct dye and dyeing process using this composition

(30) Priorité: 30.12.2002 FR 0216845
(43) Date de publication de la demande: 14.07.2004
(73) Titulaire: L'Oréal, 75008 Paris (FR)
(72) Inventeur: Guerin, Frédéric, 75009 Paris (FR); Lagrange, Alain, 77700 Coupvray (FR)
(74) Mandataire: Poulin, Gérard

(56) Documents cités:
- DE-B- 1 248 193
- GB-A- 876 663
- US-A- 3 685 956
- DATABASE CAPLUS AMERICAN CHEMICAL SOCIETY; 1999, XP002257995 accession no. STN Database accession no. 1999:757674 & KH.S. SHIKHAKIEV ET AL: IZVESTIYA VYSSHIKH UCHEBNYKH ZAVEDENII, KHIMIYA I KHIMICHESKAYA TEKHNOLOGIYA, vol. 42, no. 4, 1999, pages 83-87,
- DATABASE CAPLUS AMERICAN CHEMICAL SOCIETY; 1985, XP002257996 accession no. STN Database accession no. 1985:36721 & JP 59 162553 A (ASAHI CHEMICAL INDUSTRY CO LTD) 13 septembre 1984 (1984-09-13)

## Description

La présente invention concerne une composition de teinture des fibres kératiniques, en particulier des fibres kératiniques humaines et, plus particulièrement, des cheveux, qui comprend au moins un composé choisi parmi les colorants directs de type hétéroylarylméthane, plus précisément dihétéroylarylméthane et les précurseurs leuco de ceux-ci.

L'invention concerne, en outre, un procédé de teinture des fibres kératiniques, en particulier des fibres kératiniques humaines et, plus particulièrement, des cheveux qui met en oeuvre ladite composition.

Il existe de nombreuses compositions et de nombreux procédés pour teindre les fibres kératiniques et, en particulier, les cheveux humains.

Ainsi, il est connu de teindre les fibres kératiniques et, en particulier, les cheveux humains avec des compositions tinctoriales contenant des précurseurs de colorant d'oxydation, en particulier des ortho ou paraphénylènediamines, des ortho ou paraaminophénols, des composés hétérocycliques, tels que des dérivés de diaminopyrazole, appelés généralement « bases d'oxydation ». Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu, d'une part, au niveau des bases d'oxydation et, d'autre part, au niveau des coupleurs, permet l'obtention d'une riche palette de couleurs.

La coloration, dite « permanente », obtenue grâce à ces colorants d'oxydation, doit, par ailleurs, satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, l'ondulation permanente, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles, tout au long d'une même fibre kératinique, qui peut être en effet différemment sensibilisée, c'est-à-dire abîmée entre sa pointe et sa racine.

Il s'avère, en conclusion, que si les associations base-coupleur classiques permettent d'obtenir une palette étendue de couleurs, elles ne permettent pas de satisfaire aux critères énumérés plus haut et, notamment, conduisent souvent à la génération dans la fibre de produits de couplage variés et mal définis, ce qui pose des problèmes d'innocuité et/ou des problèmes de ténacité difficiles à maîtriser, comme par exemple un virage sélectif.

Une autre manière de teindre les fibres kératiniques, en particulier les cheveux, est d'utiliser des colorants directs.

Les colorants classiques qui sont utilisés sont en particulier des colorants du type nitrés benzèniques, anthraquinoniques, nitropyridiniques, azoïques, azoïques cationiques, xanthéniques, acridiniques aziniques, triarylméthanes, benzéniques nitrés ou des colorants naturels.

Ces colorants qui sont des molécules colorées et colorantes ayant une affinité pour les fibres sont appliqués sur les fibres kératiniques pendant un temps nécessaire à l'obtention de la coloration désirée, puis rincés.

L'utilisation des colorants directs est de ce fait très répandue car ils présentent, en outre, certains avantages par rapport aux précurseurs de colorants d'oxydation et, notamment, une diminution des risques potentiels d'allergie et l'absence de sensibilisation du cheveu due au processus oxydatif.

Les colorations obtenues sont cependant temporaires ou semi-permanentes, car la nature des interactions qui lient les colorants directs à la fibre kératinique, et leur désorption de la surface et/ou du coeur de la fibre sont responsables de leur faible puissance tinctoriale et de leur mauvaise tenue aux lavages, aux intempéries, ou à la transpiration. Ces colorants directs sont en outre généralement sensibles à la lumière du fait de la faible résistance du chromophore vis-à-vis des attaques photochimiques et conduisent dans le temps à un affadissement de la coloration des cheveux.

Il existe donc un besoin pour une composition de teinture des fibres kératiniques, en particulier des fibres kératiniques humaines et, plus particulièrement, des cheveux, qui soit d'une innocuité, d'une compatibilité, totale, vis-à-vis des fibres kératiniques, qui soit peu sélective, qui donne une grande variété de couleurs, puissantes, et qui permette, en outre, d'obtenir une coloration des fibres kératiniques tenace, stable, résistante aux agents extérieurs, tels que la lumière, les intempéries, le lavage, la transpiration, et les frottements et aux traitements ultérieurs, tels que l'ondulation permanente.

Il existe encore un besoin pour une composition de teinture qui permette le traitement de toutes sortes de fibres kératiniques, de tous types de cheveux, par exemple des cheveux blancs, même si ces cheveux ont subi préalablement un traitement, tel qu'un traitement de décoloration ou d'ondulation permanente. Il existe enfin un besoin pour une composition qui réalise la teinture sans qu'il soit nécessaire de réaliser, au préalable, une sensibilisation de la fibre kératinique, du cheveu.

Le but de la présente invention est de fournir une composition de teinture des fibres kératiniques, en particulier des fibres kératiniques humaines et, plus particulièrement, des cheveux qui réponde, entre autres, aux besoins énumérés ci-dessus.

Le but de la présente invention est encore de fournir une composition de teinture des fibres kératiniques qui ne présente pas les inconvénients, défauts, limitations et désavantages des compositions de teinture de l'art antérieur, qu'il s'agisse des compositions de teinture mettant en oeuvre une association base-coupleur, ou des compositions mettant en oeuvre un colorant direct.

Ce but et d'autres encore sont atteints, conformément à l'invention, par une composition pour la teinture des fibres kératiniques comprenant dans un milieu cosmétique approprié pour la teinture, au moins un colorant choisi parmi les composés répondant à la formule (I), (Ibis), (Iter), (II) ou (IIbis) suivante ou leurs formes tautomères, et leurs sels d'addition : 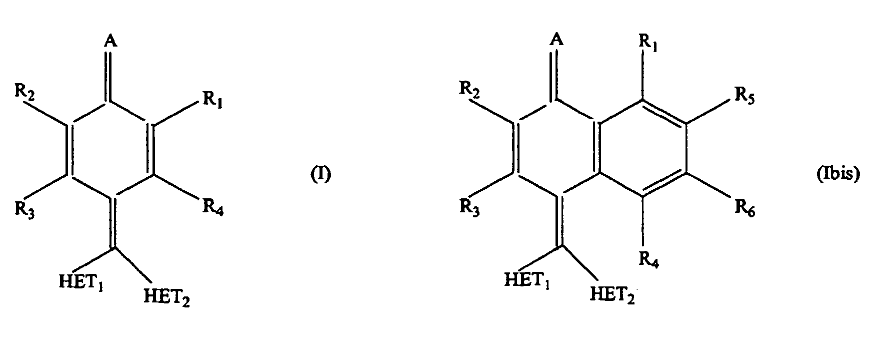 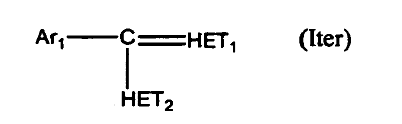 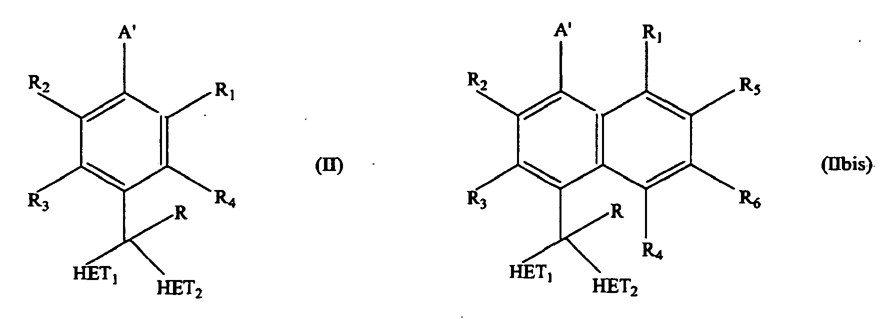 dans lesquelles :
- A représente O ; NH ; N-alkyle ; N-hydroxyalkyle ; ammonium ; N-alkylammonium ; N-(hydroxyalkyl)ammonium ; N,N-dialkylammonium, N,N-di(hydroxyalkyl)ammonium, N-(hydroxyalkyl) N-(alkyl) ammonium, dans lesquels les deux groupements alkyle peuvent former avec l'atome d'azote auquel ils sont liés, un cycle pouvant être interrompu par un ou plusieurs atomes d'azote, d'oxygène ou de soufre ;
- Ar₁ représente un radical aryle tel que phényle ou napthyle éventuellement substitué par un ou plusieurs groupements Z ;
- HET ₁, HET₂ représentent indépendamment l'un de l'autre un hétérocycle aromatique, éventuellement substitué par un ou plusieurs groupements Z' ;
- R₁ à R₆ et Z, Z' désignent indépendamment l'un de l'autre un groupement choisi parmi l'atome d'hydrogène, les atomes d'halogène, tels que F, Cl, Br et I, les radicaux -NHSO₃H ; hydroxyle ; alkyle ; alcoxy ; alkylthio ; amino, monoalkylamino ; dialkylamino, dans lesquels les deux groupements alkyle peuvent conjointement, avec l'atome d'azote, auquel ils sont liés, former un cycle pouvant être interrompu par un ou plusieurs atomes d'azote, d'oxygène ou de soufre ; les hétérocycles et les radicaux nitro ; aryle ; acyle ; alcoxycarbonyle ; carboxamido ; cyano ; -CO₂H ; -SO₃H ; -PO₃H₂ ; -PO₄H₂ ;
- A' représente un atome d'hydrogène, un groupe hydroxyle, amino, (hydroxyalkyl)amino, di(hydroxyalkyl)amino, (hydroxyalkyl)(alkyl)amino, monoalkylamino, dialkylamino, dans lesquels les deux groupements alkyle peuvent former avec l'atome d'azote auquel ils sont liés, un cycle pouvant être interrompu par un ou plusieurs atomes d'azote, d'oxygène ou de soufre ;
- R représente un atome d'hydrogène ou d'halogène ; un radical hydroxyle ; un radical alcoxy ; un radical alkylthio.

Dans les formules (I), (Ibis), (Iter), (II) et (IIbis) ci-dessus, le terme alkyle utilisé pour les radicaux alkyle, ainsi que pour les groupements comportant une partie alkyle, signifie, sauf indication différente, une chaîne carbonée, linéaire ou ramifiée, comportant de 1 à 30 atomes de carbone, de préférence de 1 à 8, mieux de 1 à 4, pouvant être portée et/ou interrompue par un ou plusieurs atomes d'oxygène de soufre ou d'azote, pouvant être substituée par un ou plusieurs groupes choisis parmi les atomes d'halogène, tel le chlore, le brome, l'iode et le fluor ; les hétérocycles ; les radicaux aryle ; hydroxyle ; alcoxy ; amino ; acyle ; carboxamido ; -CO₂H ; -SO₃H ; -PO₃H₂ ; -PO₄H₂ ; -NHSO₃H ; sulfonamide ; monoalkylamino ; trialkylammonium ; ou bien encore par un radical dialkylamino, dans lequel les deux groupements alkyle peuvent former, conjointement avec l'atome d'azote dudit groupement dialkyl(C₁-C₄)amino, auquel ils sont liés, un cycle pouvant être interrompu par un ou plusieurs atomes d'azote, d'oxygène ou de soufre.

De même, selon l'invention, le terme alcoxy utilisé pour les radicaux alcoxy ainsi que pour les groupements comportant une partie alcoxy, signifie, sauf indication différente, une chaîne O-alkyle, le terme alkyle ayant la signification indiquée ci-dessus. Les radicaux alcoxy des groupes alcoxycarbonyle ont, de préférence, de 1 à 4 atomes de carbone. Les groupes acyle ont, de préférence, de 2 à 4 atomes de carbone.

Selon l'invention, on entend par hétérocycle, un cycle aromatique contenant 5, 6 ou 7 sommets, et de 1 à 3 hétéroatomes choisis parmi les atomes d'azote, de soufre et d'oxygène. Ces hétérocycles peuvent être condensés sur d'autres hétérocycles ou sur d'autres cycles, notamment aromatiques, tels qu'un groupement phényle. Ces hétérocycles peuvent, en outre, être quaternisés par un radical alkyle. Les termes alkyle et alcoxy ont les significations indiqués ci-dessus.

Selon une forme de réalisation avantageuse, les groupes HET₁ et HET₂ représente nt un hétérocycle insaturé, aromatique éventuellement substitué par un ou plusieurs groupements Z'.

Parmi les hétérocycles, et notamment ceux de HET₁ et HET₂, on peut notamment citer à titre d'exemple les cycles : thiophène, benzothiophène, furane, benzofurane, indole, indoline, carbazole, pyridine, déhydroquinoléine, chromone, julolidine, thiadiazole, triazole, isoxazole, oxazole, thiazole, isothiazole, imidazole, pyrazole, triazine, thiazine, pyrazine, pyridazine, pyrimidine, pyridine, diazépine, oxazépine, benzotriazole, benzoxazole, benzimidazole, benzothiazole, morpholine, pipéridine, pipérazine, azétidine, pyrrolidine, aziridine.

Selon l'invention, on entend par aryle, sauf spécifié autrement, un radical aryle en C₆ à C₃₀ pouvant être substitué par un ou plusieurs radicaux alkyle ; alcoxy ; acyle ; cyano ; carboxamido ; -CO₂H ; -SO₃H ; -PO₃H₂ ; -PO₄H₂ ; hydroxyle ; amino ; monoalkyl(C₁-C₄)amino ; ou dialkyl(C₁-C₄)amino ; dans lequel les deux groupements alkyle peuvent former, conjointement avec l'atome d'azote dudit groupement dialkyl(C₁-C₄)amino, auquel ils sont liés, un cycle pouvant être interrompu par un ou plusieurs atomes d'azote, d'oxygène ou de soufre. De préférence, le groupe aryle est un groupe phényle ou un groupe naphtyle pouvant être substitué comme indiqué ci-dessus.

Dans la formule (Iter) de préférence Ar₁ est un radical phényle, de préférence encore non substitué, c'est-à-dire que, de préférence, Z sur ledit phényle est H.

Les composés (I), (Ibis) ou (Iter) peuvent être définis comme étant des colorants directs du type dihétéroylarylméthane et les composés de formules (II) ou (IIbis) sont les précurseurs leuco des dihétéroylarylméthane de formule (I), (Ibis) ou (Iter).

Les composés leuco (II) ou (IIbis) sont généralement peu ou pas colorés et peuvent être transformés par simple oxydation à l'air ou en présence d'un agent oxydant en un composé dihétéroylarylméthane de formule (I), (Ibis) ou (Iter).

A titre de composés de formule (I), (Ibis), (Iter), (II) ou (IIbis) utilisables dans le cadre de la présente invention, on peut citer les composés suivants pour lesquels les contre ions sont ou non spécifiés, et leurs sels d'addition :
4-[Bis-(2-méthyl-1H-indol-3-yl)-méthylène]-cyclohexa-2,5-diènylidene)-diméthyl-ammonium
Chlorure de 1,2,2,4-Tétraméthyl-6-[phényl-(1,2,2,4-tétraméthyl-1,2,3,4-tétrahydro-quinolin-6-yl)-méthylène]-2,3,4,6-tétrahydroquinolinium
Chlorure de 1,2,2,3-Tétraméthyl-5-[(1-méthyl-2-phényl-1H-indol-3-yl)-phényl-méthylène]-3,5-dihydro-2H-indolinum
Chlorure de 9-Éthyl-3-[(9-éthyl-9H-carbazol-3-yl)-phényl-méthylène]-3H-carbazolium
1-Éthyl-3-[(1-éthyl-2-méthyl-1H-indol-3-yl)-p-tolyl-méthylène]-2-méthyl-3a,7a-dihydro-3H-indolium
6-{(4-Nitro-phényl)-[1-(4-sulfo-butyl)-1,2,3,4-tétrahydro-quinolin-6-yl]-méthylèn}-1-(4-sulfo-butyl)-2,3,4,6-tétrahydro-quinolinium
6-{(4-Cyano-phényl)-[1-(4-sulfo-butyl)-1,2,3,4-tétrahydro-quinolin-6-yl]-méthylèn}-1-(4-sulfo-butyl)-2,3,4,6-tétrahydro-quinolinium
Diéthyl-{4-[(2-méthyl-1H-indol-3-yl)-thiophen-2-yl-méthylène]-cyclohexa-2,5-diènylidene}-ammonium
1-Éthyl-3-[(1-éthyl-2-méthyl-1H-indol-3-yl)-(4-méthoxy-phényl)-méthylène]-2-méthyl-3a,7a-dihydro-3H-indolium
Éthanaminium, N-[4-[(3-carboxypyrazinyl)(1-éthyl-2-méthyl-1H-indol-3-yl)méthylène]-3-éthoxy-2,5-cyclohexadièn-1-ylidene]-N-éthyl-, sel interne
Éthanaminium, N-[4-[(3-carboxypyrazinyl)(1-éthyl-2-méthyl-1H-indol-3-yl)méthylène]-3-éthoxy-2,5-cyclohexadièn-1-ylidene]-N-éthyl-, chlorure
Cyclohepta[b]furylium, 3-[[4-(diméthylamino)phényl](2-oxo-2H-cyclohepta[b]furan-3-yl)méthylène]-2,3-dihydro-2-oxo
Méthanaminium, N-[4-[bis(1,2,3,4,10,14b-hexahydro-2-méthyldibenz[c,f]pyrazino[1,2-a]azepin-8-yl) méthylène]-2,5-cyclohexadièn-1-ylidene]-N-méthyl-, perchlorate, diperchlorate
Méthanaminium, N-[4-[bis(1,2,3,4,10,14b-hexahydro-2-méthyldibenzo [c,f]pyrazino[1,2-a]azepin-8-yl)méthylène]-2,5-cyclohexadièn-1-ylidene]-N-méthyl-, perchlorate
Méthanaminium, N-[4- [bis (1,2,3,4,10,14b-hexahydro-2-méthyldibenzo [c,f]pyrazino[1,2-a]azepin-8-yl)méthylène]-2,5-cyclohexadièn-1-ylidene]-N-méthyl-
Méthanaminium, N-[4-[bis(2,3-dihydro-1,5-diméthyl-3-oxo-2-phényl-1H-pyrazol-4-yl)méthylène]-2,5-cyclohexadièn-1-ylidene]-N-méthyl-
Morpholinium, 4-[5-[[4-(diméthylamino)phényl][5-(4-morpholinyl)-2-thiényl]méthylène]-2(5H)-thiénylidene]-, perchlorate
Morpholinium, 4-[5-[[4-(diméthylamino)phényl][5-(4-morpholinyl)-2-thiényl]méthylène]-2(5H)-thiénylidène]-
1H-Indolizinium, 7-(diméthylamino)-1-[[7-(diméthylamino)-3- (éthoxycarbonyl)-1-indolizinyl][4-(diméthylamino)phényl]méthylène]-3- (éthoxycarbonyl)-, perchlorate
1H-Indolizinium, 7-(diméthylamino)-1-[[7-(diméthylamino)-3-(éthoxycarbonyl)-1-indolizinyl][4-(diméthylamino)phényl]méthylène]-3- (éthoxycarbonyl)-
Cyclohepta[b]pyrrolium, 3-[(1,2-dihydro-2-oxo-1-phénylcyclohepta[b]pyrrol-3-yl)[4-(diméthylamino)phényl]méthylène]-2,3-dihydro-2-oxo-1-phényl-, hexafluorophosphate
Cyclohepta [b] pyrrolium, 3-[(1,2-dihydro-2-oxo-1-phénylcyclohepta[b]pyrrol-3-yl)[4-(diméthylamino)phényl]méthylène]-2,3-dihydro-2-oxo-1-phényl-
Quinolinium, 6-[[4-(diméthylamino)phényl](1,2,3,4-tétrahydro-1,2,2,4-tétraméthyl-6-quinolinyl)méthylène]-2,3,4,6-tétrahydro-1,2,2,4-tétraméthyl-, chlorure
Quinolinium, 6-[(1,2-dihydro-1,2,2,4-tétraméthyl-6-quinolinyl)[4-(diméthylamino)phényl]méthylène]-2,6-dihydro-1,2,2,4-tétraméthyl-,chlorure
3H-Carbazolium, 9-éthyl-3-[(9-éthyl-9H-carbazol-3-yl)[4-(méthylphénylamino)phényl]méthylène]-, tétrafluoroborate(1-)
3H-Carbazolium, 3-[[4-(diméthylamino)phényl](9-éthyl-9H-carbazol-3-yl)méthylène]-9-éthyl-, tétrafluoroborate(1-)
3H-Carbazolium, 9-éthyl-3-[(9-éthyl-9H-carbazol-3-yl)[4- (méthylphénylamino) phényl]méthylène]-, perchlorate
3H-Carbazolium, 9-éthyl-3-[(9-éthyl-9H-carbazol-3-yl)[4- (méthylphénylamino) phényl]méthylène]-, bromure
3H-Carbazolium, 9-éthyl-3-[(9-éthyl-9H-carbazol-3-yl)[4- (méthylphénylamino) phényl]méthylène]-, chlorure
3H-Carbazolium, 3-[[4-(diméthylamino)phényl] (9-éthyl-9H-carbazol-3-yl)méthylène]-9-éthyl-, bromure
3H-Carbazolium, 3-[[4-(diméthylamino)phényl](9-éthyl-9H-carbazol-3-yl)méthylène]-9-éthyl-, chlorure
3H-Carbazolium, 9-éthyl-3-[(9-éthyl-9H-carbazol-3-yl)[4- (méthylphénylamino) phényl]méthylène]-, iodure
3H-Carbazolium, 3-[[4-(diméthylamino)phényl](9-éthyl-9H-carbazol-3-yl)méthylène]-9-éthyl-, iodure
3H-Carbazolium, 3-[[4-(diméthylamino)phényl](9-éthyl-9H-carbazol-3-yl)méthylène]-9-éthyl-, perchlorate
1H-Pyrazolium, 4-[(2,3-dihydro-1,5-diméthyl-3-oxo-2-phényl-1H-pyrazol-4-yl)[4-(triméthylammonio) phényl]méthylène]-4,5-dihydro-2,3-diméthyl-5-oxo-1-phényl-, diperchlorate
1H-Pyrazolium, 4-[(2,3-dihydro-1,5-diméthyl-3-oxo-2-phényl-1H-pyrazol-4-yl)[4-(triméthylammonio)phényl]méthylène]-4,5-dihydro-2,3-diméthyl-5-oxo-1-phényl-
5H-Benzo[a]carbazolium, 11-éthyl-5-[(11-éthyl-3-méthoxy-8-méthyl-11H-benzo[a] carbazol-5-yl)(4-nitrophényl)méthylène]-3-méthoxy-8-méthyl-, tétrafluoroborate
5H-Benzo[a]carbazolium, 11-éthyl-5-[(11-éthyl-3-méthoxy-8-méthyl-11H-benzo[a] carbazol-5-yl)(4-nitrophényl)méthylène]-3-méthoxy-8-méthyl-
5H-Benzo[a]carbazolium, 11-éthyl-5-[(11-éthyl-2-méthoxy-8-méthyl-11H-benzo[a]carbazol-5-yl)(4-nitrophényl)méthylène]-2-méthoxy-8-méthyl-, tétrafluoroborate
5H-Benzo[a]carbazolium, 11-éthyl-5-[(11-éthyl-2-méthoxy-8-méthyl-11H-benzo[a]carbazol-5-yl)(4-nitrophényl)méthylène]-2-méthoxy-8-méthyl-
5H-Benzo[a]carbazolium, 11-éthyl-5-[(11-éthyl-8-méthoxy-11H-benzo[a]carbazol-5-yl)(4-nitrophényl)méthylène]-8-méthoxy-,tétrafluoroborate
5H-Benzo[a]carbazolium, 11-éthyl-5-[(11-éthyl-8-méthoxy-11H-benzo[a]carbazol-5-yl)(4-nitrophényl)methylène]-8-méthoxy-
5H-Benzo[a]carbazolium, 11-éthyl-5-[(11-éthyl-4-méthoxy-11H-benzo[a]carbazol-5-yl)(4-nitrophényl)méthylène]-4-méthoxy-,tétrafluoroborate(
5H-Benzo[a]carbazolium, 11-éthyl-5-[(11-éthyl-4-méthoxy-11H-benzo[a]carbazol-5-yl)(4-nitrophényl)méthylène]-4-méthoxy-
5H-Benzo[a]carbazolium, 11-éthyl-5-[(11-éthyl-3-méthoxy-11H-benzo[a]carbazol-5-yl)(4-nitrophényl)méthylène]-3-méthoxy-,tétrafluoroborate
5H-Benzo[a]carbazolium, 11-éthyl-5-[(11-éthyl-3-méthoxy-11H-benzo[a]carbazol-5-yl)(4-nitrophényl)méthylène]-3-méthoxy-
5H-Benzo[a]carbazolium, 11-éthyl-5-[(11-éthyl-2-méthoxy-11H-benzo[a]carbazol-5-yl)(4-nitrophényl)méthylène]-2-méthoxy-,tétrafluoroborate
5H-Benzo[a]carbazolium, 11-éthyl-5-[(11-éthyl-2-méthoxy-11H-benzo[a]carbazol-5-yl)(4-nitrophényl)méthylène]-2-méthoxy-
5H-Benzo[a]carbazolium, 5-[(2-chloro-4-nitrophényl)(11-éthyl-11H-benzo[a]carbazol-5-yl)méthylène]-11-éthyl-, sel avec l'acide trifluorométhanesulfonique(1:1)
5H-Benzo[a]carbazolium, 5-[(2-chloro-4-nitrophényl)(11-éthyl-11H-benzo[a]carbazol-5-yl)méthylène]-11-éthyl
5H-Benzo[a]carbazolium, 11-éthyl-5-[(11-éthyl-11H-benzo[a]carbazol-5-yl)(4-nitrophényl)méthylène]-, tétrafluoroborate
5H-Benzo[a]carbazolium, 11-éthyl-5-[(11-éthyl-11H-benzo[a]carbazol-5-yl)(4-nitrophényl)méthylène]-
5H-Benzo[a]carbazolium, 5-[[4-(diéthylamino)phényl](11-éthyl-11H-benzo[a]carbazol-5-yl)méthylène]-11-éthyl-, tétrafluoroborate(1-)
5H-Benzo[a] carbazolium, 5-[[4-(diéthylamino)phényl](11-éthyl-11H-benzo[a]carbazol-5-yl)méthylène]-11-éthyl-
5H-Benzo[a]carbazolium, 5-[[4-(diméthylamino)phényl](11-éthyl-11H-benzo[a]carbazol-5-yl)méthylène]-11-éthyl-, tétrafluoroborate(1-)
5H-Benzo[a]carbazolium, 5-[[4-(diméthylamino)phényl](11-éthyl-11H-benzo[a]carbazol-5-yl)méthylène]-11-éthyl-
Sel de 1H-Pyrazolium, 4-[(2,3-dihydro-1,5-diméthyl-3-oxo-2-phényl-1H-pyrazol-4-yl)[4-(diméthylamino)phényl]méthylène]-4,5-dihydro-2,3-diméthyl-5-oxo-1-phényl-et d'un monoacide
3H-Carbazolium, 3-[[4-[bis(phénylméthyl)amino]phényl](9-éthyl-9H-carbazol-3-yl)méthylène]-9-éthyl-, iodure
3H-Imidazo[1,2-a]benzimidazolium, 3-[[4-(diméthylamino)phényl](9-méthyl-2-phényl-9H-imidazo[1,2-a]benzimidazol-3-yl)méthylène]-9-méthyl-2-phényl-, chlorure
3H-Imidazo[1,2-a]benzimidazolium, 3-[[4-(diméthylamino)phényl](2,9-diméthyl-9H-imidazo[1,2-a]benzimidazol-3-yl)méthylène]-2,9-diméthyl-,bromure
1H-Pyrazolium, 4-[(2,3-dihydro-1,5-diméthyl-3-oxo-2-phényl-1H-pyrazol-4-yl)[4-(dipropylamino)phényl]méthylène]-4,5-dihydro-2,3-diméthyl-5-oxo-1-phényl-
1H-Pyrazolium, 4-[[4-(diéthylamino)phényl](2,3-dihydro-1,5-diméthyl-3-oxo-2-phényl-1H-pyrazol-4-yl)méthylène]-4,5-dihydro-2,3-diméthyl-5-oxo-1-phényl-
1H-Pyrazolium, 4-[(2,3-dihydro-1,5-diméthyl-3-oxo-2-phényl-1H-pyrazol-4-yl)(4-nitrophényl)méthylène]-4,5-dihydro-2,3-diméthyl-5-oxo-1-phényl-
1H-Pyrazolium, 4-[(2,3-dihydro-1,5-diméthyl-3-oxo-2-phényl-1H-pyrazol-4-yl)[4-(diméthylamino)phényl]méthylène]-4,5-dihydro-2,3-diméthyl-5-oxo-1-phényl-
1H-Pyrazolium, 4-[(4-aminophényl)(2,3-dihydro-1,5-diméthyl-3-oxo-2-phényl-1H-pyrazol-4-yl)méthylène]-2,3-diméthy1-5-oxo-1-phényl
3H-Indolizinium, 3-[[4-(diméthylamino)phényl](6-éthenyl-1,2-diméthyl-3-indolizinyl)méthylène]-6-éthenyl-1,2-diméthyl-
3H-Indolizinium, 3-[p-(diméthylamino)-α-(6-éthyl-1,2-diméthyl-3-indolizinyl)benzylidene]-6-éthyl-1,2-diméthyl-, iodure
3H-Indolizinium, 3-[p-(diméthylamino)-α-(1,2-diméthyl-6-vinyl-3-indolizinyl)benzylidene]-1,2-diméthyl-6-vinyl-, perchlorate
2-Pyrazolinium, 4-(p-amino-α-antipyrinylbenzylidene)-2,3-diméthyl-5-oxo-1-phényl-, perchlorate
2-Pyrazolinium, 4-(α-antipyrinyl-p-nitrobenzylidene)-2,3-diméthyl-5-oxo-1-phényl-, perchlorate
2-Pyrazolinium, 4-[α-antipyrinyl-p-(diéthylamino)benzylidene]-2,3-diméthyl-5-oxo-1-phényl-, perchlorate
2-Pyrazolinium, 4-[α-antipyrinyl-p-(diméthylamino)benzylidene]-2,3-diméthyl-5-oxo-1-phényl-, perchlorate
Méthylium, bis [6-(diméthylamino)-3-méthyl-1H-indol-2-yl][4-(diméthylamino)phényl]-, perchlorate
Méthylium, bis[6-(diméthylamino)-3-méthyl-1H-indol-2-yl][4-(diméthylamino)phényl]-
Méthylium, bis[6-(diméthylamino)-3-méthyl-1H-indol-2-yl](4-nitrophényl)-, perchlorate
Méthylium, bis[6-(diméthylamino)-3-méthyl-1H-indol-2-yl](4-nitrophényl)-
Méthylium, bis(benzo[b]thién-2-yl)[4-(diméthylamino)phényl]-, tétrafluoroborate
Méthylium, bis(benzo[b]thién-2-yl)[4-(diméthylamino)phényl]-
Méthylium, [4-(diméthylamino)phényl]bis(1-méthyl-1H-pyrrol-2-yl)-, tétrafluoroborate
Méthylium, [4-(diméthylamino)phényl]bis(1-méthyl-1H-pyrrol-2-yl)-Diantipyrinyl(p-nitrophényl)méthylium perchlorate
Méthylium, bis(9,10-dihydro-9,9,10-triméthyl-3-acridinyl)[4-(diméthylamino)phényl]-, chlorure
Méthylium, [4-(diméthylamino)phényl]bis(10-méthyl-10H-phenothiazin-2-yl)-, chlorure
3H-Pyrazol-3-one, 4,4'-[[4-(diméthylamino)phényl]méthylène]bis[1,2-dihydro-1,5-diméthyl-2-phényl-, mono[tétrafluoroborate(1-)]
3H-Pyrazol-3-one, 4,4'-[[4-(diméthylamino)phényl]méthylène]bis[1,2-dihydro-1,5-diméthyl-2-phényl-, bis [tétrafluoroborate(1-)]
Sel de 3H-Pyrazol-3-one, 4,4'-[[4-(diméthylamino)phényl]méthylène]bis[1,2-dihydro-1,5-diméthyl-2-phényl-et d'un diacide
Sel de 3H-Pyrazol-3-one, 4,4'-[[4-(diméthylamino)phényl]méthylène]bis[1,2-dihydro-1,5-diméthyl-2-phényl-,et d'un monoacide
Quinolinium, 4,4'-[(4-nitrophényl)méthylène]bis[1-éthyl-, dichlorure
Méthylium, bis(9-éthyl-9H-carbazol-3-yl)(4-nitrophényl)-, iodure
Méthylium, [4-(diméthylamno)phényl]bis(9-éthyl-9H-carbazol-3-yl)-, iodure
Méthylium, 9H-carbazol-3-yl[4-(diméthylamino)phényl](6-méthyl-9H-carbazol-3-yl)-Quinolizinium, 2,2'-[(4-nitrophényl)méthylène]bis[1,2,3,4-tétrahydro-1-oxo-
Méthylium, 9H-carbazol-3-yl[4-(diméthylamino)phényl](6-méthyl-9H-carbazol-3-yl)-, tétrafluoroborate
(Borate(1-), tétrafluoro-, 9H-carbazol-3-yl[4-(diméthylamino)phényl](6-méthyl-9H-carbazol-3-yl)méthylium)
(6-Méthyl-3,3'-dicarbazolyl-p-diméthylaminophénylméthyl fluoroborate) 6,6'-Dichloro-9,9'-dibutyl-3,3'-dicarbazolyl-p-nitrophénylméthyl chlorure
Quinolizinium, 2,2'-(p-nitrobenzylidene)bis[1,2,3,4-tétrahydro-1-oxo-, diperchlorate
Benzylium, p-(diéthylamino)-α,α-bis(2,3-diméthyl-5-oxo-1-phényl-3-pyrazolin-4-yl)-
Benzylium, α,α-diantipyrinyl-p-(diméthylamino)-
Benzylium, α,α-bis(2,3-diméthyl-5-oxo-1-phényl-3-pyrazolin-4-yl)-p-nitro-
Benzylium, p-amino-α,α-di-4-antipyrinyl-.

Les dihétéroylarylméthanes de formule (I), (Ibis), ou (Iter), et les composés leuco de formule (II) ou (IIbis) sont des composés connus et utilisés pour la teinture des textiles comme décrit dans le document US-A- 3 685 956. Ces composés peuvent être préparés selon des procédés de synthèses bien connus dans la littérature et tels que décrits, par exemple, dans les documents DE-A-1 569 750, FR-A-2 188 202, US-A-3 995 088, US-A-4 154 463, US-A-4 355 823, US-A-5 094 688, US-A-5 266 699 et US-A-5 362 612.

Il s'avère que les compositions de l'invention permettent, de manière surprenante, d'obtenir des colorations intenses, même sur des cheveux non sensibilisés.

Les compositions selon l'invention permettent d'obtenir des reflets variés chromatiques ou sombres, très puissants, peu sélectifs et tenaces.

Ainsi, les colorants dihétéroylarylméthanes de formule (I), (Ibis) ou (Iter) ou les leucos de formule (II) ou (IIbis) inclus dans les compositions de l'invention permettent d'obtenir toutes les nuances du vert au bleu, en passant par les rouges et, en particulier, ils permettent d'obtenir aussi les nuances noires.

Les colorations obtenues avec les compositions de l'invention sont tenaces, stables, résistantes, vis-à-vis des intempéries, du lavage, de la transpiration, des frottements et des traitements ultérieurs, tels que l'ondulation permanente.

Ces colorations sont, en particulier, particulièrement tenaces à la lumière.

Le ou les composés de formule (I), (Ibis), (Iter), (II), (IIbis) ci -dessus et/ou leur ou leurs sel(s) d'addition représentent généralement de 0,0001 à 10 % en poids du poids total de la composition de teinture, de préférence de 0,005 à 10 % en poids et, de préférence encore, de 0,01 à 6 % de poids du poids total de la composition de teinture selon l'invention.

La composition tinctoriale conforme à l'invention peut en outre contenir un ou plusieurs colorant(s) direct(s) différent(s) des composés de formule (I), (Ibis), (Iter), (II) ou (IIbis). Le ou les colorant(s) direct (s) utile (s) selon l'invention sont par exemple choisis parmi les colorants directs nitrés benzéniques neutres, acides ou cationiques, les colorants directs azoïques neutres acides ou cationiques, les colorants directs quinoniques neutres, acides ou cationiques, les colorants directs aziniques, les colorants directs triarylméthaniques, les colorants directs indoaminiques et les colorants directs naturels.

Parmi les colorants directs benzéniques, on peut citer de manière non limitative les composés suivants :
- 1,4-diamino-2-nitrobenzène ;
- 1-amino-2 nitro-4-β- hydroxyéthylaminobenzène ;
- 1-amino-2 nitro-4-bis(β-hydroxyéthyl)-aminobenzène ;
- 1,4-Bis(β-hydroxyéthylamino)-2-nitrobenzène ;
- 1-β-hydroxyéthylamino-2-nitro-4-bis-(β-hydroxyéthylamino)-benzène ;
- 1-β-hydroxyéthylamino-2-nitro-4-aminobenzène ;
- 1-β-hydroxyéthylamino-2-nitro-4-(éthyl)(β-hydroxyéthyl)-aminobenzène ;
- 1-amino-3-méthyl-4-β-hydroxyéthylamino-6-nitrobenzène ;
- 1-amino-2-nitro-4-β-hydroxyéthylamino-5-chlorobenzène ;
- 1,2-Diamino-4-nitrobenzène ;
- 1-amino-2-β-hydroxyéthylamino-5-nitrobenzène ;
- 1,2-Bis-(β-hydroxyéthylamino)-4-nitrobenzène ;
- 1-amino-2-tris-(hydroxyméthyl)-méthylamino-5-nitrobenzène ;
- 1-Hydroxy-2-amino-5-nitrobenzène ;
- 1-Hydroxy-2-amino-4-nitrobenzène ;
- 1-Hydroxy-3-nitro-4-aminobenzène ;
- 1-Hydroxy-2-amino-4,6-dinitrobenzène ;
- 1-β-hydroxyéthyloxy-2-β-hydroxyéthylamino-5-nitrobenzène ;
- 1-Méthoxy-2-β-hydroxyéthylamino-5-nitrobenzène ;
- 1-β-hydroxyéthyloxy-3-méthylamino-4-nitrobenzène ;
- 1-β,γ-dihydroxypropyloxy-3-méthylamino-4-nitrobenzène ;
- 1-β-hydroxyéthylamino-4-β,γ-dihydroxypropyloxy-2-nitrobenzène ;
- 1-β,γ-dihydroxypropylamino-4-trifluorométhyl-2-nitrobenzène ;
- 1-β-hydroxyéthylamino-4-trifluorométhyl-2-nitrobenzène ;
- 1-β-hydroxyéthylamino-3-méthyl-2-nitrobenzène ;
- 1-β-aminoéthylamino-5-méthoxy-2-nitrobenzène ;
- 1-Hydroxy-2-chloro-6-éthylamino-4-nitrobenzène ;
- 1-Hydroxy-2-chloro-6-amino-4-nitrobenzène ;
- 1-Hydroxy-6-bis-(β-hydroxyéthyl)-amino-3-nitrobenzène ;
- 1-β-hydroxyéthylamino-2-nitrobenzène ;
- 1-Hydroxy-4-β-hydroxyéthylamino-3-nitrobenzène.

Parmi les colorants directs azoïques, on peut citer les colorants azoïques cationiques décrits dans les demandes de brevets WO 95/15144, WO-95/01772, EP-714954 et WO-A-01/66646 dont le contenu fait partie intégrante de l'invention.

Parmi ces composés, on peut tout particulièrement citer les colorants suivants :
- chlorure de 1,3-diméthyl-2-[[4-(diméthylamino)phényl]azo]-1H-Imidazolium ;
- chlorure de 1,3-diméthyl-2-[(4-aminophényl)azo]-1H-Imidazolium ;
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3^{e} édition :
- Disperse Red 17 ;
- Acid Yellow 9 ;
- Acid Black 1 ;
- Basic Red 22 ;
- Basic Red 76 ;
- Basic Yellow 57 ;
- Basic Brown 16 ;
- Acid Yellow 36 ;
- Acid Orange 7 ;
- Acid Red 33 ;
- Acid Red 35 ;
- Basic Brown 17 ;
- Acid Yellow 23 ;
- Acid Orange 24 ;
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques, on peut citer les colorants suivants :
- Disperse Red 15 ;
- Solvent Violet 13 ;
- Acid Violet 43 ;
- Disperse Violet 1 ;
- Disperse Violet 4 ;
- Disperse Blue 1 ;
- Disperse Violet 8 ;
- Disperse Blue 3 ;
- Disperse Red 11 ;
- Acid Blue 62 ;
- Disperse Blue 7 ;
- Basic Blue 22 ;
- Disperse Violet 15 ;
- Basic Blue 99,
ainsi que les composés suivants :
- 1-N-méthylmorpholiniumpropylamino-4-hydroxyanthraquinone ;
- 1-Aminopropylamino-4-méthylaminoanthraquinone ;
- 1-Aminopropylaminoanthraquinone ;
- 5-β-hydroxyéthyl-1,4-diaminoanthraquinone ;
- 2-Aminoéthylaminoanthraquinone ;
- 1,4-Bis-(β,γ-dihydroxypropylamino)-anthraquinone.

Parmi les colorants aziniques, on peut citer les composés suivants :
- Basic Blue 17 ;
- Basic Red 2.

Parmi les colorants triarylméthaniques, on peut citer les composés suivants :
- Basic Green 1 ;
- Acid blue 9 ;
- Basic Violet 3 ;
- Basic Violet 14 ;
- Basic Blue 7 ;
- Acid Violet 49 ;
- Basic Blue 26 ;
- Acid Blue 7.

Parmi les colorants indoaminiques, on peut citer les composés suivants :
- 2-β-hydroxyéthlyamino-5-[bis-(β-4'-hydroxyéthyl)amino]anilino-1,4-benzoquinone ;
- 2-β-hydroxyéthylamino-5-(2'-méthoxy-4'-amino)anilino-1,4-benzoquinone ;
- 3-N(2'-Chloro-4'-hydroxy)phényl-acétylamino-6-méthoxy-1,4-benzoquinone imine :
   - 3-N(3'-Chloro-4'-méthylamino)phényl-uréido-6-méthyl-1,4-benzoquinone imine ;
   - 3-[4'-N-(Ethyl,carbamylméthyl)-amino]-phényl-uréido-6-méthyl-1,4-benzoquinone imine.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs additionnels différents des composés de formules (I), (Ibis), (Iter), (II) et (IIbis) représentent de préférence de 0,001 à 20% en poids environ du poids total de la composition prête à l'emploi et encore plus préférentiellement de 0,005 à 10% en poids environ.

La composition de la présente invention peut de plus contenir une ou plusieurs base (s) d'oxydation et éventuellement un ou plusieurs coupleur(s) classiquement utilisés pour la coloration par oxydation.

A titre d'exemple de base d'oxydation, on peut citer les paraphénylènediamines, les bis-phénylalkylènediamines, les para-aminophénols, les ortho-aminophénols, les bases hétérocycliques et leurs sels d'addition.

Les coupleurs sont par exemple les coupleurs métaphénylènediamines, les coupleurs méta-aminophénols, les coupleurs métadiphénols, les coupleurs naphtaléniques, les coupleurs hétérocycliques et leurs sels d'addition.

Lorsqu'ils sont présents, le ou les base(s) d'oxydation et/ou le ou les coupleurs sont chacun généralement présents en une quantité comprise entre 0,001 et 10 % en poids environ du poids total de la composition tinctoriale, de préférence entre 0,005 et 6 %.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci dessus sont en général présents en quantité comprise pour chacun d'eux entre 0,01 et 20 % en poids par rapport au poids de la composition.

De préférence, les compositions contiennent un solvant choisi parmi les alcanols en C₂-C₄ et les polyols de poids moléculaire inférieur à 1 000.

De préférence, les compositions contiennent au moins un agent tensioactif et/ou au moins un agent épaississant minéral ou organique.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, de préférence entre 5 et 11 environ, de préférence encore de 6 à 8,5.

Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 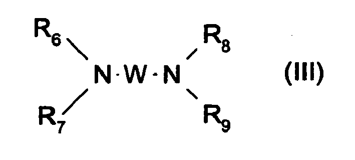 dans laquelle W est un reste propylène éventuellement substitué par un groupement hydroxyle ou un radical alkyle en C₁-C₄ ; R₆, R₇, R₈ et R₉, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a aussi pour objet un procédé de teinture directe des fibres kératiniques et, en particulier, des fibres kératiniques humaines, telles que les cheveux, qui comprend l'application d'au moins une composition tinctoriale contenant au moins un composé, colorant, de formule (I), (Ibis), (Iter), (II) ou (IIbis), telle que définie précédemment sur les fibres kératiniques. Après un temps de pause, les fibres kératiniques sont rincées laissant apparaître des fibres colorées. Le temps de pause est généralement compris entre 3 à 50 minutes environ, de préférence 5 à 30 minutes environ.

Rappelons que par coloration ou teinture directe, on entend une coloration effectuée sans agent oxydant autre que l'oxygène de l'air.

Lorsque la composition tinctoriale comprend soit au moins un composé de formule (II) ou (IIbis) soit au moins un composé de formule (I), (Ibis), ou (Iter) et au moins une base d'oxydation, et éventuellement un ou plusieurs coupleur(s), la composition tinctoriale contient de préférence un oxydant.

L'invention a donc également pour objet un procédé de teinture des fibres kératiniques et, en particulier, des fibres kératiniques humaines, telles que les cheveux, dans lequel on applique sur lesdites fibres au moins une composition de teinture comprenant soit au moins un composé de formule (II) ou (IIbis), soit au moins un composé de formule (I), (Ibis) ou (Iter) et au moins une base d'oxydation et éventuellement un ou plusieurs coupleurs, la couleur étant révélée à pH acide, neutre ou alcalin, à l'aide d'un agent oxydant.

Les agents oxydants utilisables sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

L'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention. La composition oxydante peut également renfermer divers adjuvants utilisés classiquement dans les compositions pour la teinture des cheveux et tels que définis précédemment. En cas de mélange avec la composition tinctoriale, le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11, mieux entre 6 et 8,5. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Un autre objet de l'invention est un dispositif à plusieurs compartiments ou « kit » de teinture, dans lequel un premier compartiment renferme une composition tinctoriale définie ci-dessus, comprenant soit au moins un composé de formule (II) ou (IIbis), soit au moins un composé de formule (I), (Ibis) ou (Iter) et au moins une base d'oxydation et éventuellement un ou plusieurs coupleur(s), et un deuxième compartiment renferme la composition oxydante.

Ce dispositif peut être équipé d'un moyen permettant de délivrer sur les cheveux le mélange souhaité, tel que les dispositifs décrits dans le brevet FR-2 586 913 au nom de la demanderesse.

Dans le cas d'une composition contenant au moins un composé de formule (II) ou (IIbis), il est souhaitable d'éviter le contact entre lesdits composés et l'oxygène de l'air. Un mode de conditionnement particulièrement adapté peut être alors un dispositif aérosol.

Avec les colorants de l'invention, on peut également réaliser de la coloration directe éclaircissante en l'absence de colorants d'oxydation et en présence d'un agent oxydant dans des conditions telles (par exemple peroxyde d'hydrogène en milieu alcalin) que celui-ci soit apte à éclaircir les fibres kératiniques par action sur les pigments initialement présents dans lesdites fibres.

Les exemples qui suivent sont destinés à illustrer l'invention.

### Exemple 1

On a préparé la composition de teinture suivante :

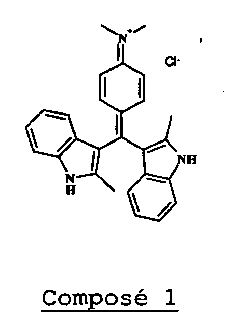

| | |
|---|---|
| {4-[Bis-(2-méthyl-1H-indol-3-yl)-methylène]-cyclohexa-2,5-diénylidène}-diméthyl-ammonium chlorure | 0,427 g |
| Alcool benzylique | 4,0 g |
| Polyéthylèneglycol 6OE | 6,0 g |
| Hydroxyéthylcellulose | 0,7 g |
| Alkylpolyglucoside en solution aqueuse à 60% M.A* | 4,5 g M.A |
| Tampon phosphate q.s pH | 7 |
| Eau déminéralisée q.s.p | 100, g |

| | |
|---|---|
| * Matière Active | |

On a appliqué la composition ci-dessus sur des mèches de cheveux gris naturels ou permanentés à 90% de blancs et on a laissé poser pendant 20 minutes. Après rinçage à l'eau courante et séchage, les cheveux ont été teints dans une nuance bleu.

### Exemple 2

On a préparé la composition de teinture suivante :

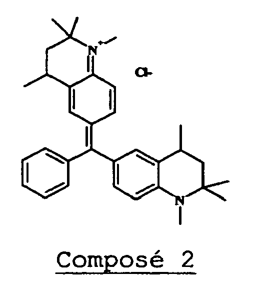

| | |
|---|---|
| Chlorure de 1,2,2,4-Tetraméthyl-6-[phényl-(1,2,2,4-tetraméthyl-1,2,3,4-tetrahydro-quinolin-6-yl)-methylène]-2,3,4,6-tetrahydroquinolinium | 0,50 g |
| Diéthanolamide oléïque | 3 g |
| Acide laurique | 1 g |
| Monoéthyléther de l'éthylèneglycol | 5 g |
| Hydroxyéthylcellulose | 2 g |
| 2-amino-2-méthyl-1-propanol q.s pH | 9,5 |
| Eau déminéralisée q.s.p. | 100 g |

On a appliqué la composition ci-dessus sur des mèches de cheveux gris naturels ou permanentés à 90% de blancs et on a laissé poser pendant 30 minutes. Après rinçage à l'eau courante et séchage, les cheveux ont été teints dans une nuance bleu.

### Exemples 3 à 6

On a préparé les cinq compositions de teinture directe réunies dans le tableau suivant :
(toutes teneurs exprimées en grammes)

| Composé 1 | Composé 2 | Composé 3 | Composé 4 |
|---|---|---|---|
| 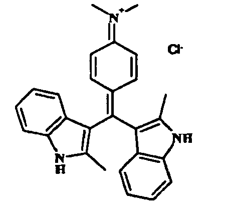 | 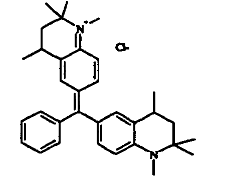 | 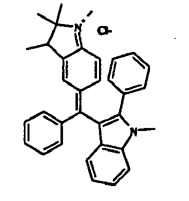 | 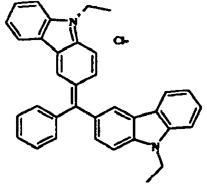 |
| {4-[Bis-(2-méthyl-1H-indol-3-yl)-méthylène]-cyclohexa-2,5-diénylidène}-diméthyl-ammonium chlorure | Chlorure de 1,2,2,4-Tetraméthyl-6-[phényl-(1,2,2,4-tétraméthyl-1,2,3,4-tétrahydro-quinolin-6-yl)-méthylène]-2,3,4,6-tétrahydroquinolinium | Chlorure de 1,2,2,3-Tétraméthyl-5-[(1-méthyl-2-phényl-1H-indol-3-yl)-phényl-méthylène]-3,5-dihydro-2H-indolinum | Chlorure de 9-Ethyl-3-[(9-éthyl-9H-carbazol-3-yl)-phényl-méthylène]-3H-carbazolium |

| | Exemple 3 | Exemple 4 | Exemple 5 | Exemple 6 |
|---|---|---|---|---|
| Colorant direct de formule (1) | 0,2 | | | |
| Colorant direct de formule (2) | | 0,2 | | |
| Colorant direct de formule (3) | | | 0,2 | |
| Colorant direct de formule (4) | | | | 0,2 |
| Acide polyacrylique réticulé vendu sous la dénomination Carbopol 2984 par la société Goodrich | 1,0 MA* | | | |
| Copolymère acrylate d'ammonium/acrylamide vendu sous la dénomination Bozepol C Nouveau par la société Hoechst | | 1,0 MA* | | |
| Copolymère acide méthacrylique/acrylate d'éthyle réticulé vendu en dispersion aqueuse à 38% de matière active sous la dénomination Viscoatex 538C par la société Coatex | | | 1,0 MA* | |
| Copolymère acide acrylique/acrylate d'éthyle réticulé vendu en dispersion aqueuse à 28% de matière active sous la dénomination Aculyn 33 par la société Rohm & Haas | | | | 1,0 MA* |
| Ethanol | 10 | 10 | 10 | 10 |
| 2-amino-2-méthyl-1-propanol qs | pH 9 | pH 9 | pH 9 | pH 9 |
| Eau déminéralisée qsp | 100 | 100 | 100 | 100 |

| | | | | |
|---|---|---|---|---|
| MA* désigne Matière Active | | | | |

Les compositions ci-dessus ont été appliquées chacune pendant 30 minutes sur des mèches de cheveux gris naturels à 90 % de blancs. Les mèches de cheveux ont ensuite été rincées, lavées avec un shampooing standard puis séchées.

Les mèches ont été teintes dans les nuances suivantes :

| Exemples | Nuances obtenues |
|---|---|
| 3 | Bleu puissant |
| 4 | Bleu puissant |
| 5 | Bleu violet puissant |
| 6 | vert puissant |

### Exemples 7 à 10

On a préparé les compositions 7 (A) à 10 (A), conformes à l'invention, suivantes (teneurs en grammes) :

| COMPOSITION | 7 (A) | 8 (A) | 9 (A) | 10 (A) |
|---|---|---|---|---|
| Paratoluylènediamine | 0,25 | - | - | - |
| Para-aminophénol | 0,30 | 0,50 | 0,15 | - |
| Paraphénylènediamine | - | 0,20 | | 0,30 |
| 5-N-(β-hydroxyéthyl)amino 2-méthyl phénol | 0,5 | 0,8 | 0,17 | - |
| 5-amino 2-méthyl phénol | - | - | - | 0,30 |
| Colorant de structure (2) | 0,15 | - | - | - |
| Colorant de structure (3) | - | 0,20 | 0,05 | - |
| Colorant de structure (4) | - | - | - | 0,1 |
| Support de teinture commun (*) | (*) | (*) | (*) | (*) |
| Eau q.s.p. | 100 g | 100 g | 100 g | 100 g |

| | | | | |
|---|---|---|---|---|
| (*) support de teinture commun : - Alcool oléique polyglycérolé à 2 moles de glycérol 4,0 g - Alcool oléique polyglycérolé à 4 moles de glycérol, à 78 % de matières actives (M.A.) 5,69 g M.A. - Acide oléique 3,0 g - Amine oléique à 2 moles d'oxyde d'éthylène vendue sous la dénomination commerciale ETHOMEEN O12 par la société AKZO 7,0 g - Laurylamino succinamate de diéthylaminopropyle, sel de sodium, à 55 % de M.A. 3,0 g M.A. - Alcool oléique 5,0 g - Diéthanolamide d'acide oléique 12,0 g - Propylèneglycol 3,5 g - Alcool éthylique 7,0 g - Dipropylèneglycol 0,5 g - Monométhyléther de propylèneglycol 9,0 g - Métabisulfite de sodium en solution aqueuse, à 35 % de M.A. 0,455 g M.A. - Acétate d'ammonium 0,8 g - Antioxydant, séquestrant q.s. - Parfum, conservateur q.s. - Ammoniaque à 20 % de NH₃ 10,0 g | | | | |

Au moment de l'emploi, on a mélangé chacune de ces compositions 7 (A) à 10 (A) avec une quantité égale d'une composition (B) constituée par une solution de peroxyde d'hydrogène à 20 volumes (6 % en poids).

Chaque composition résultante (composition prête à l'emploi conforme à l'invention) a été appliquée pendant 30 minutes sur des mèches de cheveux gris naturels à 90 % de blancs. Les mèches de cheveux ont ensuite été rincées, lavées avec un shampooing standard puis séchées.

Les mèches de cheveux ont été teintes dans les nuances figurant dans le tableau ci-dessous :

| EXEMPLE [COMPOSITION] | NUANCE OBTENUE |
|---|---|
| 7 [7 (A)] | Blond foncé à reflet bleu |
| 8 [8 (A)] | Blond à reflet bleu violacé |
| 9 [9 (A)] | Blond clair à reflet bleu violacé |
| 10 [10 (A)] | Blond à reflet vert |

Les nuances obtenues ont présenté une très bonne ténacité aux shampooings ultérieurs.

Selon une variante de l'invention, les colorants directs peuvent être incorporés dans les compositions colorantes au moment de l'emploi.

### Exemple 11

On a préparé la composition 11 (A) suivante :
- 1,4-diamino benzène 0,40 g
- 5-amino 2-méthyl phénol 0,45 g
- Support de teinture commun tel que décrit précédemment pour les exemples 7 à 10 (*)
- Eau déminéralisée q.s.p. 100 g

On a préparé la composition 11 (A') suivante : 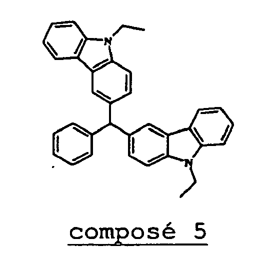 bis(9-éthyl-9H-carbazol-3-yl)phénylméthane
- Colorant de structure (5) 4 g
- Polyammonium quaternaire vendu sous la dénomination commerciale CELQUAT SC-240 par la société National Starch 10 g
- Sciure de bois q.s.p. 100 g

Au moment de l'emploi, on a mélangé une partie en poids de la composition 11 (A) ci-dessus avec 0,1 partie en poids de la composition 11 (A') et avec une partie en poids d'une composition (B) constituée par une solution de peroxyde d'hydrogène à 20 volumes (6 % en poids).

La composition résultante a été appliquée pendant 30 minutes sur des mèches de cheveux gris naturels à 90 % de blancs. Les cheveux ont ensuite été rincées, lavées avec un shampooing standard puis séchées.

Les cheveux ont été teints dans une nuance châtain clair à reflet vert résistant très bien aux shampooings ultérieurs.

## Revendications

1. Utilisation d'une Composition comprenant dans un milieu cosmétique approprié pour la teinture, au moins un colorant choisi parmi les composés répondant à la formule (I), (Ibis), (Iter), (II) ou (IIbis) suivante ou leurs formes tautomères, et leurs sels d'addition : 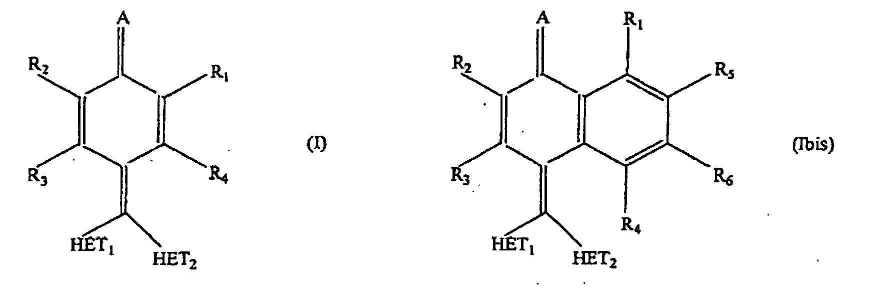 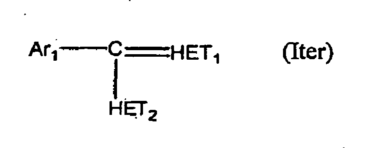 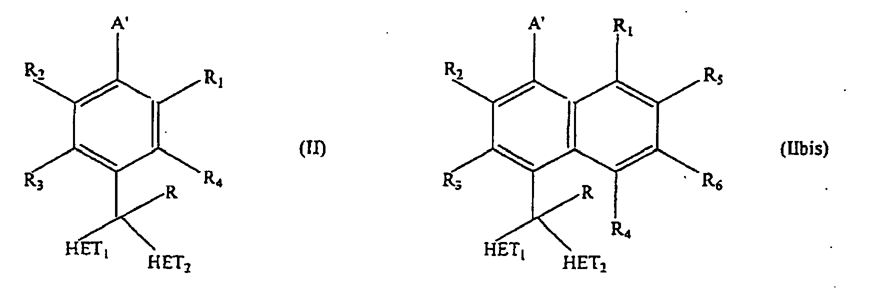 dans lesquelles :
- A représente O ; NH ; N-alkyle N-hydroxyalkyle ; ammonium ; N-alkylammonium ; N-(hydroxyalkyl)ammonium ; N,N-dialkylammonium, N,N-di(hydroxyalkyl)ammonium, N-(hydroxyalkyl)-N-(alkyl) ammonium, dans lesquels les deux groupements alkyle peuvent former avec l'atome d'azote auquel ils sont liés, un cycle pouvant être interrompu par un ou plusieurs atomes d'azote, d'oxygène ou de soufre ;
- HET₁, HET₂ représentent indépendamment l'un de l'autre un hétérocycle aromatique, éventuellement substitué par un ou plusieurs groupements Z' ;
- Ar₁ représente un radical aryle éventuellement substitué par un ou plusieurs groupements Z ;
- R₁ à R₆ et Z, Z' désignent indépendamment l'un de l'autre un groupement choisi parmi l'atome d'hydrogène, les atomes d'halogène, tels que F, Cl, Br et I, les radicaux -NHSO₃H ; hydroxyle ; alkyle ; alcoxy ; alkylthio ; amino, monoalkylamino ; dialkylamino, dans lesquels les deux groupements alkyle peuvent conjointement, avec l'atome d'azote, auquel ils sont liés, former un cycle pouvant être interrompu par un ou plusieurs atomes d'azote, d'oxygène ou de soufre ; les hétérocycles et les radiaux nitro ; aryle acyle ; alcoxycarbonyle ; carboxamido ; cyano ; -CO₂H ; -SO₃H ; -PO₃H₂ ; -PO₄H₂ ;
- A' représente un atome d'hydrogène, un groupe hydroxyle, amino, (hydroxyalkyl)amino, di(hydroxyalkyl)amino, (hydroxyalkyl)(alkyl)amino, monoalkylamino, dialkylamino, dans lesquels les deux groupements alkyle peuvent former avec l'atome d'azote auquel ils sont liés, un cycle pouvant être interrompu par un ou plusieurs atomes d'azote, d'oxygène ou de soufre ;
- R représente un atome d'hydrogène ou d'halogène ; un radical hydroxyle ; un radical alcoxy ; un radical alkylthio,
pour la teinture des fibres kératiniques.

2. Utilisation selon la revendication 1, dans laquelle les hétérocycles et notamment ceux de HET₁ et HET₂, sont choisis parmi les hétérocycles suivants : thiophène, benzothiophène, furane, benzofurane, indole, indoline, carbazole, pyridine, déhydroquinoléine, chromone, julolidine, thiadiazole, triazole, isoxazole, oxazole, thiazole, isothiazole, imidazole, pyrazole, triazine, thiazine, pyrazine, pyridazine, pyrimidine, pyridine, diazépine, oxazépine, benzotriazole, benzoxazole, benzimidazole, benzothiazole, morpholine, pipéridine, pipérazine, azétidine, pyrrolidine, aziridine.

3. Utilisation selon la revendication 1, **caractérisé en ce que** la composition comprend un composé de formule (I), (Ibis), (Iter), (II) ou (IIbis) choisi parmi les composés suivants pour lesquels les contre ions sont ou non spécifiés, et leurs sels d'addiction
4-[Bis-(2-méthyl-1H-indol-3-yl)-méthylène)-cyclohexa-2,5-diènylidene]-diméthyl-ammonium
Chlorure de 1,2,2,4-Tétraméthyl-6-[phényl-(1,2,2,4-tétraméthyl-1,2,3,4-tétrahydro-quinaolin-6-yl)-méthylène]-2,3,4,6-tétrahydroquinolinium
Chlorure de 1,2,2,3-Tétraméthyl-5-[(1-méthyl-2-phényl-1H-indol-3-yl)-phényl-méthylène]-3,5-dihydro-2H-indolinum
Chlorure de 9-Éthyl-3-[(9-éthyl-9H-carbazal-3-yl)-phényl-méthylène]-3H-carbazolium
1-Éthyl-3-(1-éthyl-2-méthyl-1H-indol-yl)-p-tolyl-méthylène]-2-méthyl-3a,7a-dihydro-3H-indolium
6-{(4-Nitro-phényl)-[1-(4-sulfo-butyl)-1,2,3,4-tétrahydro-quinolin-6-yl]-méthylèn}-1-(4-sulfo-butyl)-2,3,4,6-tétrahydro-quinolinium
6-{(4-Cyano-pényl)-[1-(4-sulfo-butyl)-1,2,3,4-tétrahydro-quinolin-6-yl]-méthylèn}-1-(4-sulfo-butyl)-2,3,4,6-tétrahydro-quinolinium
Diéthyl-{4-[(2-méthyl-1H-indol-3-yl)-thiophen-2-yl-méthylène]-cyclchexa-2,5-diènylidene}-ammoxium
1-Éthyl-3-[(1-éthyl-2-méthyl-1H-indol-3-yl)-(4-méthoxy-phényl)-méthylène] -2-méthyl-3a,7a-dihydro-3H-indolium
Éthanaminium, N-[4-[(3-carboxypyrazinyl)(1-éthyl-2-méthyl-1H-indol-3-yl)méthylènel-3-éthoxy-2,5-cyclohexadièn-1-ylidene]-N-éthyl-, sel interne
Éthanaminium, N-[4-[(3-carboxypyrazinyl)(1-éthyl-2-méthyl-1H-indol-3-yl)méthylène]-3-éthoxy-2,5-cyclchexadièn-1-ylidene]-N-éthyl-, chlorure
Cyclohepta[b]furylium, 3-[[4-(diméthylamino)phényl](2-oxo-2H-cyclohepta[b]furan-3-yl)méthylène]-2,3-dihydro-2-oxo
Méthanaminium, N-[4-[bis(1,2,3,4,10,14b-hexahydro-2-méthyldibenz[c,f]pyrazino[1,2-a]azepin-8-yl) méthylène]-2,5-cyclohexadièn-1-ylidene]-N-méthyl-, perchlorate, diperchlorate
Méthanaminium, N-[4-[bis(1,2,3,4,10,4b-hexahydro-2-méthyldibenzo [c,f]pyrazino[1,2-a]azepin-8-yl)méthylène]-2,5-cyclohexadièn-1-ylidene]-N-méthyl-, perchlorate
Méthanaminium, N-[4-[bis(1,2,3,4,10,14b-hexahydno-2-méthyldibenzo [c,f]pyrazino[1,2-a]azepin-8-yl)méthylène]-2,5-cyclohexadièn-1-ylidene]-N-méthyl-
Méthanaminium, N-[4-[bis(2,3-dihydro-1,5-diméthyl-3-oxo-2-phényl-1H-pyrazol-4-yl)méthylène]-2,5-cyclohexadièn-1-ylidene]-N-méthyl-
Morpholinium, 4-[5-[[4-(diméthylamino)phényl][5-(4-morpholinyl) -2-thiényl]méthylène]-2(5H)-thiénylidene]-, perchlorate
Morpholinium, 4-[5-[[4-(diméthylamino)phényl][5-(4-morpholinyl)-2-thiényl]méthylène]-2(5H)-thiénylidéne]-
1H-Indolizinium, 7-(diméthylamino)-1-[[7-(diméthylamino)-3- (éthoxycarbonyl)-1-indolizinyl][4-(diméthylamino)phényl]méthylène]-3- (éthoxycarbonyl)-, perchlorate
1H-Indolizinium, 7-(diméthylamino)-1-[[7-(diméthylamino)-3- (éthoxycarbonyl)-1-indolizinyl][4-(diméthylamino)phényl]méthylène]-3- (éthoxycabonyl)-
Cyclohepta[b]pyrrolium, 3-[(1,2-dihydro-2-oxo-1-phénylcyclohepta[b]pyrrol-3-yl)[4-(diméthylamino)phényl]méthylène]-2,3-dihydro-2-oxo-phényl-, hexafluorophosphate
Cyclohepta[b]pyrrolium, 3-[(1,2-dihydro-2-oxo-1-phénylcohepta[b]pyrrol-3-yl)[4-(diméthylamino)phényl]méthylène]-2,3-dihydro-2-oxo-1-phényl-
Quinolinium, 6-[[4-(diméthylamino)phényl](1,2,3,4-tétrahydro-1,2,2,4-tétraméthyl-6-quinolinyl)méthylène]-2,3,4,6-tétrahydro-1,2,2,4-tétraméthyl-, chlorure
Quinolinium, 6-[(1,2-dihydro-1,2,2,4-tétraméthyl-6-quinolinyl)[4-(diméthylamino)phényl]méthylène]-2,6-dihydro-1,2,2,4-tétraméthyl-, chlorure
3H-Carbazolium, 9-éthyl-3-[(9-éthyl-9H-carbazol-3-yl)[4-(méthylphénylanino)phényl]méthylène]-, tétrfluorobarate(1-)
3H-Carbazoliun, 3-[[4-(diméthylamino)phényl](9-éthyl-9H-catbazol-3-yl)méthylène]-9-éthyl-, tétrafluoroborate(1-)
3H-Carbazolium, 9-éthyl-3-[(9-éthyl-9H-carbazol-3-yl)[4- (méthylphénylamino) phényl]méthylène]-, perchlorate
3H-Carbazolium, 9-éthyl-3-[(9-éthyl-9H-carbazol-3-yl)[4- (méthylphénylamino) phényl]méthylènel-, bromure
3H-Carbazolium, 9-éthyl-3-[(9-éthyl-9H-carbazol-3-yl)[4-(méthylphénylamino) phényl]méthylène]-, chlorure
3H-Carbazalium, 3-[[4-(diméthylamino)phényl](9-éthyl-9H-carbazol-3-yl)méthylène]-9-éthyl-, bromure
3H-Carbazoliwn, 3-[[9-(diméthylamino)phényl](9-éthyl-9H-carbazol-3-yl)méthylène]-9-éthyl-, chlorure
3H-Carbazolium, 9-éthyl-3-[(9-éthyl-9H-carbazol-3-yl)[4-(méthylphénylamino) prényl]méthylène]-, iodure
3H-Carbazolium, 3-[[4-(dimethylamino)phényl](9-éthyl-9H-carbazol-3-yl)méthylène]-9-éthyl-, iodure
3H-Carbazolium, 3-[[4-(diméthylamino)phényl](9-éthyl-9H-carbazol-3-yl)méthylène]-9-éthyl-, perchlorate
1H-Pyrazoliun, 4- [(2,3-dihydro-1,5-diméthyl-3-oxo-2-phényl-1H-pyrazol-4-yl)[4-(triméthylammonio)phényl]méthylène]-4,5-dihydro-2,3-diméthyl-5-oxo-1-phényl-, diperchlorate
1H-Pyrazolium, 4-[(2,3-dihydro-1,5-diméthyl-3-oxo-2-phényl-1H-pyrazol-4-yl)[4- (triméthylammonio)phényl]méthylène]-4,5-dihydro-2,3-diméthyl-5-oxo-1-phényl-
5H-Benzo[a]carbazolium, 11-éthyl-5-[(11-éthyl-3-méthoxy-8-méthyl-11H-benzo[a] carbazol-5-yl)(4-nitrophényl)méthylène]-3-méthoxy-8-méthyl-, tétrafluoroborate
5H-Benzo[a]carbazolium, 11-éthyl-5-[(11-éthyl-3-méthoxy-8-méthyl-11H-benzo[a] carbazol-5-yl)(4-nitrophényl)méthylène]-3-méthoxy-8-méthyl-
5H-Benzo[a]carbazolium, 11-éthyl-5-[(11-éthyl-2-méthoxy-8-méthyl-11H-henzo[a]carbazol-5-yl)(4-nitrophényl)méthylène]-2-méthoxy-8-méthyl-, tétrafluoroborate
5H-Bnzo[a]carbazolium, 11-éthyl-5-[(11-éthyl-2-méthoxy-8-méthyl-11H-benzo[a]carbazol-5-yl)(4-nitrophényl)méthylène]-2-méthoxy-8-méthyl-
5H-Benzo[a]carbazolium, 11-éthyl-5-[(11-éthyl-8-méthoxy-11H-benzo[a]carbazol-5-yl)(4-nitrophényl)méthylène]-8-méthoxy-, tétrafluoroborate
5H-Benzo[a]carbazolium, 11-éthyl-5-[11-éthyl-8-méthoxy-11H-benzo[a]carbazol-5-yl)(4-nitrophényl)méthylène] -8-méthoxy-
5H-Benzo[a]carbazolim, 11-éthyl-5-[(11-éthyl-4-méthoxy-11H-benzo[a]carbazol-5-yl)(4-nitrophényl)méthylène]-4-méthoxoy-, tétrafluoroborate (
5H-Benzo[a]carbazolium, 11-éthyl-5-[(11-éthyl-4-méthoxy-11H-benzo[a]caroazol-5-yl)(4-nitrophényl)méthylène]-4-méthoxy-
5H-Benzo[a]carbazolium, 11-éthyl-5-[11-éthyl-3-méthoxy-11H-benzo[a]carbazol-5-yl)(4-nitrophényl)méthylène]-3-méthoxy-,tétrafluoroborate
5H-Benzo[a]carbazolium, 11-éthyl-5-[(11-éthyl-3-méthoxy-11H-benzo[a]carbazol-5-yl)(4-nitrophényl)méthylène]-3-méthoxy-
5H-Henzo[a]carbazolium, 11-éthyl-5[(11-éthyl-2-méthoxy-11H-benzo[a]carbazol-5-yl)(4-nitrophémyl)méthylène]-2-méthoxy-,tétrafluoroborate
5H-Benzo[a]carbazolium, 11-éthyl-5-[(11-éthyl-2-méthoxy-11H-benzo[a]carbazol-5-yl)(4-nitrophényl)méthylène]-méthoxy-
5H-Benzo[a]carbazolium, 5-[(2-chloro-4-nitrophényl)(11-éthyl-11H-benzo[a]carbazol-5-yl)méthylène]-11-éthyl-, sel avec l'acide trifluorométhanesulfonique (1:1)
5H-Benzo[a] carbazolium, 5-[(2-chloro-4-nitrophényl)(11-éthyl-11H-benzo[a]carbazol-5-yl)méthylène] -11-éthyl
5H-Benzo[a]carbazolium, 11-èthyl-5-[(11-éthyl-11H-benzo[a]carbazol-5-yl)(4-nitrophényl)méthylènel-, tétraflucroborate
5H-benzo[a]carbazolium, 11-éthyl-5-[(11-éthyl-11H-benzo[a]carbazol-5-yl)(4-nitrophényl)méthylène]-
5H-Benzo[a]carbazolium, 5-[[4-(diéthylamino)phényl](11-éthyl-11H-benzo[a]carbazol-5-yl)méthylène]-11-éthyl-, tétrafluoroborate(1-)
5H-Benzo[a]carbazolium, 5-[[4-(diéthylamino)phényl](11-éthyl-11H-benzo[a]carbazol-5-yl)méthylène]-11-éthyl-
5H-Benzo[a]carbazolium, 5-[[4-(diméthylamino)phényl](11-éthyl-11H-benzo[a]carbazol-5-yl)méthylène]-11-éthyl-, tétrafluoroborate(1-)
5H-Benzo[a]carbazolium, 5-[[4-(diméthylamino)phénpyl](11-éthyl-11H-benzo[a]carbazol-5-yl)méthylène]-11-éthyl-
Sel de 1H-Pyrazolium, 4-[(2,3-dihydro-1,5-diméthyl-3-oxo-2-phényl-1H-pyrazol-4-yl)[4-(diméthylamino)phényl]méthylène]-4,5-dihydro-2,3-diméthyl-5-oxo-1-phényl- et d'un monoacide
3H-Carbazolium, 3-[[4-[bis(phénylméthyl)amino]phényl](9-éthyl-9H-carbazol-3-yl)méthylène]-9-éthyl-, iodure
3H-Imidazo[1,2-a]benzimidazolium, 3-[[4-(diméthylamino)phényl](9-méthyl-2-phényl-9H-imidazo[1,2-a]benzimidazol-3-yl)méthylène]-9-méthyl-2-phényl-, chlorure
3H-Imidazo[1,2-a]benzimidazolium, 3-[[4-(diméthylamino)phényl](2,9-diméthyl-9H-imidazo[1,2-a]benzimidazol-3-yl)méthylène]-2,9-diméthyl-,bromure
1H-Pyrazolium, 4-[(2,3-dihydro-1,5-diméthyl-3-oxo-2-phényl-1H-pyrazol-4-yl)[4-(dipropylamino)phényl]méthylène]-4,5-dihydro-2,3-diméthyl-5-oxo-1-phényl-
1H-Pyrazolium, 4-[[4-(diéthylamino)phényl](2,3-dihydro-1,5-diméthyl-3-oxo-2-phényl-1H-pyrazol-4-yl)méthylène]-4,5-dihydro-2,3-diméthyl-5-oxo-1-phényl-
1H-Pyrazolium, 4-[(2,3-dihydro-1,5-diméthyl-3-oxo-2-phényl-1H-pyrazol-4-yl)(4-nitrophényl)méthylène]-4,5-dihydro-2,3-diméthyl-5-oxo-1-phényl-
1H-Pyrazolium, 4-[(2,3-dihydro-1,5-diméthyl-3-oxo-2-phényl-1H-pyrazol-4-yl)[4-(diméthylamino)phényl]méthylène]-4,5-dihydro-2,3-diméthyl-5-oxo-1-phényl-
1H-Pyrazoliun, 4-[(4-aminophényl)(2,3-dihydro-1,5-diméthyl-3-oxo-2-phényl-1H-pyrazol-4-yl)méthylène]-2,3-diméthyl-5-oxo-1-phényl
3H-Indolizinium, 3-[[4-(diméthylamino)phényl](6-éthenyl-1,2-diméthyl-3-indolizinyl)méthylène]-6-éthenyl-1,2-diméthyl-
3H-Indolinium, 3-[p-(diméthylamino)-α-(6-éthyl-1,2-diméthyl-3-indolizinyl)benzylidene]-6-éthyl-1,2-diméthyl-, iodure
3H-Indolizinium, 3-[p-(diméthylamino)-α-(1,2-diméthyl-6-vinyl-3-indolizinyl)benzylidene]-1,2-diméthyl-6-vinyl-, perchlorate
2-Pyrazolinium, 4-(p-amino-α-antipyrinylbenzylidene)-2,3-diméthyl-5-oxo-1-phényl-, perchlorate
2-Pyrazolinium, 9-(α-antipyrinyl-p-nitrobenzylidene)-2,3-diméthyl-5-oxo-1-phényl-, perchlorate
2-Pyrazolinium, 9-[α-antipyrinyl-p-(diéthylamino)benzylidene]-2,3-diméthyl-5-oxo-1-phényl-, perchlorate
2-pyrazolinium, 4-[α-antipyrinyl-p-(diméthylamino)benzylidene]-2,3-diméthyl-5-oxo-1-phényl-, perchlorate
Méthylium, bis[6-(diméthylamino)-3-méthyl-1H-indol-2-yl][4-(diméthylamino)phényl]-, perchlorate
Méthylium, bis[6-(diméthylamino)-3-méthyl-1H-indol-2-yl][4-(diméthylamino)phényl]-
Méthylium, bis[6-(diséthylamino)-3-méthyl-1H-indol-2-yl](4-nitrophényl)-, perchlorate
Méthylium, bis[6-(diméthylamino)-3-méthyl-1H-indol-2-yl](4-nitrophényl)-
Méthylium, bis(benzo[b]thién-2-yl)[4-(diméthylamino)phényl]-, tétrafluoroborate
Méthylium, bis(benzo[b]thién-2-yl)[4-(diméthylamino)phényl]-
Méthylium, [4-(diméthylamino)phényl]bis(1-méthyl-1H-pyrrol-2-yl)-, tétrafluoroborate
Méthylium, [4-(diméthylamino)phényl]bis(1-méthyl-1H-pyrrol-2-yl)-Diantipyrinyl (p-nitrophényl)méthylium perchlorate
Méthylium, bis(9,10-dihydro-9,9,10-triméthyl-3-acridinyl)[4-(diméthylamino)phényl]-, chlorure
Méthylium, [4-(diméthylamino)phényl]bis(10-méthyl-10H-phenothiazin-2-yl)-, chlorure
3H-Pyrazol-3-one, 4,4'-[[4-(diméthylamino)phényl]méthylène]bis[1,2-dihydro-1,5-diméthyl-2-phényl-, mono[tétrafluoroborate(1-)]
3H-Pyrazol-3-one, 4,4'-[[4-(diméthylamino)phényl]méthylène]bis[1,2-dihydro-1,5-diméthyl-2-phényl-, bis[tétrafluoroborate(1-)]
Sel de 3H-Pyrazol-3-one, 4,4'-[[4-(diméthylamino)phényl]méthylène]bis[1,2-dihydro-1,5-diméthyl-2-phényl-et d'wn diacide
Sel de 3H-Pyrazol-3-one, 4,4'-[[4-(diméthylamino)ményl]méthyléne]bis[1,2-dihydro-1,5-diméthyl-2-phényl-, et d'un monoacide
Quinolinium, 4,4'-[(4-nitrophényl)méthylène]bis[1-éthyl-, dichlorure Méthylium, bis(9-éthyl-9H-carbazol-3-yl)(4-nitrophényl)-, iodurs
Méthylium, [4-(diméthylamino)phényl]bis(9-éthyl-9H-carbazol-3-yl)-, ioduré
Méthylium, 9H-carbazol-3-yl[4-(diméthylamino)phényl](6-méthyl-9H-carbazol-3-yl)-
Quinolizinium, 2,2'-[(4-nitrophényl)méthylène]bis[1,2,3,4-tétrahydro-1-oxo-Méthylium, 9H-carbazol-3-yl[4-(diméthylamino)phényl](6-méthyl-9H-carbazol-3-yl)-, tétrafluoroboraté
6,6-Dichloro-9,9'-dibutyl-3,3'-dicarbazolyl-p-nitrophénylméthyl chlorure
Quinolizinium, 2,2'-(p-nitrobenzylidene)bis[1,2,3,4-tétrahydro-1-oxo-, diperchlorate
Benzylium, p-(diéthylamino)-α,α-bis(2,3-diméthyl-5-oxo-1-phényl-3-pyrazolin-4-yl)-
Benzylium, α,α-diantipyrinyl-p-(diméthylamino)-
Benzylium, α,α-bis(2,3-diméthyl-5-oxo-1-phényl-3-pyrazolin-4-yl)-p-nitro-
Benzylium, p-amino-α,α-di-4-antipyrinyl-.

4. Utilisation selon l'une quelconque des revendications précédentes, **caractérisée en ce que** le ou les composés de formule (I), (Ibis), (Iter), (II) et (IIbis) et/ou le ou les sel(s) d'addition représentent de 0,0001 à 10 % en poids du poids total de la composition de teinture, de préférence de 0,005 à 10 % en poids et, de préférence encore, de 0, 01 à 6 % en poids du poids total de la composition de teinture.

5. Utilisation selon l'une quelconque des revendications 1 à 4, **caractérisée en ce que** la composition contient, en outre, un ou plusieurs colorant(s) direct(s) différent(s) des composés de formule (I), (Ibis), (Iter), (II) et (IIbis).

6. Utilisation selon la revendication 5, **caractérisée en ce que** le ou les colorant(s) direct(s) différent(s) des composés de formule (I), (Ibis), (Iter), (II) et (IIbis) sont choisis parmi les colorants directs nitrés benzéniques neutres, acides ou cationiques, les colorants directs azoïques neutres acides ou cationiques, les colorants directs quinoniques neutres, acides ou cationiques, les colorants directs aziniques, les colorants directs triarylméthaniques, les colorants directs indoaminiques et les colorants directs naturels.

7. Utilisation selon l'une quelconque des revendications 5 et 6, **caractérisée en ce que** le ou les colorant(s) direct(s) différents des composés (I), (Ibis), (Iter), (II) et (IIbis), représentent de 0,001 à 20 % en poids, de préférence de 0,005 à 10 % en poids du poids total de la composition de teinture.

8. Utilisation selon l'une quelconque des revendications précédentes, **caractérisée en ce que** la composition contient, en outre, une ou plusieurs base(s) d'oxydation et éventuellement un ou plusieurs coupleur(s).

9. Utilisation selon la revendication 8, **caractérisé en ce que** la ou les base (s) d'oxydation sont choisies parmi les paraphénylènediamines, les bis-phénylalkylènediamines, les para-aminophénols, les ortho-aminophénols, les bases hétérocycliques et leurs sels d'addition.

10. Utilisation selon l'une quelconque des revendications 8 et 9, **caractérisée en ce que** le ou les coupleur(s) sont choisi(s) parmi les coupleurs métaphénylènediamines, les coupleurs méta-aminophénols, les coupleurs métadiphénols, les coupleurs naphtaléniques, les coupleurs hétérocycliques et leurs sels d'addition.

11. Utilisation selon l'une quelconque des revendications 8 à 10, **caractérisée en ce que** le ou les base(s) d'oxydation et/ou le ou les coupleur(s) sont présents chacun en une quantité dé 0,001 à 10 % en poids, de préférence de 0,005 à 6 % en poids du poids total de la composition de teinture.

12. Utilisation selon l'une quelconque des revendications précédentes, **caractérisée en ce que** la composition a un pH de 3 à 12, de préférence de 5 à 11, de préférence encore de 6 à 8, 5.

13. Procédé de teinture directe des fibres kératiniques **caractérisé en ce qu'**il comprend l'application d'au moins une composition de teinture telle que définie dans l'une quelconque des revendications 1 à 12 contenant au moins un composé de formule (I), (Ibis), (Iter), (II) ou (IIbis) sur les fibres kératiniques, puis l'observation d'un temps de pause suivi du rinçage des fibres.

14. Procédé selon la revendication 13, **caractérisé en ce que** le temps de pause est de 3 à 50 minutes, de préférence de 5 à 30 minutes.

15. Procédé de teinture des fibres kératiniques, dans lequel on applique sur lesdites fibres au moins une composition de teinture telle que définie dans l'une quelconque des Revendications 1 à 12 comprenant soit au moins un composé de formules (II) ou (IIbis), soit au moins un composé de formule (I), (Ibis), ou (Iter) et au moins une base d'oxydation et éventuellement un ou plusieurs coupleur(s) la couleur étant révélée à pH acide, neutre ou alcalin, à l'aide d'un agent oxydant.

16. Procédé selon la revendication 15, **caractérisé en ce que** l'agent oxydant est choisi parmi le peroxyde d'hydrogène, le peroxyde d'urée, 1 les bromates de métaux alcalins, les persels tels que les perborates et persulfates, les peracides et les enzymes oxydases, tel que les peroxydases, les oxydo-réductases à 2 électrons, comme les uricases et les oxygénases à 4 électrons, comme les laccases.

17. Dispositif à plusieurs compartiments comprenant un premier compartiment renfermant une composition tinctoriale telle que définie dans l'une quelconque des revendications 1 à 12, comprenant soit au moins un composé de formule, (II) ou (IIbis), soit au moins un composé de formule (I), (Ibis) ou (Iter) et an moins une base d'oxydation et éventuellement un ou plusieurs coupleur(s), et un deuxième compartiment renfermant une composition oxydante.

18. Composition pour la teinture des fibres kératiniques comprenant dans un milieu cosmétique approprié pour la teinture, au moins un colorant choisi parmi les composés répondant à la formule (I), (Ibis), (Iter), (II) ou (IIbis) suivante ou leurs formes tautomères, et leurs sels d'addition : 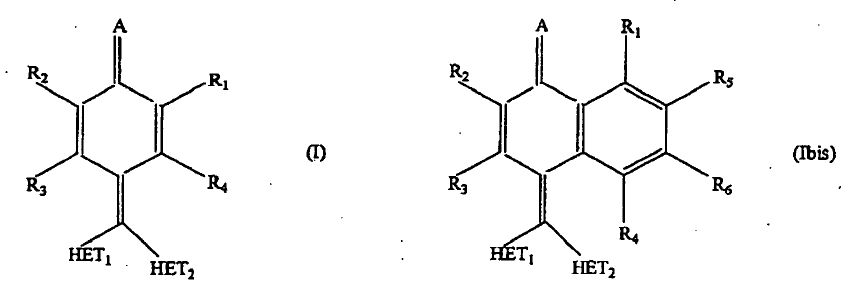 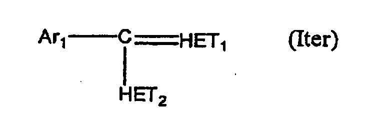 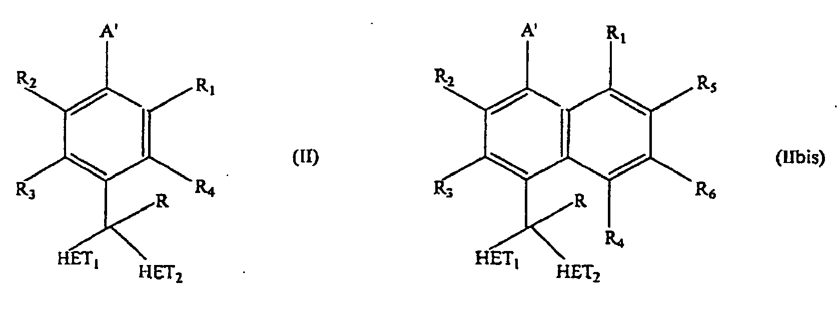 dans lesquelles :
- A représente O ; NH ; N-alkyle N-hydroxyalkyle ; ammonium ; N-alkylammonium ; N-(hydroxyalkyl)ammonium ; N, N-dialkylammonium, N,N-di(hydroxyalkyl)ammonium, N-(hydroxyalkyl)-N-(alkyl) ammonium, dans lesquels les deux groupements alkyle peuvent former avec l'atome d'azote auquel ils sont liés, un cycle pouvant être interrompu par un ou plusieurs atomes d'azote, d'oxygène ou de soufre ;
- HET₁, HET₂ représentent indépendamment l'un de l'autre un hétérocycle aromatique, éventuellement substitué par un ou plusieurs groupements Z' ;
- Ar₁ représente un radical aryle éventuellement substitué par un ou plusieurs groupements Z ;
- R₁ à R₆ et Z, Z' désignent indépendamment l'un de l'autre un groupement choisi parmi l'atome d'hydrogène, les atomes d'halogène, tels que F, Cl, Br et I, les radicaux -NHSO₃H ; hydroxyle ; alkyle ; alcoxy ; alkylthio ; amino, monoalkylamino ; dïalkylamino, dans lesquels les deux groupements alkyle peuvent conjointement, avec l'atome d'azote, auquel ils sont liés, former un cycle pouvant être interrompu par un ou plusieurs atomes d'azote, d'oxygène ou de soufre ; les hétérocycles et les radicaux nitro ; aryle ; acyle ; alcoxycarbonyle ; carboxamido ; cyano ; -CO₂H ; -SO₃H ; -PO₃H₂ ; -PO₄H₂ ;
- A' représente un atome d'hydrogène, un groupe hydroxyle, amino, (hydroxyalkyl)amino, di(hydroxyalkyl)amino, (hydroxyalkyl)(alkyl)amino, monoalkylamino, dialkylamino, dans lesquels les deux groupements alkyle peuvent former avec l'atome d'azote auquel ils sont liés, un cycle pouvant être interrompu par un ou plusieurs atomes d'azote, d'oxygène ou de soufre ;
- R représente un atome d'hydrogène ou d'halogène ; un radical hydroxyle ; un radical alcoxy ; un radical alkylthio;
ladite composition ayant un pH de 6 à 12.

19. Composition de teinture selon la revendication 18, **caractérisé en ce qu'**elle comprend un composé de formule (I), (Ibis), (Iter), (II) ou (IIbis) choisi parmi les composés suivants pour lesquels les contre ions sont ou non spécifiés, et leurs sels d'addition :
4-[Bis-(2-méthyl-1H-indol-3-yl)-méthylène]-cyclohexa-2,5-diènylidene]-diméthyl-ammonium
Chlorure de 1,2,2,4-Tétraméthyl-6-[phényl-(1,2,2,4-tétraméthyl-1,2,3,4-tétrahydro-quinolin-6-yl)-méthylène]-2,3,4,6-tétrahydroquinolinium
Chlorure de 1,2,2,3-Tétraméthyl-5-[(1-méthyl-2-phényl-1H-indol-3-yl)-phényl-méthylène]-3,5-dihydro-2H-indolinum
Chlorure de 9-Éthyl-3-[(9-éthyl-9H-carbazol-3-yl)-phényl-méthylène]-3H-carbazolium
1-Éthyl-3-[(1-éthyl-2-méthyl-1H-indol-3-yl)-p-tolyl-méthylène]-2-méthyl-3a,7a-dihydro-3H-indolium
6-[(4-Nitro-phényl)-[1-(4-sulfo-butyl)-1,2,3,4-tétrahydro-quinolin-6-yl]-méthylèn)-1-(4-sulfo-butyl)-2,3,4,6-tétrahydro-quinolinium
6-{(4-Cyano-phényl)-[1-(4-sulfo-butyl)-1,2,3,4-tétrahydro-quinolin-6-yl]-méthylèn}-1-(4-sulfo-butyl)-2,3,4,6-tétrahydro-quinolinium
Diéthyl-{4-[(2-méthyl-1H-indol-3-yl)-thiophen-2-yl-méthylène]-cyclchexa-2,5-diènylidene}-ammonium
1-Éthyl-3-[(1-éthyl-2-méthyl-1H-indol-3-yl)-(4-méthoxy-phényl)-méthylène]-2-méthyl-3a,7a-dihydro-3H-indolium
Éthanaminium, N-[4-[(3-carboxypyrazinyl)(1-éthyl-2-méthyl-1H-indol-3-yl)méthylène]-3-éthoxy-2,5-cyclohexadièn-1-ylidene]-N-éthyl-, sel interne
Éthanaminium, N-[4-[(3-carboxypyrazinyl)(1-éthyl-2-méthyl-1H-indol-3-yl)méthyléne]-3-éthoxy-2,5-cyclohexadièn-1-ylidene]-N-éthyl-, chlorure
Cyclohepta[b]furylium, 3-[[4-(diméthylamino)phényl](2-oxo-2H-cyclohepta[b]furan-3-yl)méthylène]-2,3-dihydro-2-oxo
Méthanaminium, N-[4-[bis(1,2,3,4,10,14b-hexahydro-2-méthyldibenz[c,f]pyrazino[1,2-a]azepin-8-yl)méthylène]-2,5-cyclohexadièn-1-ylidene]-N-méthyl-, perchlorate, diperchlorate
Méthanaminium, N-[4-[bis(1,2,3,4,10,14b-hexahydro-2-méthyldibenzo [c,f]pyrazino[1,2-a]azepin-8-yl)méthylène]-2,5-cyclohexadièn-1-ylidene]-N-méthyl-, perchlorate
Méthanaminium, N-[4-[bis(1,2,3,4,10,14b-hexahydro-2-méthyldibenzo [c,f]pyrazino[1,2-a]azepin-8-yl)méthylène]-2,5-cyclohexadièn-1-ylidene]-N-méthyl-
Méthanaminium, N-[4-[bis(2,3-dihydro-1,5-diméthyl-3-oxo-2-phényl-1H-pyrazol-4-yl)méthylène]-2,5-cyclohexadièn-1-ylidene]-N-méthyl-
Morpholinium, 4-[5-[[4-(diméthylamino)phényl][5-(4-morpholinyl)-2-thiényl]méthylène]-2(5H)-thiénylidene]-, perchlorate
Morpholinium, 4-[5-[[4-(diméthylamino)phényl][5-(4-morpholinyl)-2-thiényl]méthylène]-2(5H)-thiénylidène]-
1H-Indolizinium, 7-(diméthylamino)-1-[[7-(diméthylamino)-3- (éthoxycarbonyl)-1-indolizinyl][4-(diméthylamino)phényl]méthylène]-3- (éthoxycarbonyl)-, perchlorate
1H-Indolizinium, 7-(diméthylamino)-1-[[7-(diméthylamino)-3- (éthoxycarbonyl)-1-indolizinyl][4-(diméthylamino)phényl]méthylène]-3- (éthoxycarbonyl)-
Cyclohepta[b]pyrrolium, 3-[(1,2-dihydro-2-oxo-1-phénylcyclohepta[b]pyrrol-3-yl)[4-(diméthylamino)phényl]méthylène]-2,3-dihydro-2-oxo-1-phényl-, hexafluorophosphate
Cyclohepta[b]pyrrolium, 3-[(1,2-dihydro-2-oxo-1-phénylcyclohepta[b]pyrrol-3-yl)[4-(diméthylamino)phényl]méthylène]-2,3-dihydro-2-oxo-1-phényl-
Quinolinium, 6-[[4-(diméthylamino)phényl](1,2,3,4-tétrahydro-1,2,2,4-tétraméthyl-6-quinolinyl)méthylène]-2,3,4,6-tétrahydro-1,2,2,4-tétraméthyl-, Chlorure
Quinolinium, 6-[(1,2-dihydro-1,2,2,4-tétraméthyl-6-quinolinyl)[4-(diméthylamino)phényl]méthylène]-2,6-dihydro-1,2,2,4-tétraméthyl-,chlorure
3H-Carbazolium, 9-éthyl-3-[(9-éthyl-9H-carbazol-3-yl)[4-(méthylphénylamino)phényl]méthylène]-, tétrafluoroborate(1-)
3H-Carbazolium, 3-[[4-(diméthylamino)phényl](9-éthyl-9H-carbazol-3-yl)méthylène]-9-éthyl-, tétrafluaroborate(1-)
3H-Carbazolium, 9-éthyl-3-[(9-éthyl-9H-carbazol-3-yl)[4- (tréthylphénylamino) phény]méthylènel-, perchlorate
3H-Carbazolium, 9-éthyl-3-[(9-éthyl-9H-cazbaznl-3-yl)[4- (méthylphénylamino) phényl]méthylème]-, bromure
3H-Carbazolium, 9-éthyl-3-[(9-éthyl-9H-cazbazol-3-yl)[4- (méthylphénylamino) phényl]méthylène]-, chlorure
3H-Carbazolium, 3-[[4-(diméthylamino)phényl](9-éthyl-9H-carbazol-3-yl)méthyléne]-9-éthyl-, bromure
3H-Carbazolium, 3-[4-(diméthylamino)phényl](9-éthyl-9H-carbazol-3-yl)méthyléne]-9-éthyl-, chlorure
3H-Carbazolium, 9-éthyl-3-[(9-éthyl-9H-carbazol-3-yl)[4- (méthylphénylamino) phényl]méthylène]-, iodure
3H-Carbazolium, 3-[[4-(diméthylamino)phényl](9-éthyl-9H-carbazol-3-yl)méthylène]-9-éthyl-, indure
3H-Carbazolium, 3-[[4-(diméthylamino)(9-éthyl-9H-carbazol-3-yl)méthylène]-8-éthyl-, perchlorate
1H-Pyrazolium, 4-[(2,3-dihydro-1,5-diméthyl-3-oxo-2-phényl-1H-pyrazol-4-yl)[4-(triméthylammonio) phényl]méthylène]-4,5-dihydro-2,3-diméthyl-5-oxo-1-phényl-, diperchlorate
1H-Pyrazolium, 4-[(2,3-dihydro-1,5-diméthyl-3-oxo-2-phéhyl-1H-pyrazol-4-yl)[4-(triméthylanmonio)phényl]méthylène]-4,5-dihydro-2,3-diméthyl-5-oxo-1-phéhyl-
5H-Benzo[a]carbazolium, 11-éthyl-5-[(11-éthyl-3-méthoxy-8-méthyl-11H-benzo[a] carbazol-5-yl)(4-nitrophényl)méthylène]-3-méthoxy-8-méthyl-, tétrafluoraborate
5H-Benzo[a]carbazolium, 11-éthyl-5-[(11-éthyl-3-méthoxy-8-méthyl-11H-benzo[a] carbazal-5-yl)(4-nitrophéthyl)méthylène]-3-méthoxy-8-méthyl-
5H-Benzo[a]carbazolium, 11-éthyl-5-[(11-éthyl-2-méthoxy-8-méthyl-11H-benzo[a]carbazol-5-yl)(4-nitrophényl)méthylène]-2-méthoxy-8-méthyl-, tétrafluoroborate
5H-Benzo[a]carbazolium, 11-éthyl-5-[(11-éthyl-2-méthoxy-8-méthyl-11H-benzo[a]carbazol-5-yl)(4-nitrophéthyl)méthylène]-2-méthoxy-8-méthyl-
5H-Benzo[a]carbazolium, 11-éthyl-5-[(11-éthyl-8-méthoxy-11H-benzo[a]carbazol-5-yl)(4-nitrophényl)méthylène]-8-méthoxy-, tétrafluoroborate
5H-Benzo[a]carbazoliun, 11-éthyl-5-[(11-éthyl-8-méthoxy-11H-benzo[a]carbazol-5-yl)(4-nitrophényl)méthylène]-8-méthoxy-
5H-Benzo[a]carbazolium, 11-éthyl-5-[(11-éthyl-4-méthoxy-11H-benzo[a]carbazol-5-yl)(4-nitrophényl)méthylène]-4-méthoxy-,tétrafluoroborate(
5H-Benzo[a]carbazolium, 11-éthyl-5-[(11-éthyl-4-méthoxy-11H-benzo[a]carbazol-5-yl)(4-nitrophényl)méthylène]-4-méthoxy-
5H-Benzo[a]carbazolium, 11-éthyl-5-[(11-éthyl-3-méthoxy-11H-benzo[a]carbazol-5-yl)(4-nitrophényl)méthylène]-3-méthoxy-, tétrafluoroborate
5H-Benzo[a]carbazolium, 11-éthyl-5-[(11-éthyl-3-méthoxy-11H-benzo[a]carbazol-5-yl)(4-nitrophényl)méthylène]-3-méthoxy-
5H-Benzo[a]carbazolium, 11-éthyl-5-[(11-éthyl-2-méthoxy-11H-benzo[a]carbazol-5-yl)(4-nitrophényl)méthylène]-2-méthoxy-,tétrafluoroborate
5H-Benzo[a]carbazolium, 11-éthyl-5-[(11-éthyl-2-méthoxy-11H-benzo[a]carbazol-5-yl)(4-nitrophényl)méthylène]-2-méthoxy-
5H-Benzo[a]carbazolium, 5-[(2-chloro-4-nitropényl)(11-éthyl-11H-benzo[a]carbazol-5-yl)méthylène]-11-éthyl-, sel avec l'acide trifluorométhanesulfonique (1:1)
5H-Benzo[a]carbazolium, 5-[(2-chloro-4-nitrophényl)(11-éthyl-11H-benzo[a]carbazal-5-yl)méthylène]-11-éthyl
5H-Benzo[a]carbazolium, 11-éthyl-5-[(11-éthyl-11H-benzo[a]carbazol-5-yl)(4-nitrophényl)méthylène]-, tétrafluoroborate
5H-Benzo[a]carbazolium, 11-éthyl-5-[(11-éthyl-11H-benzo[a]carbazol-5-yl)(4-nitrophényl)méthylène]-
5H-Benzo[a]carbazolium, 5-[[4-(diéthylamino)phényl](11-éthyl-11H-benzo[a]carbazol-5-yl)méthylène]-11-éthyl-, tétrafluoroborate(1-)
5H-Benzo[a]carbazolium, 5-[[4-(diéthylamino)phényl](11-éthyl-11H-benzo[a]carbazol-5-yl)méthylène]-11-éthyl-
5H-Benzo[a]carbazolium, 5-[[4-(diméthylamino)phényl](11-éthyl-11H-benzo[a]carbazol-5-yl)méthylène]-11-éthyl-, tétrafluoroborate(1-)
5H-Benzo[a]carbazolium, 5-[[4-(diméthylamino)phényl](11-éthyl-11H-benzo[a]carbazol-5-yl)méthylène]-11-éthyl-
Sel de 1H-Pyrazolium, 4-[(2,3-dihydro-1,5-diméthyl-3-2-phényl-1H-pyrazol-4-yl)[4-(diméthylamino)phényl]méthylène]-4,5-dihydro-2,3-diméthyl-5-oxo-1-phényl-et d'un monoacide
3H-Carbazolium, 3-[[4-[bis(phénylméthyl)amino]phényl](9-éthyl-9H-carbazol-3-yl)méthylène]-9-éthyl-, iodure
3H-Imidazo [1,2-a]benzimidazolium, 3-[[4-(diméthylamino)phényl](9-méthyl-2-phényl-9H-imidazo[1,2-a]benzimidazol-3-yl)méthylène]-9-méthyl-2-phényl-, chlorure
3H-Imidazo[1,2-a]benzimidazolium, 3-[[4-(diméthylamino)phényl](2,9-diméthyl-9H-imidazo[1,2-a]benzimidazol-3-yl)méthylène]-2,9-diméthyl-,bromure
1H-Pyrazolium, 4-[(2,3-dihydro-1,5-diméthyl-3-oxo-2-phényl-1H-pyrazol-4-yl)[4-(dipropylamino)phényl]méthylène]-4,5-dihydro-2,3-diméthyl-5-oxo-1-phényl-
1H-Pyrazolium, 4-[[4-(diéthylamino)phényl](2,3-dihydro-1,5-diméthyl-3-oxo-2-phényl-1H-pyrazol-4-yl)méthylène]-4,5-dihydro-2,3-diméthyl-5-oxo-1-phényl-
1H-Pyrazolium, 4-[(2,3-dihydro-1,5-diméthyl-3-oxo-2-phényl-1H-pyrazol-4-yl)(4-nitrophényl)méthylène]-4,5-dihydro-2,3-diméthyl-5-oxo-1-phényl-
1H-Pyrazolium, 4-[(2,3-dihydro-1,5-diméthyl-3-oxo-2-phényl-1H-pyrazol-4-yl)[4-(diméthylamino)phényl]méthylène]-4,5-dihydro-2,3-diméthyl-5-oxo-1-phényl-
1H-Pyrazolium, 4-[(4-aminophényl)(2,3-dihydro-1,5-diméthyl-3-phényl-1H-pyrazol-4-yl)méthylène]-2,3-diméthyl-5-oxo-1-phényl
3H-Indolizinium, 3-[[4-(diméthylamino)phényl](6-éthenyl-1,2-diméthyl-3-indolizinyl)méthylène]-6-éthenyl-1,2-diméthyl-
3H-Indolizinium, 3-[p-(diméthylamino)-α-(6-éthyl-1,2-diméthyl-3-indolizinyl)benzylidene]-6-éthyl-1,2-diméthyl-, iodure
3H-Indolizinium, 3-[p-(diméthylamino)-α-(1,2-diméthyl-6-vinyl-3-indolizinyl)benzylidene]-1,2-diméthyl-6-vinyl-, perchlorate
2-Pyrazolinium, 4-(p-amino-α-antipyrinylbenzylidene)-2,3-diméthyl-5-oxo-1-phényl-, perchlorate
2-Pyrazolinium, 4-(α-antipyrinyl-p-nitrobenzylidene)-2,3-diméthyl-5-oxo-1-phényl-, perchlorate
2-Pyrazolinium, 4-[α-antipyrinyl-p-(diéthylamino)benzylidene]-2,3-diméthyl-5-oxo-1-phényl-, perchlorate
2-Pyrazolinium, 4-[α-antipyrinyl-p-(diméthylamino)benzylidene]-2,3-diméthyl-5-oxo-1-phényl-, perchlorate
Méthylium, bis[6-(diméthylamino)-3-méthyl-1H-indol-2-yl][4-(diméthylamino)phényl]-, perchlorate
Méthylium, bis[6-(diméthylamino)-3-méthyl-1H-indol-2-yl][4-(diméthylamino)phényl]-
Méthylium, bis[6-(diméthylamino)-3-méthyl-1H-indol-2-yl](4-nitrophényl)-, perchlorate
Méthylium, bis[6-(diméthylamino)-3-méthyl-1H-indol-2-yl](4-nitrophényl)-
Méthylium, bis(benzo[b]thién-2-yl)[4-(diméthylamino)phényl]-, tétrafluoroborate
Méthylium, bis(benzo[b]thién-2-yl)[4-(diméthylamino)phényl]-
Méthylium, [4-(diméthylamino)phényl]bis(1-méthyl-1H-pyrrol-2-yl)-, tétrafluoroborate
Méthylium, [4-(diméthylamino)phényl]bis(1-méthyl-1H-pyrrol-2-yl)-Diantipyrinyl(p-nitrophényl)méthylium perchlorate
Méthylium, bis(9,10-dihydro-9,9,10-triméthyl-3-acridinyl)[4-(diméthylamino)phényl]-, chlorure
Méthylium, [4-(diméthylamino)phényl]bis(10-méthyl-10H-phenothiazin-2-yl)-, chlorure
3H-Pyrazol-3-one, 4,4'-[[4-(diméthylamino)phényl]méthylène]bis[1,2-dihydro-1,5-diméthyl-2-phényl-, mono[tétrafluoroborate(1-)]
3H-Pyrazol-3-one, 4,4'-[[4-(diméthylamino)phényl]méthylène]bis[1,2-dihydro-1,5-diméthyl-2-phényl-, bis[tétrafluoroborate(1-)]
Sel de 3H-Pyrazol-3-one, 4,4'-[[4-(diméthylamino)phényl]méthylène]bis[1,2-dihydro-1,5-diméthyl-2-phenyl-et d'un diacide
Sel de 3H-Pyrazol-3-one, 4,4'-[[4-(diméthylamino)phényl]méthylène]bis[1,2-dihydro-1,5-diméthyl-2-phényl-,et d'un monoacide
Quinolinium, 4,4'-[(4-nitrophényl)méthylène]bis[1-éthyl-, dichlorure
Méthylium, bis(9-éthyl-9H-carbazol-3-yl)(4-nitrophényl)-, indure
Méthylium, [4-(diméthylamino)phényl]bis(9-éthyl-9H-carbazol-3-yl)-, indure Méthylium, 9H-carbazol-3-yl[4-(diméthylamino)phényl](6-méthyl-9H-carbazol-3-yl)-
Quinolizinium, 2,2'-[(4-nitrophényl)méthylène]bis[1,2,3,4-tétrahydro-1-oxo-Méthylium, 9H-carbazol-3-yl[4-(diméthylamino)phényl](6-méthyl-9H-carbazal-3-yl)-, tétrafluoroborate
6,6'-Dichloro-9,9'-dibutyl-3,3'-dicarbazolyl-p-nitrophénylméthyl Chlorure
Quinolizinium, 2,2'-(p-nitrobenzylidene)bis[1,2,3,4-tétrahydro-1-oxo-, diperchlorate
Benzylium, p-(diéthylamino)-α,α-bis(2,3-diméthyl-5-oxo-1-phényl-3-pyrazolin-4-yl)-
Benzylium, α,α-diantipyrinyl-p-(diméthylamino)-
Benzylium, α,α-bis(2,3-diméthyl-5-oxo-1-phényl-3-pyrazolin-4-yl)-p-nitro-Benzylium, p-amino-α,α-di-4-antipyrinyl-.

20. Composition selon l'une quelconque des revendications 18 or 19, **caractérisée en ce que** le ou les composés de formule (I), (Ibis), (II) et (IIbis) et/ou le ou les sel(s) d'addition représentent de 0,0001 à 10 % en poids du poids total de la composition de teinture, de préférence de 0,005 à 10 % en poids et, de préférence encore, de 0,01 à 6 % en poids du poids total de la composition de teinture.

21. Composition selon l'une quelconque des revendications 18 à 20, **caractérisée en ce qu'**elle contient, en outre, un ou plusieurs colorant(s) direct(s) différent(s) des composés de formule (I), (Ibis), (II) et (IIbis).

22. Composition selon la revendication 21, **caractérisée en ce que** le ou les colorant(s) direct(s) différent(s) des composés de formule (I), (Ibis), (II) et (IIbis) sont choisis parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

23. Composition selon l'une quelconque des revendications 21 et 22, **caractérisée en ce que** le ou les colorant(s) direct(s) différents des composés (I), (Ibis), (II) et (IIbis), représentent de 0,001 à 20 % en poids, de préférence de 0,005 à 10 % en poids du poids total de la composition de teinture.

24. Composition selon l'une quelconque des revendications 20 à 23, **caractérisée en ce qu'**elle contient, en outre, une ou plusieurs base(s) d'oxydation et éventuellement un ou plusieurs coupleur(s).

25. Composition selon la revendication 24, **caractérisé en ce que** la ou les base (s) d'oxydation sont choisies parmi les paraphénylènediamines, les bis-phénylalkylènediamines, les para-aminophénols, les ortho-aminophénols, les bases hétérocycliques et leurs sels d' addition.

26. Composition selon l'une quelconque des revendications 24 et 25, **caractérisée en ce que** le ou les coupleur(s) sont choisi(s) parmi les coupleurs métaphénylènediamines, les coupleurs méta-aminophénols, les coupleurs métadiphénols, les coupleurs naphtaléniques, les coupleurs hétérocycliques et leurs sels d'addition.

27. Composition selon l'une quelconque des revendications 24 à 26, **caractérisée en ce que** le ou les base(s) d'oxydation et/ou le ou les coupleur(s) sont présents chacun en une quantité de 0,001 à 10 % en poids, de préférence de 0,005 à 6 % en poids du poids total de la composition de teinture.

## Claims

1. Use of a composition including, in a cosmetic medium suitable for dyeing, at least one dye chosen from among the compounds according to the following formula (I), (Ibis), (Iter), (II) or (IIbis) or their tautomeric forms, and their addition salts: 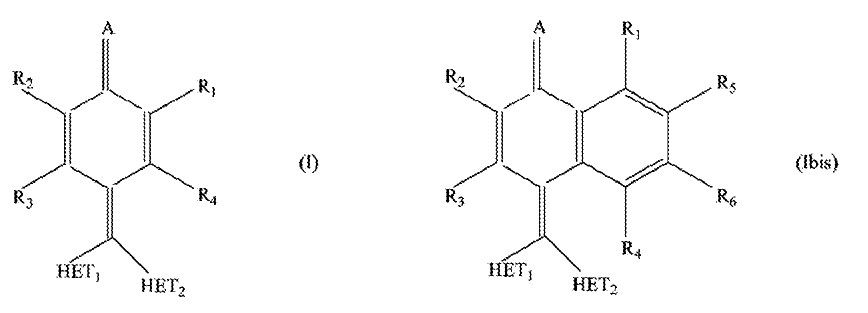 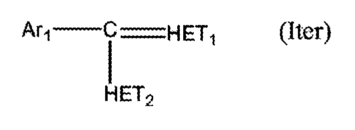 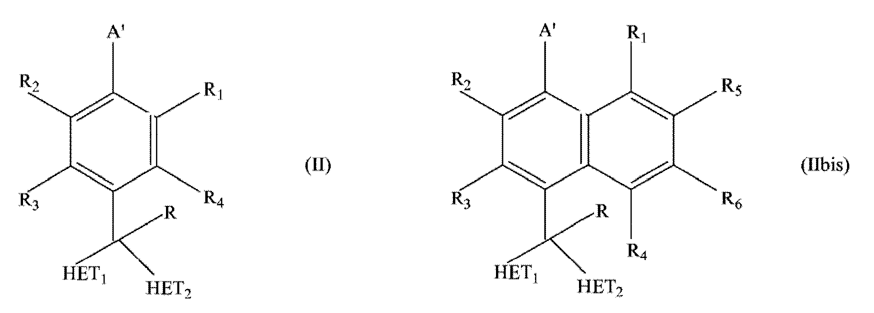 in which:
- A represents O; NH; N-alkyl N-hydroxyalkyl; ammonium ; N-alkylammonium ; N-(hydroxyalkyl) ammonium ; N,N-dialkylammonium, N,N-di(hydroxyalkyl) ammonium, N-(hydroxyalkyl) N-(alkyl) ammonium, in which the two alkyl groups may form, together with the nitrogen atom to which they are linked, a cycle that may be interrupted by one or several atoms of nitrogen, oxygen or sulphur ;
- HET₁, HET₂ independently from each other represent an aromatic heterocycle, optionally substituted with one or several groups Z';
- Ar₁ represents an aryl radical, optionally substituted with one or several groups Z ;
- R₁ to R₆ and Z, Z' independently from each other represent a group chosen from among an hydrogen atom, the halogen atoms, such as F, Cl, Br and I, and the - NHSO₃H; hydroxyl; alkyl; alcoxy; alkylthio; amino; monoalkylamino; dialkylamino radicals, in which the two alkyl groups may, together with the nitrogen atom to which they are linked, form a cycle that may be interrupted by one or several atoms of nitrogen, oxygen or sulphur ; the heterocycles and the nitro; aryl; acyl; alcoxycarbonyl; carboxamido; cyano; -CO₂H; -SO₃H; -PO₃H_{2;} -PO₄H₂; radicals;
- A' represents an hydrogen atom, an hydroxyl, amino, (hydroxyalkyl)amino, di(hydroxyalkyl)amino, (hydroxyalkyl)(alkyl)amino, monoalkylamino, dialkylamino group, in which the two alkyl groups may form, with the nitrogen atom to which they are linked, a cycle that may to be interrupted by one or several atoms of nitrogen, oxygen or sulphur;
- R represents an atom of hydrogen or halogen; a hydroxyl radical; an alcoxy radical; an alkylthio radical ; for dyeing keratin fibres.

2. Use according to claim 1, wherein the heterocycles, and particularly those of HET₁, and HET₂, are chosen from among the following heterocycles: thiophene, benzothiophene, furane, benzofurane, indole, indoline, carbazole, pyridine, dehydroquinoline, chromone, julolidine, thiadiazole, triazole, isoxazole, oxazole, thiazole, isothiazole, imidazole, pyrazole, triazine, thiazine, pyrazine, pyridazine, pyrimidine, pyridine, diazepine, oxazepine, benzotriazole, benzoxazole, benzimidazole, benzothiazole, morpholine, piperidine, piperazine, azetidine, pyrrolidine, aziridine.

3. Use according to claim 1, **characterised in that** the composition comprises a compound of formula (I), (Ibis), (Iter), (II) or (IIbis) chosen from among the following compounds for which the counter-ions are specified or not, and their addition salts:
4-[Bis-(2-methyl-1H-indol-3-yl)-methylene]-cyclohexa-2,5-dienylidene}-dimethylammonium
1,2,2,4-Tetramethyl-6-[phenyl-(1,2,2,4-tetramethyl-1,2,3,4-tetrahydro-quinolin-6-yl)-methylene]-2,3,4,6-tetrahydroquinolinium chloride
1,2,2,3-Tetramethyl-5-[(1-methyl-2-phenyl-1H-indol-3-yl)-phenyl-methylene]-3,5-dihydro-2H-indolinium chloride
9-Ethyl-3-[(9-ethyl-9H-carbazol-3-yl)-phenyl-methylene]-3H-carbazolium chloride
1-Ethyl-3-[(1-ethyl-2-methyl-1H-indol-3-yl)-p-tolyl-methylene]-2-methyl-3a,7a-dihydro-3H-indolium
6-{(4-Nitro-phenyl)-[1-(4-sulfo-butyl)-1,2,3,4-tetrahydro-quinolin-6-yl]-methylen}-1-(4-sulfo-butyl)-2,3,4,6-tetrahydro-quinolinium
6-{(4-Cyano-phenyl)-[1-(4-sulfo-butyl)-1,2,3,4-tetrahydro-quinolin-6-yl]-methylen}-1-(4-sulfo-butyl)-2,3,4,6-tetrahydro-quinolinium
Diethyl-{4-[(2-methyl-1H-indol-3-yl)-thiophen-2-yl-methylene]-cyclohexa-2,5-dienylidene}-ammonium
1-Ethyl-3-[(1-ethyl-2-methyl-1H-indol-3-yl)-(4-methoxy-phenyl)-methylene]-2-methyl-3a,7a-dihydro-3H-indolium
Ethanaminium, N-[4-[(3-carboxypyrazinyl)(1-ethyl-2-methyl-1H-indol-3-yl)methylene]-3-ethoxy-2,5-cyclohexadien-1-ylidene]-N-ethyl-, internal salt
Ethanaminium, N-[4-[(3-carboxypyrazinyl)(1-ethyl-2-methyl-1H-indol-3-yl)methylene]-3-ethoxy-2,5-cyclohexadien-1-ylidene]-N-ethyl-, chloride
Cyclohepta[b]furylium, 3-[[4-(dimethylamino)phenyl](2-oxo-2H-cyclohepta[b]furan-3-yl)methylene]-2,3-dihydro-2-oxo
Methanaminium, N-[4-[bis(1,2,3,4,10,14b-hexahydro-2-methyldibenz[c,f]pyrazino[1,2-a]azepin-8-yl) methylene]-2,5-cyclohexadien-1-ylidene]-N-methyl-, perchlorate, diperchlorate
Methanaminium, N-[4-[bis(1,2,3,4,10,14b-hexahydro-2-methyldibenzo [c,f]pyrazino[1,2-a]azepin-8-yl)methylene]-2,5-cyclohexadien-1-ylidene]-N-methyl-, perchlorate
Methanaminium, N-[4-[bis(1,2,3,4,10,14b-hexahydro-2-methyldibenzo [c,f]pyrazino[1,2-a]azepin-8-yl)methylene]-2,5-cyclohexadien-1-ylidene]-N-methyl-
Methanaminium, N-[4-[bis(2,3-dihydro-1,5-dimethyl-3-oxo-2-phenyl-1H-pyrazol-4-yl)methylene]-2,5-cyclohexadien-1-ylidene]-N-methyl-
Morpholinium, 4-[5-[[4-(dimethylamino)phenyl][5-(4-morpholinyl)-2-thienyl]methylene]-2(5H)-thienylidene]-, perchlorate
Morpholinium, 4-[5-[[4-(dimethylamino)phenyl][5-(4-morpholinyl)-2-thienyl]methylene]-2(5H)-thienylidene]-
1H-Indolizinium, 7-(dimethylamino)-1-[[7-(dimethylamino)-3- (ethoxycarbonyl)-1-indolizinyl][4-(dimethylamino)phenyl]methylene]-3- (ethoxycarbonyl)-, perchlorate
1H-Indolizinium, 7-(dimethylamino)-1-[[7-(dimethylamino)-3- (ethoxycarbonyl)-1-indolizinyl][4-(dimethylamino)phenyl]methylene]-3- (ethoxycarbonyl)-
Cyclohepta[b]pyrrolium, 3-[(1,2-dihydro-2-oxo-1-phenylcyclohepta[b]pyrrol-3-yl)[4-(dimethylamino)phenyl]methylene]-2,3-dihydro-2-oxo-1-phenyl-, hexafluorophosphate
Cyclohepta[b]pyrrolium, 3-[(1,2-dihydro-2-oxo-1-phenylcyclohepta[b]pyrrol-3-yl)[4-(dimethylamino)phenyl]methylene]-2,3-dihydro-2-oxo-1-phenyl-
Quinolinium, 6-[[4-(dimethylamino)phenyl](1,2,3,4-tetrahydro-1,2,2,4-tetramethyl-6-quinolinyl)methylene]-2,3,4,6-tetrahydro-1,2,2,4-tetraméthyl-, chloride
Quinolinium, 6-[(1,2-dihydro-1,2,2,4-tetramethyl-6-quinolinyl)[4-(dimethylamino)phenyl]methylene]-2,6-dihydro-1,2,2,4-tetramethyl-,chloride
3H-Carbazolium, 9-ethyl-3-[(9-ethyl-9H-carbazol-3-yl)[4-(methylphenylamino)phenyl]methylene]-, tetrafluoroborate(1-)
3H-Carbazolium, 3-[[4-(dimethylamino)phenyl](9-ethyl-9H-carbazol-3-yl)methylene]-9-ethyl-, tetrafluoroborate(1-)
3H-Carbazolium, 9-ethyl-3-[(9-ethyl-9H-carbazol-3-yl)[4- (methylphenylamino) phenyl]methylene]-, perchlorate
3H-Carbazolium, 9-ethyl-3-[(9-ethyl-9H-carbazol-3-yl)[4- (methylphenylamino) phenyl]methylene]-, bromide
3H-Carbazolium, 9-ethyl-3-[(9-ethyl-9H-carbazol-3-yl)[4- (methylphenylamino) phenyl]methylene]-, chloride
3H-Carbazolium, 3-[[4-(dimethylamino)phenyl](9-ethyl-9H-carbazol-3-yl)methylene]-9-ethyl-, bromide
3H-Carbazolium, 3-[[4-(dimethylamino)phenyl](9-ethyl-9H-carbazol-3-yl)methylene]-9-ethyl-, chloride
3H-Carbazolium, 9-ethyl-3-[(9-ethyl-9H-carbazol-3-yl)[4- (methylphenylamino) phenyl]methylene]-, iodide
3H-Carbazolium, 3-[[4-(dimethylamino)phenyl](9-ethyl-9H-carbazol-3-yl)methylene]-9-ethyl-, iodide
3H-Carbazolium, 3-[[4-(dimethylamino)phenyl](9-ethyl-9H-carbazol-3-yl)methylene]-9-éthyl-, perchlorate
1H-Pyrazolium, 4-[(2,3-dihydro-1,5-dimethyl-3-oxo-2-phenyl-1H-pyrazol-4-yl)[4-(trimethylammonio) phenyl]methylene]-4,5-dihydro-2,3-dimethyl-5-oxo-1-phenyl-, diperchlorate
1H-Pyrazolium, 4-[(2,3-dihydro-1,5-dimethyl-3-oxo-2-phenyl-1H-pyrazol-4-yl)[4-(trimethylammonio)phenyl]methylene]-4,5-dihydro-2,3-dimethyl-5-oxo-1-phenyl-
5H-Benzo[a]carbazolium, 11-ethyl-5-[(11-ethyl-3-methoxy-8-methyl-11H-benzo[a] carbazol-5-yl)(4-nitrophenyl)methylene]-3-methoxy-8-methyl-, tetrafluoroborate
5H-Benzo[a]carbazolium, 11-ethyl-5-[(11-ethyl-3-methoxy-8-methyl-11H-benzo[a] carbazol-5-yl)(4-nitrophenyl)methylene]-3-methoxy-8-methyl-
5H-Benzo[a]carbazolium, 11-ethyl-5-[(11-ethyl-2-methoxy-8-methyl-11H-benzo[a]carbazol-5-yl)(4-nitrophenyl)methylene]-2-methoxy-8-methyl-, tetrafluoroborate
5H-Benzo[a]carbazolium, 11-ethyl-5-[(11-ethyl-2-methoxy-8-methyl-11H-benzo[a]carbazol-5-yl)(4-nitrophenyl)methylene]-2-methoxy-8-methyl-
5H-Benzo[a]carbazolium, 11-ethyl-5-[(11-ethyl-8-methoxy-11H-benzo[a]carbazol-5-yl)(4-nitrophenyl)methylene]-8-methoxy-,tetrafluoroborate
5H-Benzo[a]carbazolium, 11-ethyl-5-[(11-ethyl-8-methoxy-11H-benzo[a]carbazol-5-yl)(4-nitrophenyl)methylene]-8-methoxy-
5H-Benzo[a]carbazolium, 11-ethyl-5-[(11-ethyl-4-methoxy-11H-benzo[a]carbazol-5-yl)(4-nitrophenyl)methylene]-4-methoxy-,tetrafluoroborate(
5H-Benzo[a]carbazolium, 11-ethyl-5-[(11-ethyl-4-methoxy-11H-benzo[a]carbazol-5-yl)(4-nitrophenyl)methylene]-4-methoxy-
5H-Benzo[a]carbazolium, 11-ethyl-5-[(11-ethyl-3-methoxy-11H-benzo[a]carbazol-5-yl)(4-nitrophenyl)methylene]-3-methoxy-,tetrafluoroborate
5H-Benzo[a]carbazolium, 11-ethyl-5-[(11-ethyl-3-methoxy-11H-benzo[a]carbazol-5-yl)(4-nitrophenyl)methylene]-3-methoxy-
5H-Benzo[a]carbazolium, 11-ethyl-5-[(11-ethyl-2-methoxy-11H-benzo[a]carbazol-5-yl)(4-nitrophenyl)methylene]-2-methoxy-,tetrafluoroborate
5H-Benzo[a]carbazolium, 11-ethyl-5-[(11-ethyl-2-methoxy-11H-benzo[a]carbazol-5-yl)(4-nitrophenyl)methylene]-2-methoxy-
5H-Benzo[a]carbazolium, 5-[(2-chloro-4-nitrophenyl)(11-ethyl-11H-benzo[a]carbazol-5-yl)methylene]-11-ethyl-, salt with trifluoromethanesulfonic acid (1:1)
5H-Benzo[a]carbazolium, 5-[(2-chloro-4-nitrophenyl)(11-ethyl-11H-benzo[a]carbazol-5-yl)methylene]-11-ethyl
5H-Benzo[a]carbazolium, 11-ethyl-5-[(11-ethyl-11H-benzo[a]carbazol-5-yl)(4-nitrophenyl)methylene]-, tetrafluoroborate
5H-Benzo[a]carbazolium, 11-ethyl-5-[(11-ethyl-11H-benzo[a]carbazol-5-yl)(4-nitrophenyl)methylene]-
5H-Benzo[a]carbazolium, 5-[[4-(diethylamino)phenyl](11-ethyl-11H-benzo[a]carbazol-5-yl)methylene]-11-ethyl-, tetrafluoroborate(1-)
5H-Benzo[a]carbazolium, 5-[[4-(diethylamino)phenyl](11-ethyl-11H-benzo[a]carbazol-5-yl)methylene]-11-ethyl-
5H-Benzo[a]carbazolium, 5-[[4-(dimethylamino)phenyl](11-ethyl-11H-benzo[a]carbazol-5-yl)methylene]-11-ethyl-, tetrafluoroborate(1-)
5H-Benzo[a]carbazolium, 5-[[4-(dimethylamino)phenyl](11-ethyl-11H-benzo[a]carbazol-5-yl)methylene]-11-ethyl-
Salt of 1H-Pyrazolium, 4-[(2,3-dihydro-1,5-dimethyl-3-oxo-2-phenyl-1H-pyrazol-4-yl)[4-(dimethylamino)phenyl]methylene]-4,5-dihydro-2,3-dimethyl-5-oxo-1-phenyl-and a monoacid
3H-Carbazolium, 3-[[4-[bis(phenylmethyl)amino]phenyl](9-ethyl-9H-carbazol-3-yl)methylène]-9-ethyl-, iodide
3H-Imidazo[1,2-a]benzimidazolium, 3-[[4-(dimethylamino)phenyl](9-methyl-2-phenyl-9H-imidazo[1,2-a]benzimidazol-3-yl)methylene]-9-methyl-2-phenyl-, chloride
3H-Imidazo[1,2-a]benzimidazolium, 3-[[4-(dimethylamino)phenyl](2,9-dimethyl-9H-imidazo[1,2-a]benzimidazol-3-yl)methylene]-2,9-dimethyl-,bromide
1H-Pyrazolium, 4-[(2,3-dihydro-1,5-dimethyl-3-oxo-2-phenyl-1H-pyrazol-4-yl)[4-(dipropylamino)phenyl]methylene]-4,5-dihydro-2,3-dimethyl-5-oxo-1-phenyl-
1H-Pyrazolium, 4-[[4-(diethylamino)phenyl](2,3-dihydro-1,5-dimethyl-3-oxo-2-phenyl-1H-pyrazol-4-yl)methylene]-4,5-dihydro-2,3-dimethyl-5-oxo-1-phenyl-
1H-Pyrazolium, 4-[(2,3-dihydro-1,5-dimethyl-3-oxo-2-phenyl-1H-pyrazol-4-yl)(4-nitrophenyl)methylene]-4,5-dihydro-2,3-dimethyl-5-oxo-1-phenyl-
1H-Pyrazolium, 4-[(2,3-dihydro-1,5-dimethyl-3-oxo-2-phenyl-1H-pyrazol-4-yl)[4-(dimethylamino)phenyl]methylene]-4,5-dihydro-2,3-dimethyl-5-oxo-1-phenyl-
1H-Pyrazolium, 4-[(4-aminophenyl)(2,3-dihydro-1,5-dimethyl-3-oxo-2-phenyl-1H-pyrazol-4-yl)methylene]-2,3-dimethyl-5-oxo-1-phenyl
3H-Indolizinium, 3-[[4-(dimethylamino)phenyl](6-ethenyl-1,2-dimethyl-3-indolizinyl)methylene]-6-ethenyl-1,2-dimethyl-
3H-Indolizinium, 3-[p-(dimethylamino)-α-(6-ethyl-1,2-dimethyl-3-indolizinyl)benzylidene]-6-ethyl-1,2-dimethyl-, iodide
3H-Indolizinium, 3-[p-(dimethylamino)-α-(1,2-dimethyl-6-vinyl-3-indolizinyl)benzylidene]-1,2-dimethyl-6-vinyl-, perchlorate
2-Pyrazolinium, 4-(p-amino-α-antipyrinylbenzylidene)-2,3-dimethyl-5-oxo-1-phenyl-, perchlorate
2-Pyrazolinium, 4-(α-antipyrinyl-p-nitrobenzylidene)-2,3-dimethyl-5-oxo-1-phenyl-, perchlorate
2-Pyrazolinium, 4-[α-antipyrinyl-p-(diethylamino)benzylidene]-2,3-dimethyl-5-oxo-1-phenyl-, perchlorate
2-Pyrazolinium, 4-[α-antipyrinyl-p-(dimethylamino)benzylidene]-2,3-dimethyl-5-oxo-1-phenyl-, perchlorate
Methylium, bis[6-(dimethylamino)-3-methyl-1H-indol-2-yl][4-(dimethylamino)phenyl]-, perchlorate
Methylium, bis[6-(dimethylamino)-3-methyl-1H-indol-2-yl][4-(dimethylamino)phenyl]-
Methylium, bis[6-(dimethylamino)-3-methyl-1H-indol-2-yl](4-nitrophenyl)-, perchlorate
Methylium, bis[6-(dimethylamino)-3-methyl-1H-indol-2-yl](4-nitrophenyl)-
Methylium, bis(benzo[b]thien-2-yl)[4-(dimethylamino)phenyl]-, tetrafluoroborate
Methylium, bis(benzo[b]thien-2-yl)[4-(dimethylamino)phenyl]-
Methylium, [4-(dimethylamino)phenyl]bis(l-methyl-1H-pyrrol-2-yl)-, tetrafluoroborate
Methylium, [4-(dimethylamino)phenyl]bis(1-methyl-1H-pyrrol-2-yl)-Diantipyrinyl(p-nitrophenyl)methylium perchlorate
Methylium, bis(9,10-dihydro-9,9,10-trimethyl-3-acridinyl)[4-(dimethylamino)phenyl]-, chloride
Methylium, [4-(dimethylamino)phenyl]bis(10-methyl-10H-phenothiazin-2-yl)-, chloride
3H-Pyrazol-3-one, 4,4'-[[4-(dimethylamino)phenyl]methylene]bis[1,2-dihydro-1,5-dimethyl-2-phenyl-, mono[tetrafluoroborate(1-)]
3H-Pyrazol-3-one, 4,4'-[[4-(dimethylamino)phenyl]methylene]bis[1,2-dihydro-1,5-dimethyl-2-phenyl-, bis[tetrafluoroborate(1-)]
Salt of 3H-Pyrazol-3-one, 4,4'-[[4-(dimethylamino)phenyl]methylene]bis[1,2-dihydro-1,5-dimethyl-2-phenyl-and a diacid
Salt of 3H-Pyrazol-3-one, 4,4'-[[4-(dimethylamino)phenyl]methylene]bis[1,2-dihydro-1,5-dimethyl-2-phenyl-, and a monoacid
Quinolinium, 4,4'-[(4-nitrophenyl)methylene]bis[1-ethyl-, dichloride
Methylium, bis(9-ethyl-9H-carbazol-3-yl)(4-nitrophenyl)-, iodide
Methylium, [4-(dimethylamino)phenyl]bis(9-ethyl-9H-carbazol-3-yl)-, iodide
Methylium, 9H-carbazol-3-yl[4-(dimethylamino)phenyl](6-methyl-9H-carbazol-3-yl)-
Quinolizinium, 2,2'-[(4-nitrophenyl)methylene]bis[1,2,3,4-tetrahydro-1-oxo-
Methylium, 9H-carbazol-3-yl[4-(dimethylamino)phenyl](6-methyl-9H-carbazol-3-yl)-, tetrafluoroborate
6,6'-Dichloro-9,9'-dibutyl-3,3'-dicarbazolyl-p-nitrophenylmethyl chloride
Quinolizinium, 2,2'-(p-nitrobenzylidene)bis[1,2,3,4-tétrahydro-1-oxo-, diperchlorate
Benzylium, p-(diethylamino)-α,α-bis(2,3-dimethyl-5-oxo-1-phenyl-3-pyrazolin-4-yl)-
Benzylium, α,α-diantipyrinyl-p-(dimethylamino)-
Benzylium, α,α-bis(2,3-dimethyl-5-oxo-1-phenyl-3-pyrazolin-4-yl)-p-nitro-
Benzylium, p-amino-α,α-di-4-antipyrinyl-.

4. Use according to any one of the preceding claims, **characterised in that** the compound or compounds of formula (I), (Ibis), (Iter), (II) and (IIbis) and/or their additive salt(s) represent from 0.0001 to 10% by weight of the total weight of the dyeing composition, preferably from 0.005 to 10% by weight and, even more preferably, from 0.01 to 6% by weight of the total weight of the dye composition.

5. Use according to any one of claims 1 to 4, **characterised in that** the composition further contains one or several direct dye(s) different from the compounds of formula (I), (Ibis), (Iter), (II) or (IIbis).

6. Use according to claim 5, **characterised in that** the direct dye(s) different from the compounds of formula (I), (Ibis), (Iter), (II) and (IIbis) are chosen from among the neutral, acid or cationic benzenic nitrated direct dyes, the neutral, acid or cationic azoic direct dyes, the neutral, acid or cationic quinonic direct dyes, the azinic direct dyes, the triarylmethanic direct dyes, the indoaminic direct dyes and the natural direct dyes.

7. Use according to any one of claims 5 and 6, **characterised in that** the direct dye(s) different from the compounds (I), (Ibis), (Iter), (II) and (IIbis), represent from 0.001 to 20% by weight, preferably from 0.005 to 10% by weight of the total weight of the dye composition.

8. Use according to any one of the preceding claims, **characterised in that** the composition further contains one or several oxidation base(s) and optionally one or several coupler(s).

9. Use according to claim 8, **characterised in that** the oxidation base or bases are chosen from among the paraphenylenediamines, the bis-phenylalkylenediamines, the para-aminophenols, the ortho-aminophenols, the heterocyclic bases and their addition salts.

10. Use according to any one of claims 8 and 9, **characterised in that** the coupler(s) are chosen from among the metaphenylenediamine couplers, the meta-aminophenol couplers, the metadiphenol couplers, the naphthalenic couplers, the heterocyclic couplers and their addition salts.

11. Use according to any one of claims 8 to 10, **characterised in that** the oxidation base or bases and/or the coupler(s) are each present in an amount from 0.001 to 10% by weight, preferably from 0.005 to 6% by weight of the total weight of the dye composition.

12. Use according to any one of the preceding claims, **characterised in that** the composition has a pH of from 3 to 12, preferably from 5 to 11, and even more preferably from 6 to 8.5.

13. Method for the direct dyeing of keratin fibres, **characterised in that** it comprises the application on the keratin fibres of at least one dyeing composition as defined in any one of claims 1 to 12 containing at least one compound of formula (I), (Ibis), (Iter), (II) or (IIbis), and then respecting a pause time followed by rinsing of the fibres.

14. Method according to claim 13, **characterised in that** the pause time is from 3 to 50 minutes, preferably from 5 to 30 minutes.

15. Method for dyeing keratin fibres, in which at least one dye composition as defined in any one of claims 1 to 12, is applied on said fibres, said composition comprising either at least one compound of formula (II) or (IIbis), or at least one compound of formula (I), (Ibis) or (Iter) and at least one oxidation base and optionally one or several coupler(s), the colour being developed at acid, neutral or alkaline pH with the aid of an oxidizing agent.

16. Method according to claim 15, **characterised in that** the oxidizing agent is chosen from among hydrogen peroxide, urea peroxide, the alkaline metal bromates, the persalts such as the perborates and persulphates, the peracids and the oxidase enzymes such as the peroxydases, the oxydo-reductases with 2 electrons such as the uricases and the oxygenases with 4 electrons such as the laccases.

17. Device with several compartments, comprising a first compartment containing a dyeing composition as defined in any one of claims 1 to 12, comprising either at least one compound of formula (II) or (IIbis) or at least one compound of formula (I), (Ibis) or (Iter) and at least one oxidation base, and optionally one or several coupler(s), and a second compartment enclosing an oxidizing composition.

18. Composition for dyeing keratin fibres including, in a cosmetic medium suitable for dyeing, at least one dye chosen from among the compounds according to the following formula (I), (Ibis), (Iter), (II) or (IIbis) or their tautomeric forms, and their addition salts: 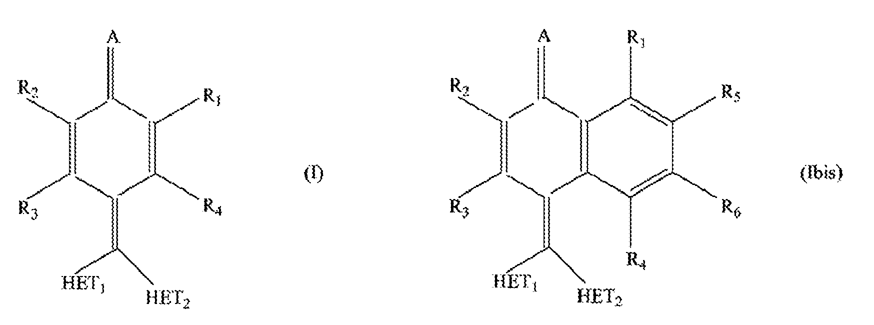 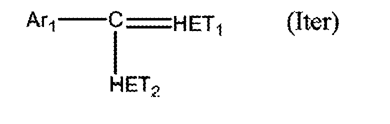 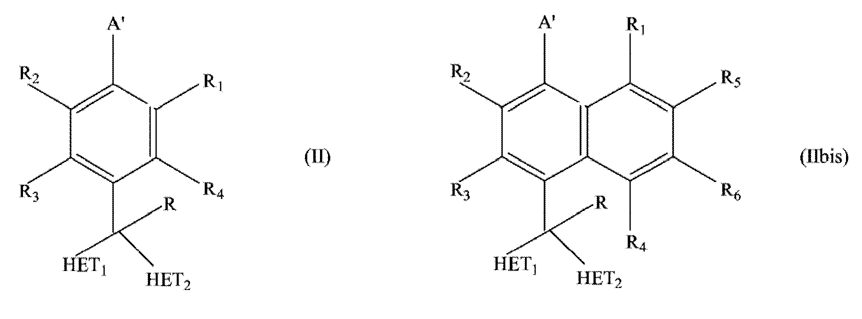 in which:
- A represents 0; NH; N-alkyl N-hydroxyalkyl; ammonium ; N-alkylammonium ; N-(hydroxyalkyl) ammonium ; N,N-dialkylammonium, N,N-di(hydroxyalkyl) ammonium, N-(hydroxyalkyl) N-(alkyl) ammonium, in which the two alkyl groups may form, together with the nitrogen atom to which they are linked, a cycle that may be interrupted by one or several atoms of nitrogen, oxygen or sulphur ;
- HET₁, HET₂ independently from each other represent an aromatic heterocycle, optionally substituted with one or several groups Z';
- Ar₁ represents an aryl radical, optionally substituted with one or several groups Z ;
- R₁ to R₆ and Z, Z' independently from each other represent a group chosen from among an hydrogen atom, the halogen atoms, such as F, Cl, Br and I, and the - NHSO₃H; hydroxyl; alkyl; alcoxy; alkylthio; amino; monoalkylamino; dialkylamino radicals, in which the two alkyl groups may, together with the nitrogen atom to which they are linked, form a cycle that may be interrupted by one or several atoms of nitrogen, oxygen or sulphur ; the heterocycles and the nitro; aryl; acyl; alcoxycarbonyl; carboxamido; cyano; -CO₂H; -SO₃H; -PO₃H₂; -PO₄H₂; radicals;
- A' represents an hydrogen atom, an hydroxyl, amino, (hydroxyalkyl)amino, di(hydroxyalkyl)amino, (hydroxyalkyl)(alkyl)amino, monoalkylamino, dialkylamino group, in which the two alkyl groups may form, with the nitrogen atom to which they are linked, a cycle that may to be interrupted by one or several atoms of nitrogen, oxygen or sulphur;
- R represents an atom of hydrogen or halogen; a hydroxyl radical; an alcoxy radical; an alkylthio radical ; said composition having a pH from 6 to 12.

19. Composition for dyeing according to claim 18, **characterised in that** the composition comprises a compound of formula (I), (Ibis), (Iter), (II) or (IIbis) chosen from among the following compounds for which the counter-ions are specified or not, and their addition salts:
4-[Bis-(2-methyl-1H-indol-3-yl)-methylene]-cyclohexa-2,5-dienylidene}-dimethylammonium
1,2,2,4-Tetramethyl-6-[phenyl-(1,2,2,4-tetramethyl-1,2,3,4-tetrahydro-quinolin-6-yl)-methylene]-2,3,4,6-tetrahydroquinolinium chloride
1,2,2,3-Tetramethyl-5-[(1-methyl-2-phenyl-1H-indol-3-yl)-phenyl-methylene]-3,5-dihydro-2H-indolinium chloride
9-Ethyl-3-[(9-ethyl-9H-carbazol-3-yl)-phenyl-methylene]-3H-carbazolium chloride
1-Ethyl-3-[(1-ethyl-2-methyl-1H-indol-3-yl)-p-tolyl-methylene]-2-methyl-3a,7a-dihydro-3H-indolium
6-{(4-Nitro-phenyl)-[1-(4-sulfo-butyl)-1,2,3,4-tetrahydro-quinolin-6-yl]-methylen}-1-(4-sulfo-butyl)-2,3,4,6-tetrahydro-quinolinium
6-{(4-Cyano-phenyl)-[1-(4-sulfo-butyl)-1,2,3,4-tetrahydro-quinolin-6-yl]-methylen}-1-(4-sulfo-butyl)-2,3,4,6-tetrahydro-quinolinium
Diethyl-{4-[(2-methyl-1H-indol-3-yl)-thiophen-2-yl-methylene]-cyclohexa-2,5-dienylidene}-ammonium
1-Ethyl-3-[(1-éthyl-2-methyl-1H-indol-3-yl)-(4-methoxy-phenyl)-methylene]-2-methyl-3a,7a-dihydro-3H-indolium
Ethanaminium, N-[4-[(3-carboxypyrazinyl)(1-ethyl-2-methyl-1H-indol-3-yl)methylene]-3-ethoxy-2,5-cyclohexadien-1-ylidene]-N-ethyl-, internal salt
Ethanaminium, N-[4-[(3-carboxypyrazinyl)(1-ethyl-2-methyl-1H-indol-3-yl)methylene]-3-ethoxy-2,5-cyclohexadien-1-ylidene]-N-ethyl-, chloride
Cyclohepta[b]furylium, 3-[[4-(dimethylamino)phenyl](2-oxo-2H-cyclohepta[b]furan-3-yl)methylene]-2,3-dihydro-2-oxo
Methanaminium, N-[4-[bis(1,2,3,4,10,14b-hexahydro-
2-methyldibenz[c,f]pyrazino[1,2-a]azepin-8-yl) methylene]-2,5-cyclohexadien-1-ylidene]-N-methyl-, perchlorate, diperchlorate
Methanaminium, N-[4-[bis(1,2,3,4,10,14b-hexahydro-2-methyldibenzo [c,f]pyrazino[1,2-a]azepin-8-yl)methylene]-2,5-cyclohexadien-1-ylidene]-N-methyl-, perchlorate
Methanaminium, N-[4-[bis(1,2,3,4,10,14b-hexahydro-2-methyldibenzo [c,f]pyrazino[1,2-a]azepin-8-yl)methylene]-2,5-cyclohexadien-1-ylidene]-N-methyl-
Methanaminium, N-[4-[bis(2,3-dihydro-1,5-dimethyl-3-oxo-2-phenyl-1H-pyrazol-4-yl)methylene]-2,5-cyclohexadien-1-ylidene]-N-methyl-
Morpholinium, 4-[5-[[4-(dimethylamino)phenyl][5-(4-morpholinyl)-2-thienyl]methylene]-2(5H)-thienylidene]-, perchlorate
Morpholinium, 4-[5-[[4-(dimethylamino)phenyl][5-(4-morpholinyl)-2-thienyl]methylene]-2(5H)-thienylidene]-
1H-Indolizinium, 7-(dimethylamino)-1-[[7-(dimethylamino)-3- (ethoxycarbonyl)-1-indolizinyl][4-(dimethylamino)phenyl]methylene]-3- (ethoxycarbonyl)-, perchlorate
1H-Indolizinium, 7-(dimethylamino)-1-[[7-(dimethylamino)-3- (ethoxycarbonyl)-1-indolizinyl][4-(dimethylamino)phenyl]methylene]-3- (ethoxycarbonyl)-
Cyclohepta[b]pyrrolium, 3-[(1,2-dihydro-2-oxo-1-phenylcyclohepta[b]pyrrol-3-yl)[4-(dimethylamino)phenyl]methylene]-2,3-dihydro-2-oxo-1-phenyl-, hexafluorophosphate
Cyclohepta[b]pyrrolium, 3-[(1,2-dihydro-2-oxo-1-phenylcyclohepta[b]pyrrol-3-yl)[4-(dimethylamino)phenyl]methylene]-2,3-dihydro-2-oxo-1-phenyl-
Quinolinium, 6-[[4-(dimethylamino)phenyl](1,2,3,4-tetrahydro-1,2,2,4-tetramethyl-6-quinolinyl)methylene]-2,3,4,6-tetrahydro-1,2,2,4-tetraméthyl-, chloride
Quinolinium, 6-[(1,2-dihydro-1,2,2,4-tetramethyl-6-quinolinyl)[4-(dimethylamino)phenyl]methylene]-2,6-dihydro-1,2,2,4-tetramethyl-,chloride
3H-Carbazolium, 9-ethyl-3-[(9-ethyl-9H-carbazol-3-yl)[4-(methylphenylamino)phenyl]methylene]-, tetrafluoroborate(1-)
3H-Carbazolium, 3-[[4-(dimethylamino)phenyl](9-ethyl-9H-carbazol-3-yl)methylene]-9-ethyl-, tetrafluoroborate(1-)
3H-Carbazolium, 9-ethyl-3-[(9-ethyl-9H-carbazol-3-yl)[4- (methylphenylamino) phenyl]methylene]-, perchlorate
3H-Carbazolium, 9-ethyl-3-[(9-ethyl-9H-carbazol-3-yl)[4- (methylphenylamino) phenyl]methylene]-, bromide
3H-Carbazolium, 9-ethyl-3-[(9-ethyl-9H-carbazol-3-yl)[4- (methylphenylamino) phenyl]methylene]-, chloride
3H-Carbazolium, 3-[[4-(dimethylamino)phenyl](9-ethyl-9H-carbazol-3-yl)methylene]-9-ethyl-, bromide
3H-Carbazolium, 3-[[4-(dimethylamino)phenyl](9-ethyl-9H-carbazol-3-yl)methylene]-9-ethyl-, chloride
3H-Carbazolium, 9-ethyl-3-[(9-ethyl-9H-carbazol-3-yl)[4- (methylphenylamino) phenyl]methylene]-, iodide
3H-Carbazolium, 3-[[4-(dimethylamino)phenyl](9-ethyl-9H-carbazol-3-yl)methylene]-9-ethyl-, iodide
3H-Carbazolium, 3-[[4-(dimethylamino)phenyl](9-ethyl-9H-carbazol-3-yl)methylene]-9-éthyl-, perchlorate
1H-Pyrazolium, 4-[(2,3-dihydro-1,5-dimethyl-3-oxo-2-phenyl-1H-pyrazol-4-yl)[4-(trimethylammonio) phenyl]methylene]-4,5-dihydro-2,3-dimethyl-5-oxo-1-phenyl-, diperchlorate
1H-Pyrazolium, 4-[(2,3-dihydro-1,5-dimethyl-3-oxo-2-phenyl-1H-pyrazol-4-yl)[4-(trimethylammonio)phenyl]methylene]-4,5-dihydro-2,3-dimethyl-5-oxo-1-phenyl-
5H-Benzo[a]carbazolium, 11-ethyl-5-[(11-ethyl-3-methoxy-8-methyl-11H-benzo[a] carbazol-5-yl)(4-nitrophenyl)methylene]-3-methoxy-8-methyl-, tetrafluoroborate
5H-Benzo[a]carbazolium, 11-ethyl-5-[(11-ethyl-3-methoxy-8-methyl-11H-benzo[a] carbazol-5-yl)(4-nitrophenyl)methylene]-3-methoxy-8-methyl-
5H-Benzo[a]carbazolium, 11-ethyl-5-[(11-ethyl-2-methoxy-8-methyl-11H-benzo[a]carbazol-5-yl)(4-nitrophenyl)methylene]-2-methoxy-8-methyl-, tetrafluoroborate
5H-Benzo[a]carbazolium, 11-ethyl-5-[(11-ethyl-2-methoxy-8-methyl-11H-benzo[a]carbazol-5-yl)(4-nitrophenyl)methylene]-2-methoxy-8-methyl-
5H-Benzo[a]carbazolium, 11-ethyl-5-[(11-ethyl-8-methoxy-11H-benzo[a]carbazol-5-yl)(4-nitrophenyl)methylene]-8-methoxy-,tetrafluoroborate
5H-Benzo[a]carbazolium, 11-ethyl-5-[(11-ethyl-8-methoxy-11H-benzo[a]carbazol-5-yl)(4-nitrophenyl)methylene]-8-methoxy-
5H-Benzo[a]carbazolium, 11-ethyl-5-[(11-ethyl-4-methoxy-11H-benzo[a]carbazol-5-yl)(4-nitrophenyl)methylene]-4-methoxy-,tetrafluoroborate(
5H-Benzo[a]carbazolium, 11-ethyl-5-[(11-ethyl-4-methoxy-11H-benzo[a]carbazol-5-yl)(4-nitrophenyl)methylene]-4-methoxy-
5H-Benzo[a]carbazolium, 11-ethyl-5-[(11-ethyl-3-methoxy-11H-benzo[a]carbazol-5-yl)(4-nitrophenyl)methylene]-3-methoxy-,tetrafluoroborate
5H-Benzo[a]carbazolium, 11-ethyl-5-[(11-ethyl-3-methoxy-11H-benzo[a]carbazol-5-yl)(4-nitrophenyl)methylene]-3-methoxy-
5H-Benzo[a]carbazolium, 11-ethyl-5-[(11-ethyl-2-methoxy-11H-benzo[a]carbazol-5-yl)(4-nitrophenyl)methylene]-2-methoxy-,tetrafluoroborate
5H-Benzo[a]carbazolium, 11-ethyl-5-[(11-ethyl-2-methoxy-11H-benzo[a]carbazol-5-yl)(4-nitrophenyl)methylene]-2-methoxy-
5H-Benzo[a]carbazolium, 5-[(2-chloro-4-nitrophenyl)(11-ethyl-11H-benzo[a]carbazol-5-yl)methylene]-11-ethyl-, salt with trifluoromethanesulfonic acid (1:1)
5H-Benzo[a]carbazolium, 5-[(2-chloro-4-nitrophenyl)(11-ethyl-11H-benzo[a]carbazol-5-yl)methylene]-11-ethyl
5H-Benzo[a]carbazolium, 11-ethyl-5-[(11-ethyl-11H-benzo[a]carbazol-5-yl)(4-nitrophenyl)methylene]-, tetrafluoroborate
5H-Benzo[a]carbazolium, 11-ethyl-5-[(11-ethyl-11H-benzo[a]carbazol-5-yl)(4-nitrophenyl)methylene]-
5H-Benzo[a]carbazolium, 5-[[4-(diethylamino)phenyl](11-ethyl-11H-benzo[a]carbazol-5-yl)methylene]-11-ethyl-, tetrafluoroborate(1-)
5H-Benzo[a]carbazolium, 5-[[4-(diethylamino)phenyl](11-ethyl-11H-benzo[a]carbazol-5-yl)methylene]-11-ethyl-
5H-Benzo[a]carbazolium, 5-[[4-(dimethylamino)phenyl](11-ethyl-11H-benzo[a]carbazol-5-yl)methylene]-11-ethyl-, tetrafluoroborate(1-)
5H-Benzo[a]carbazolium, 5-[[4-(dimethylamino)phenyl](11-ethyl-11H-benzo[a]carbazol-5-yl)methylene]-11-ethyl-
Salt of 1H-Pyrazolium, 4-[(2,3-dihydro-1,5-dimethyl-3-oxo-2-phenyl-1H-pyrazol-4-yl)[4-(dimethylamino)phenyl]methylene]-4,5-dihydro-2,3-dimethyl-5-oxo-1-phenyl-and a monoacid
3H-Carbazolium, 3-[[4-[bis(phenylmethyl)amino]phenyl](9-ethyl-9H-carbazol-3-yl)methylène]-9-ethyl-, iodide
3H-Imidazo[1,2-a]benzimidazolium, 3-[[4-(dimethylamino)phenyl](9-methyl-2-phenyl-
9H-imidazo[1,2-a]benzimidazol-3-yl)methylene]-9-methyl-2-phenyl-, chloride
3H-Imidazo[1,2-a]benzimidazolium, 3-[[4-(dimethylamino)phenyl](2,9-dimethyl-9H-imidazo[1,2-a]benzimidazol-3-yl)methylene]-2,9-dimethyl-,bromide
1H-Pyrazolium, 4-[(2,3-dihydro-1,5-dimethyl-3-oxo-2-phenyl-1H-pyrazol-4-yl)[4-(dipropylamino)phenyl]methylene]-4,5-dihydro-2,3-dimethyl-5-oxo-1-phenyl-
1H-Pyrazolium, 4-[[4-(diethylamino)phenyl](2,3-dihydro-1,5-dimethyl-3-oxo-2-phenyl-1H-pyrazol-4-yl)methylene]-4,5-dihydro-2,3-dimethyl-5-oxo-1-phenyl-
1H-Pyrazolium, 4-[(2,3-dihydro-1,5-dimethyl-3-oxo-2-phenyl-1H-pyrazol-4-yl)(4-nitrophenyl)methylene]-4,5-dihydro-2,3-dimethyl-5-oxo-1-phenyl-
1H-Pyrazolium, 4-[(2,3-dihydro-1,5-dimethyl-3-oxo-2-phenyl-1H-pyrazol-4-yl)[4-(dimethylamino)phenyl]methylene]-4,5-dihydro-2,3-dimethyl-5-oxo-1-phenyl-
1H-Pyrazolium, 4-[(4-aminophenyl)(2,3-dihydro-1,5-dimethyl-3-oxo-2-phenyl-1H-pyrazol-4-yl)methylene]-2,3-dimethyl-5-oxo-1-phenyl
3H-Indolizinium, 3-[[4-(dimethylamino)phenyl](6-ethenyl-1,2-dimethyl-3-indolizinyl)methylene]-6-ethenyl-1,2-dimethyl-
3H-Indolizinium, 3-[p-(dimethylamino)-α-(6-ethyl-1,2-dimethyl-3-indolizinyl)benzylidene]-6-ethyl-1,2-dimethyl-, iodide
3H-Indolizinium, 3-[p-(dimethylamino)-α-(1,2-dimethyl-6-vinyl-3-indolizinyl)benzylidene]-1,2-dimethyl-6-vinyl-, perchlorate
2-Pyrazolinium, 4-(p-amino-α-antipyrinylbenzylidene)-2,3-dimethyl-5-oxo-1-phenyl-, perchlorate
2-Pyrazolinium, 4-(α-antipyrinyl-p-nitrobenzylidene)-2,3-dimethyl-5-oxo-1-phenyl-, perchlorate
2-Pyrazolinium, 4-[α-antipyrinyl-p-(diethylamino)benzylidene]-2,3-dimethyl-5-oxo-1-phenyl-, perchlorate
2-Pyrazolinium, 4-[α-antipyrinyl-p-(dimethylamino)benzylidene]-2,3-dimethyl-5-oxo-1-phenyl-, perchlorate
Methylium, bis[6-(dimethylamino)-3-methyl-1H-indol-2-yl][4-(dimethylamino)phenyl]-, perchlorate
Methylium, bis[6-(dimethylamino)-3-methyl-1H-indol-2-yl][4-(dimethylamino)phenyl]-
Methylium, bis[6-(dimethylamino)-3-methyl-1H-indol-2-yl](4-nitrophenyl)-, perchlorate
Methylium, bis[6-(dimethylamino)-3-methyl-1H-indol-2-yl](4-nitrophenyl)-
Methylium, bis(benzo[b]thien-2-yl)[4-(dimethylamino)phenyl]-, tetrafluoroborate
Methylium, bis(benzo[b]thien-2-yl)[4-(dimethylamino)phenyl]-
Methylium, [4-(dimethylamino)phenyl]bis(1-methyl-1H-pyrrol-2-yl)-, tetrafluoroborate
Methylium, [4-(dimethylamino)phenyl]bis(1-methyl-1H-pyrrol-2-yl)-Diantipyrinyl(p-nitrophenyl)methylium perchlorate
Methylium, bis(9,10-dihydro-9,9,10-trimethyl-3-acridinyl)[4-(dimethylamino)phenyl]-, chloride
Methylium, [4-(dimethylamino)phenyl]bis(10-methyl-10H-phenothiazin-2-yl)-, chloride
3H-Pyrazol-3-one, 4,4'-[[4-(dimethylamino)phenyl]methylene]bis[1,2-dihydro-1,5-dimethyl-2-phenyl-, mono[tetrafluoroborate(1-)]
3H-Pyrazol-3-one, 4,4'-[[4-(dimethylamino)phenyl]methylene]bis[1,2-dihydro-1,5-dimethyl-2-phenyl-, bis[tetrafluoroborate(1-)]
Salt of 3H-Pyrazol-3-one, 4,4'-[[4-(dimethylamino)phenyl]methylene]bis[1,2-dihydro-1,5-dimethyl-2-phenyl-and a diacid
Salt of 3H-Pyrazol-3-one, 4,4'-[[4-(dimethylamino)phenyl]methylene]bis[1,2-dihydro-1,5-dimethyl-2-phenyl-, and a monoacid
Quinolinium, 4,4'-[(4-nitrophenyl)methylene]bis[1-ethyl-, dichloride
Methylium, bis(9-ethyl-9H-carbazol-3-yl)(4-nitrophenyl)-, iodide
Methylium, [4-(dimethylamino)phenyl]bis(9-ethyl-9H-carbazol-3-yl)-, iodide
Methylium, 9H-carbazol-3-yl[4-(dimethylamino)phenyl](6-methyl-9H-carbazol-3-yl)-Quinolizinium, 2,2'-[(4-nitrophenyl)methylene]bis[1,2,3,4-tetrahydro-1-oxo-
Methylium, 9H-carbazol-3-yl[4-(dimethylamino)phenyl](6-methyl-9H-carbazol-3-yl)-, tetrafluoroborate
6,6'-Dichloro-9,9'-dibutyl-3,3'-dicarbazolyl-p-nitrophenylmethyl chloride
Quinolizinium, 2,2'-(p-nitrobenzylidene)bis[1,2,3,4-tétrahydro-1-oxo-, diperchlorate
Benzylium, p-(diethylamino)-α,α-bis(2,3-dimethyl-5-oxo-1-phenyl-3-pyrazolin-4-yl)-
Benzylium, α,α-diantipyrinyl-p-(dimethylamino)-
Benzylium, α,α-bis(2,3-dimethyl-5-oxo-1-phenyl-3-pyrazolin-4-yl)-p-nitro-
Benzylium, p-amino-α,α-di-4-antipyrinyl-.

20. Composition according to any one of claims 18 and 19, **characterised in that** the compound or compounds of formula (I), (Ibis), (Iter), (II) and (IIbis) and/or their additive salt(s) represent from 0.0001 to 10% by weight of the total weight of the dyeing composition, preferably from 0.005 to 10% by weight and, even more preferably, from 0.01 to 6% by weight of the total weight of the dye composition.

21. Composition according to any one of claims 18 to 20, **characterised in that** the composition further contains one or several direct dye(s) different from the compounds of formula (I), (Ibis), (Iter), (II) or (IIbis) .

22. Composition according to claim 21, **characterised in that** the direct dye(s) different from the compounds of formula (I), (Ibis), (Iter), (II) and (IIbis) are chosen from among the neutral, acid or cationic benzenic nitrated direct dyes, the neutral, acid or cationic azoic direct dyes, the neutral, acid or cationic quinonic direct dyes, the azinic direct dyes, the triarylmethanic direct dyes, the indoaminic direct dyes and the natural direct dyes.

23. Composition according to any one of claims 21 and 22, **characterised in that** the direct dye(s) different from the compounds (I), (Ibis), (Iter), (II) and (IIbis), represent from 0.001 to 20% by weight of the total weight of the dye composition.

24. Composition according to any one of claims 20 to 23, **characterised in that** the composition further contains one or several oxidation base(s) and optionally one or several coupler(s).

25. Composition according to claim 24, **characterised in that** the oxidation base or bases are chosen from among the paraphenylenediamines, the bis-phenylalkylenediamines, the para-aminophenols, the ortho-aminophenols, the heterocyclic bases and their addition salts.

26. Composition according to any one of claims 24 and 25, **characterised in that** the coupler(s) are chosen from among the metaphenylenediamine couplers, the meta-aminophenol couplers, the metadiphenol couplers, the naphthalenic couplers, the heterocyclic couplers and their addition salts.

27. Composition according to any one of claims 24 to 26, **characterised in that** the oxidation base or bases and/or the coupler(s) are each present in an amount from 0.001 to 10% by weight, preferably from 0.005 to 6% by weight of the total weight of the dye composition.

## Patentansprüche

1. Verwendung einer Zusammensetzung, die in einem zum Färben geeigneten kosmetischen Medium mindestens einen Farbstoff enthält, der unter den Verbindungen der folgenden Formel (I), (Ibis), (Iter), (II) oder (IIbis) oder deren tautomeren Formen und deren Additionssalzen ausgewählt ist: 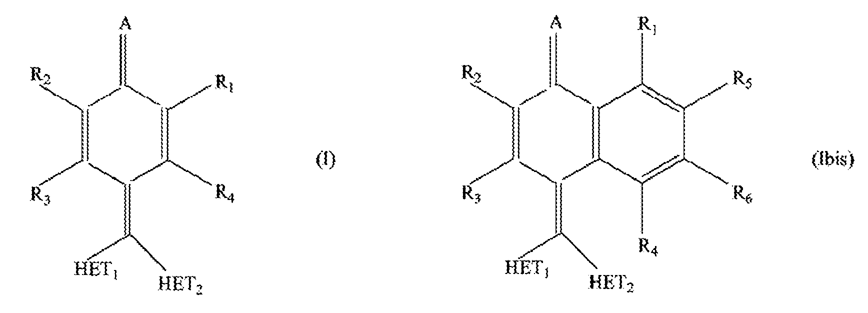 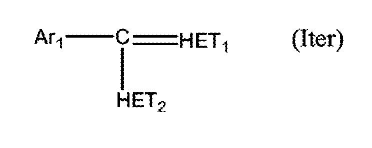 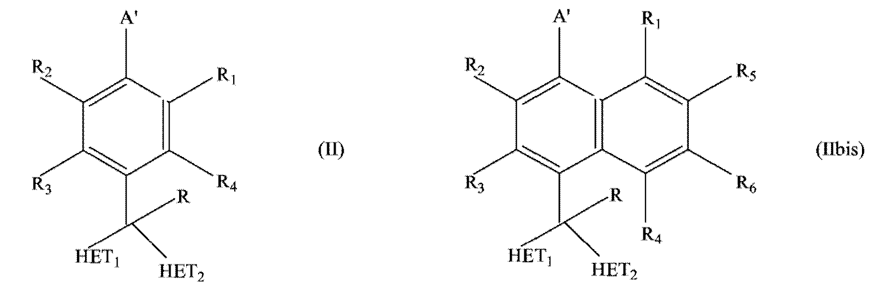 in den Formeln:
- A bedeutet O; NH; N-Alkyl, N-Hydroxyalkyl; Ammonium; N-Alkylammonium; N-(Hydroxyalkyl)ammonium; N,N-Dialkylammonium, N,N-Di(hydroxyalkyl)ammonium, N-(Hydroxyalkyl)-N-(alkyl)-ammonium, bei denen die beiden Alkylgruppen mit dem Stickstoffatom, an das sie gebunden sind, einen Ring bilden können, der durch ein oder mehrer Stickstoffatome, Sauerstoffatome oder Schwefelatome unterbrochen sein kann;
- HET₁ und HET₂ bedeuten unabhängig voneinander einen aromatischen Heterocyclus, der gegebenenfalls mit einer oder mehreren Gruppen Z' substituiert sein kann;
- Ar₁ bedeutet eine Arylgruppe, die gegebenenfalls mit einer oder mehreren Gruppen Z substituiert sein kann;
- R₁ bis R₆ und Z und Z' bedeuten unabhängig voneinander eine Gruppe, die ausgewählt sind unter einem Wasserstoffatom, Halogenatomen, wie F, Cl, Br und I, den Gruppen -NHSO₃H; Hydroxy; Alkyl; Alkoxy; Alkylthio; Amino, Monoalkylamino; Dialkylamino, bei denen die beiden Alkylgruppen gemeinsam mit dem Stickstoffatom, an das sie gebunden sind, einen Ring bilden können, der durch ein oder mehrer Stickstoffatome, Sauerstoffatome oder Schwefelatome unterbrochen sein kann; Heterocyclen und die Gruppen Nitro; Aryl; Acyl; Alkoxycarbonyl; Carboxamido; Cyano; -CO₂H; -SO₃H; -PO₃H₂; -PO₄H₂;
- A' bedeutet ein Wasserstoffatom, Hydroxy, Amino, (Hydroxyalkyl)amino, Di(hydroxyalkyl)amino, (Hydroxyalkyl)(alkyl)amino, Monoalkylamino, Dialkylamino, bei denen die beiden Alkylgruppen gemeinsam mit dem Stickstoffatom, an das sie gebunden sind, einen Ring bilden können, der durch ein oder mehrer Stickstoffatome, Sauerstoffatome oder Schwefelatome unterbrochen sein kann;
- R bedeutet ein Wasserstoffatom oder ein Halogenatom; eine Hydroxygruppe; eine Alkoxygruppe; eine Alkylthiogruppe;
zum Färben von Keratinfasern.

2. Verwendung nach Anspruch 1, bei der die Heterocyclen und insbesondere der HET₁- und der HET₂-Heterocyclus unter den folgenden Heterocyclen ausgewählt sind: Thiophen, Benzothiophen, Furan, Benzofuran, Indol, Indolin, Carbazol, Pyridin, Dehydrochinolein, Chromon, Julolidin, Thiadiazol, Triazol, Isoxazol, Oxazol, Thiazol, Isothiazol, Imidazol, Pyrazol, Triazin, Thiazin, Pyrazin, Pyridazin, Pyrimidin, Pyridin, Diazepin, Oxazepin, Benzotriazol, Benzoxazol, Benzimidazol, Benzothiazol, Morpholin, Piperidin, Piperazin, Azetidin, Pyrrolidin, Aziridin.

3. Verwendung nach Anspruch 1,**dadurch gekennzeichnet, dass** die Zusammensetzung eine Verbindung der Formel (I), (Ibis), (Iter), (II) oder (IIbis) enthält, die unter den folgenden Verbindungen, für die die Gegenionen spezifiziert oder nicht spezifiziert sind, und deren Additionssalzen ausgewählt ist:
4-[Bis-(2-methyl-1H-indol-3-yl)-methylen]-cyclohexa-2,5-dienyliden}-dimethylammonium
1,2,2,4-Tetramethyl-6-[phenyl-(1,2,2,4-tetramethyl-1,2,3,4-tetrahydro-chinolin-6-yl)-methylen]-2,3,4,6-tetrahydrochinolinium-chlorid
1,2,2,3-Tetramethyl-5-[(1-methyl-2-phenyl-1H-indol-3-yl)-phenylmethylen]-3,5-dihydro-2H-indolinum-chlorid 9-Ethyl-3-[(9-ethyl-9H-carbazol-3-yl)-phenyl-methylen]-3H-carbazolium-chlorid
1-Ethyl-3-[(1-ethyl-2-methyl-1H-indol-3-yl)-p-tolyl-methylen]-2-methyl-3a,7a-dihydro-3H-indolium
6-{(4-Nitro-phenyl)-[1-(4-sulfo-butyl)-1,2,3,4-tetrahydro-chinolin-6-yl]-methylen}-1-(4-sulfo-butyl)-2,3,4,6-tetrahydro-chinolinium
6-{(4-Cyano-phenyl)-[1-(4-sulfo-butyl)-1,2,3,4-tetrahydro-chinolin-6-yl]-methylen}-1-(4-sulfo-butyl)-2,3,4,6-tetrahydrochinolinium
Diethyl-{4-[(2-methyl-1H-indol-3-yl)-thiophen-2-yl-methylen]-cyclohexa-2,5-dienyliden}-ammonium
1-Ethyl-3-[(1-ethyl-2-methyl-1H-indol-3-yl)-(4-methoxy-phenyl)-methylen]-2-methyl-3a,7a-dihydro-3H-indolium
N-[4-[(3-Carboxypyrazinyl) (1-ethyl-2-methyl-1H-indol-3-yl)methylen]-3-ethoxy-2,5-cyclohexadien-1-yliden]-N-ethyl-ethanaminium, inneres Salz
N-[4-[(3-Carboxypyrazinyl)(1-ethyl-2-methyl-1H-indol-3-yl)methylen]-3-ethoxy-2,5-cyclohexadien-1-yliden]-N-ethyl-ethanaminium-chlorid
3-[[4-(Dimethylamino)phenyl](2-oxo-2H-cyclohepta[b]furan-3-yl)methylen]-2,3-dihydro-2-oxo-cyclohepta[b]furylium
N-[4-[Bis(1,2,3,4,10,14b-hexahydro-2-methyldibenz[c,f]pyrazino[1,2-a]azepin-8-yl) methylen]-2,5-cyclohexadien-1-yliden]-N-methyl-methanaminium-perchlorat-diperchlorat
N-[4-[Bis(1,2,3,4,10,14b-hexahydro-2-methyldibenzo-[c,f]pyrazino[1,2-a]azepin-8-yl)methylen]-2,5-cyclohexadien-1-yliden]-N-methyl-methanaminium-perchlorat
N-[4-[Bis(1,2,3,4,10,14b-hexahydro-2-methyldibenzo-[c,f]pyrazino[1,2-a]azepin-8-yl)methylen]-2,5-cyclohexadien-1-yliden]-N-methyl-methanaminium
N-[4-[Bis(2,3-dihydro-1,5-dimethyl-3-oxo-2-phenyl-1H-pyrazol-4-yl)methylen]-2,5-cyclohexadien-1-yliden]-N-methyl-methanaminium
4-[5-[[4-(Dimethylamino)phenyl][5-(4-morpholinyl)-2-thienyl]methylen]-2(5H)-thienyliden]-morpholinium-perchlorat
4-[5-[[4-(Dimethylamino)phenyl] [5-(4-morpholinyl)-2-thienyl]methylen]-2(5H)-thienyliden]-morpholinium
7-(Dimethylamino)-1-[[7-(dimethylamino)-3-(ethoxycarbonyl)-1-indolizinyl] [4-(dimethylamino)phenyl]methylen]-3-(ethoxycarbonyl)-1H-indolizinium-perchlorat
7-(Dimethylamino)-1-[[7-(dimethylamino)-3-(ethoxycarbonyl)-1-indolizinyl] [4-(dimethylamino)phenyl]methylen]-3-(ethoxycarbonyl)-1H-indolizinium
3-[(1,2-Dihydro-2-oxo-1-phenylcyclohepta[b]pyrrol-3-yl) [4-(dimethylamino)phenyl]methylen]-2,3-dihydro-2-oxo-1-phenyl-cyclohepta[b]pyrrolium-hexafluorphosphat
3-[(1,2-Dihydro-2-oxo-1-phenylcyclohepta[b]pyrrol-3-yl) [4-(dimethylamino)phenyl]methylen]-2,3-dihydro-2-oxo-1-phenyl-cyclohepta[b]pyrrolium
6-[[4-(Dimethylamino)phenyl](1,2,3,4-tetrahydro-1,2,2,4-tetramethyl-6-chinolinyl)methylen]-2,3,4,6-tetrahydro-1,2,2,4-tetramethyl-chinolinium-chlorid
6-[(1,2-Dihydro-1,2,2,4-tetramethyl-6-chinolinyl) [4-(dimethylamino)phenyl]methylen]-2,6-dihydro-1,2,2,4-tetramethyl-chinolinium-chlorid
9-Ethyl-3-[(9-ethyl-9H-carbazol-3-yl) [4-(methylphenylamino)phenyl]methylen]-3H-carbazolium-tetrafluorborat (1-)
3-[[4-(Dimethylamino)phenyl] (9-ethyl-9H-carbazol-3-yl)methylen]-9-ethyl-3H-carbazolium-tetrafluorborat (1-)
9-Ethyl-3-[(9-ethyl-9H-carbazol-3-yl)[4-(methylphenylamino) phenyl]methylen]-3H-carbazolium-perchlorat
9-Ethyl-3-[(9-ethyl-9H-carbazol-3-yl)[4-(methylphenylamino) phenyl]methylen]-3H-carbazolium-bromid
9-Ethyl-3-[(9-ethyl-9H-carbazol-3-yl)[4-(methylphenylamino) phenyl]methylen]-3H-carbazolium-chlorid
3-[[4-(Dimethylamino)phenyl] (9-ethyl-9H-carbazol-3-yl)methylen]-9-ethyl-3H-carbazolium-bromid
3-[[4-(Dimethylamino)phenyl] (9-ethyl-9H-carbazol-3-yl)methylen]-9-ethyl-3H-carbazolium-chlorid
9-Ethyl-3-[(9-ethyl-9H-carbazol-3-yl) [4-(methylphenylamino) phenyl]methylen]-3H-carbazolium-iodid
3-[[4-(Dimethylamino)phenyl] (9-ethyl-9H-carbazol-3-yl)methylen]-9-ethyl-3H-carbazolium-iodid
3-[[4-(Dimethylamino)phenyl] (9-ethyl-9H-carbazol-3-yl)methylen]-9-ethyl-3H-carbazolium-perchlorat
4-[(2,3-Dihydro-1,5-dimethyl-3-oxo-2-phenyl-1H-pyrazol-4-yl) [4-(trimethylammonio) phenyl]methylen]-4,5-dihydro-2,3-dimethyl-5-oxo-1-phenyl-1H-pyrazolium-diperchlorat
4-[(2,3-Dihydro-1,5-dimethyl-3-oxo-2-phenyl-1H-pyrazol-4-yl) [4-(trimethylammonio)phenyl]methylen]-4,5-dihydro-2,3-dimethyl-5-oxo-1-phenyl-1H-pyrazolium
11-Ethyl-5-[(11-ethyl-3-methoxy-8-methyl-11H-benzo[a]carbazol-5-yl)(4-nitrophenyl)methylen]-3-methoxy-8-methyl-5H-benzo[a]carbazolium-tetrafluorborat
11-Ethyl-5-[(11-ethyl-3-methoxy-8-methyl-11H-benzo[a]carbazol-5-yl)(4-nitrophenyl)methylen]-3-methoxy-8-methyl-5H-benzo[a]carbazolium
11-Ethyl-5-[(11-ethyl-2-methoxy-8-methyl-11H-benzo[a]carbazol-5-yl)(4-nitrophenyl)methylen]-2-methoxy-8-methyl-5H-benzo[a]carbazolium-tetrafluorborat
11-Ethyl-5-[(11-ethyl-2-methoxy-8-methyl-11H-benzo[a]carbazol-5-yl)(4-nitrophenyl)methylen]-2-methoxy-8-methyl-5H-benzo[a]carbazolium
11-Ethyl-5-[(11-ethyl-8-methoxy-11H-benzo[a]carbazol-5-yl)(4-nitrophenyl)methylen]-8-methoxy-5H-benzo[a]carbazolium-tetrafluorborat
11-Ethyl-5-[(11-ethyl-8-methoxy-11H-benzo[a]carbazol-5-yl)(4-nitrophenyl)methylen]-8-methoxy-5H-benzo[a]carbazolium
11-Ethyl-5-[(11-ethyl-4-methoxy-11H-benzo[a]carbazol-5-yl)(4-nitrophenyl)methylen]-4-methoxy-5H-benzo[a]carbazolium-tetrafluorborat
11-Ethyl-5-[(11-ethyl-4-methoxy-11H-benzo[a]carbazol-5-yl)(4-nitrophenyl)methylen]-4-methoxy-5H-benzo[a]carbazolium
11-Ethyl-5-[(11-ethyl-3-methoxy-11H-benzo[a]carbazol-5-yl)(4-nitrophenyl)methylen]-3-methoxy-5H-benzo[a]carbazolium-tetrafluorborat
11-Ethyl-5-[(11-ethyl-3-methoxy-11H-benzo[a]carbazol-5-yl)(4-nitrophenyl)methylen]-3-methoxy-5H-benzo[a]carbazolium
11-Ethyl-5-[(11-ethyl-2-methoxy-11H-benzo[a]carbazol-5-yl) (4-nitrophenyl)methylen]-2-methoxy-5H-benzo[a]carbazolium-tetrafluorborat
11-Ethyl-5-[(11-ethyl-2-methoxy-11H-benzo[a]carbazol-5-yl) (4-nitrophenyl)methylen]-2-methoxy-5H-benzo[a]carbazolium
5-[(2-Chlor-4-nitrophenyl)(11-ethyl-11H-benzo[a]carbazol-5-yl)methylen]-11-ethyl-5H-benzo[a]carbazolium, Salz mit Trifluormethansulfonsäure(1:1)
5-[(2-Chlor-4-nitrophenyl)(11-ethyl-11H-benzo[a]carbazol-5-yl)methylen]-11-ethyl-5H-benzo[a]carbazolium
11-Ethyl-5-[(11-ethyl-11H-benzo[a]carbazol-5-yl)(4-nitrophenyl)methylen]-5H-benzo[a]carbazolium-tetrafluorborat
11-Ethyl-5-[(11-ethyl-11H-benzo[a]carbazol-5-yl)(4-nitrophenyl)-methylen]-5H-benzo[a]carbazolium
5-[[4-(Diethylamino)phenyl](11-ethyl-11H-benzo[a]carbazol-5-yl)methylen]-11-ethyl-5H-benzo[a]carbazolium-tetrafluorborat(1-)
5-[[4-(Diethylamino)phenyl](11-ethyl-11H-benzo[a]carbazol-5-yl)methylen]-11-ethyl-5H-benzo[a]carbazolium
5-[[4-(Dimethylamino)phenyl](11-ethyl-11H-benzo[a]carbazol-5-yl)methylen]-11-ethyl-5H-benzo[a]carbazolium-tetrafluorborat(1-)
5-[[4-(Dimethylamino)phenyl](11-ethyl-11H-benzo[a]carbazol-5-yl)methylen]-11-ethyl-5H-benzo[a]carbazolium
4-[(2,3-Dihydro-1,5-dimethyl-3-oxo-2-phenyl-1H-pyrazol-4-yl)[4-(dimethylamino)phenyl]methylen]-4,5-dihydro-2,3-dimethyl-5-oxo-1-phenyl-; Salz von 1H-Pyrazolium und einer Monosäure
3-[[4-[Bis(phenylmethyl)amino]phenyl] (9-ethyl-9H-carbazol-3-yl)methylen]-9-ethyl-3H-carbazolium-iodid
3-[[4-(Dimethylamino)phenyl](9-methyl-2-phenyl-9H-imidazo[1,2-a]benzimidazol-3-yl)methylen]-9-methyl-2-phenyl-3H-imidazo[1,2-a]benzimidazolium-chlorid
3-[[4-(Dimethylamino)phenyl](2,9-dimethyl-9H-imidazo[1,2-a]benzimidazol-3-yl)methylen]-2,9-dimethyl-3H-imidazo[1,2-a]benzimidazolium-bromid
4-[(2,3-Dihydro-1,5-dimethyl-3-oxo-2-phenyl-1H-pyrazol-4-yl)[4-(dipropylamino)phenyl]methylen]-4,5-dihydro-2,3-dimethyl-5-oxo-1-phenyl-1H-pyrazolium
4-[[4-(Diethylamino)phenyl] (2,3-dihydro-1, 5-dimethyl-3-oxo-2-phenyl-1H-pyrazol-4-yl)methylen]-4,5-dihydro-2,3-dimethyl-5-oxo-1-phenyl-1H-pyrazolium
4-[(2,3-Dihydro-1,5-dimethyl-3-oxo-2-phenyl-1H-pyrazol-4-yl)(4-nitrophenyl)methylen]-4,5-dihydro-2,3-dimethyl-5-oxo-1-phenyl-1H-pyrazolium
4-[(2,3-Dihydro-1,5-dimethyl-3-oxo-2-phenyl-1H-pyrazol-4-yl)[4-(dimethylamino)phenyl]methylen]-4,5-dihydro-2,3-dimethyl-5-oxo-1-phenyl-1H-pyrazolium
4-[(4-Aminophenyl)(2,3-dihydro-1,5-dimethyl-3-oxo-2-phenyl-1H-pyrazol-4-yl)methylen]-2,3-dimethyl-5-oxo-1-phenyl-1H-pyrazolium
3-[[4-(Dimethylamino)phenyl](6-ethenyl-1,2-dimethyl-3-indolizinyl)methylen]-6-ethenyl-1,2-dimethyl-3H-indolizinium
3-[p-(Dimethylamino)-alpha-(6-ethyl-1,2-dimethyl-3-indolizinyl)benzyliden]-6-ethyl-1,2-dimethyl-3H-indolizinium-iodid
3-[p-(Dimethylamino)-alpha-(1,2-dimethyl-6-vinyl-3-indolizinyl)benzyliden]-1,2-dimethyl-6-vinyl-3H-indolizinium-perchlorat
4-(p-Amino-alpha-antipyrinylbenzyliden)-2,3-dimethyl-5-oxo-1-phenyl-2-pyrazolinium-perchlorat
4-(alpha-Antipyrinyl-p-nitrobenzyliden)-2,3-dimethyl-5-oxo-1-phenyl-2-pyrazolinium-perchlorat
4-[alpha-Antipyrinyl-p-(diethylamino)benzyliden]-2,3-dimethyl-5-oxo-1-phenyl-2-pyrazolinium-perchlorat
4-[alpha-Antipyrinyl-p-(dimethylamino)benzyliden]-2,3-dimethyl-5-oxo-1-phenyl-2-pyrazolinium-perchlorat
Bis[6-(dimethylamino)-3-methyl-1H-indol-2-yl] [4-(dimethylamino)phenyl]-methylium-perchlorat
Bis[6-(dimethylamino)-3-methyl-1H-indol-2-yl] [4-(dimethylamino)phenyl]-methylium
Bis[6-(dimethylamino)-3-methyl-1H-indol-2-yl] (4-nitrophenyl)-methylium-perchlorat
Bis[6-(dimethylamino)-3-methyl-1H-indol-2-yl] (4-nitrophenyl)-methylium
Bis(benzo[b]thien-2-yl) [4-(dimethylamino)phenyl]-methylium-tetrafluorborat
Bis(benzo[b]thien-2-yl) [4-(dimethylamino)phenyl]-methylium [4-(Dimethylamino)phenyl]bis(1-methyl-1H-pyrrol-2-yl)-methylium-tetrafluorborat
[4-(Dimethylamino)phenyl]bis(1-methyl-1H-pyrrol-2-yl)-methylium
Diantipyrinyl-(p-nitrophenyl)methylium-perchlorat
Bis(9,10-dihydro-9,9,10-trimethyl-3-acridinyl)[4-(dimethylamino)phenyl]-methylium-chlorid
[4-(Dimethylamino)phenyl]bis(10-methyl-10H-phenothiazin-2-yl)-methylium-chlorid
4,4'-[[4-(Dimethylamino)phenyl]methylen]bis[1,2-dihydro-1,5-dimethyl-2-phenyl-3H-pyrazol-3-on-mono[tetrafluorborat(1-)]
4,4'-[[4-(Dimethylamino)phenyl]methylen]bis[1,2-dihydro-1,5-dimethyl-2-phenyl-3H-pyrazol-3-on-bis[tetrafluorborat(1-)]
4,4'-[[4-(Dimethylamino)phenyl]methylen]bis[1,2-dihydro-1,5-dimethyl-2-phenyl-, Salz von 3H-Pyrazol-3-on und einer Disäure
4,4'-[[4-(Dimethylamino)phenyl]methylen]bis[1,2-dihydro-1,5-dimethyl-2-phenyl-, Salz von 3H-Pyrazol-3-on und einer Monosäure
4,4'-[(4-Nitrophenyl)methylen]bis[1-ethyl-chinolinium-dichlorid Bis(9-Ethyl-9H-carbazol-3-yl)(4-nitrophenyl)-methylium-iodid
[4-(Dimethylamino)phenyl]bis(9-ethyl-9H-carbazol-3-yl-methylium-iodid
9H-carbazol-3-yl-[4-(dimethylamino)phenyl](6-methyl-9H-carbazol-3-yl)-methylium
2,2'-[(4-Nitrophenyl)methylen]bis[1,2,3,4-tetrahydro-1-oxo-chinolizinium
9H-Carbazol-3-yl-[4-(dimethylamino)phenyl](6-methyl-9H-carbazol-3-yl)-methylium-tetrafluorborat
6,6'-Dichlor-9,9'-dibutyl-3,3'-dicarbazolyl-p-nitrophenylmethyl-chlorid
2,2'-(p-Nitrobenzyliden)bis[1,2,3,4-tetrahydro-1-oxo-chinolizinium-diperchlorat
p-(Diethylamino)-alpha,alpha-bis(2,3-dimethyl-5-oxo-1-phenyl-3-pyrazolin-4-yl)-benzylium
alpha,alpha-Diantipyrinyl-p-(dimethylamino)-benzylium
alpha,alpha-Bis (2,3-dimethyl-5-oxo-1-phenyl-3-pyrazolin-4-yl)-p-nitro-benzylium
p-Amino-alpha,alpha-di-4-antipyrinyl-benzylium.

4. Verwendung nach einem der vorhergehenden Ansprüche, **dadurch gekennzeichnet, dass** die Verbindung(en) der Formel (I), (Ibis), (Iter), (II) und (IIbis) und/oder ihr(e) Additionssalz(e) 0,0001 bis 10 Gew.-% des Gesamtgewichts der Farbmittelzusammensetzung, vorzugsweise 0,005 bis 10 Gew.-% und noch bevorzugter 0,01 bis 6 Gew.-% des Gesamtgewichts der Farbmittelzusammensetzung ausmachen.

5. Verwendung nach einem der Ansprüche 1 bis 4, **dadurch gekennzeichnet, dass** die Zusammensetzung ferner einen oder mehrere Direktfarbstoff(e) enthält, der (die) von den Verbindungen der Formel (I), (Ibis), (Iter), (II) und (IIbis) verschieden ist (sind).

6. Verwendung nach Anspruch 5, **dadurch gekennzeichnet, dass** der oder die Direktfarbstoff(e), der (die) von den Verbindungen der Formel (I), (Ibis), (Iter), (II) und (IIbis) verschieden ist (sind), unter den neutralen, sauren oder kationischen, nitrierten Direktfarbstoffen aus der Benzolreihe, neutralen, sauren oder kationischen direktziehenden Azofarbstoffen, neutralen, sauren oder kationischen direktziehenden Chinon-Farbstoffen, direktziehenden Azin-Farbstoffen, direktziehenden Triarylmethan-Farbstoffen, direktziehenden Indoaminfarbstoffen und direktziehenden natürlichen Farbstoffen ausgewählt ist (sind).

7. Verwendung nach einem der Ansprüche 5 und 6, **dadurch gekennzeichnet, dass** der oder die Direktfarbstoff(e), der (die) von den Verbindungen der Formel (I), (Ibis), (Iter), (II) und (IIbis) verschieden ist (sind), 0,001 bis 20 Gew.-% und vorzugsweise 0,005 bis 10 Gew-% des Gesamtgewichts der Farbmittelzusammensetzung ausmachen.

8. Verwendung nach einem der vorhergehenden Ansprüche, **dadurch gekennzeichnet, dass** die Zusammensetzung ferner eine oder mehrere Oxidationsbase(n) und gegebenenfalls einen oder mehrere Kuppler enthält.

9. Verwendung nach Anspruch 8, **dadurch gekennzeichnet, dass** die Oxidationsbase(n) unter den p-Phenylendiaminen, Bisphenylalkylendiaminen, p-Aminophenolen, o-Aminophenolen, den heterocyclischen Basen und den Additionssalzen dieser Verbindungen ausgewählt sind.

10. Verwendung nach einem der Ansprüche 8 und 9, **dadurch gekennzeichnet, dass** der oder die Kuppler unter den m-Phenylendiaminen, m-Aminophenolen, m-Dihydroxybenzolen, Naphthalinkupplern, heterocyclischen Kupplern und den Additionssalzen dieser Verbindungen ausgewählt sind.

11. Verwendung nach einem der Ansprüche 8 bis 10, **dadurch gekennzeichnet, dass** die Oxidationsbase(n) und/oder der oder die Kuppler jeweils in einer Menge von 0,001 bis 10 Gew.-% und vorzugsweise 0,005 bis 6 Gew.-% des Gesamtgewichts der Farbmittelzusammensetzung enthalten sind.

12. Verwendung nach einem der vorhergehenden Ansprüche, **dadurch gekennzeichnet, dass** die Zusammensetzung einen pH-Wert im Bereich von 3 bis 12, insbesondere im Bereich von 5 bis 11 und vorzugsweise im Bereich von 6 bis 8,5 aufweist.

13. Verfahren für die Direktfärbung von Keratinfasern, **dadurch gekennzeichnet, dass** es umfasst, mindestens eine Farbmittelzusammensetzung, wie sie in einem der Ansprüche 1 bis 12 definiert ist, die mindestens eine Verbindung der Formel (I), (Ibis), (Iter), (II) oder (IIbis) enthält, auf die Keratinfasern aufzubringen und dann nach einer Einwirkzeit die Fasern zu spülen.

14. Verfahren nach Anspruch 13, **dadurch gekennzeichnet, dass** die Einwirkzeit 3 bis 50 min, vorzugsweise 5 bis 30 min beträgt.

15. Verfahren für die Färbung von Keratinfasern, bei dem mindestens eine Farbmittelzusammensetzung, wie sie in einem der Ansprüche 1 bis 12 definiert ist, die entweder mindestens eine Verbindung der Formel (II) oder (IIbis) oder mindestens eine Verbindung der Formel (I), (Ibis) oder (Iter) und mindestens eine Oxidationsbase und gegebenenfalls einen oder mehrer Kuppler enthält, auf die Fasern aufgebracht wird, wobei die Farbe bei einem sauren, neutralen oder alkalischen pH-Wert mit Hilfe eines Oxidationsmittels gebildet wird.

16. Verfahren nach Anspruch 15, **dadurch gekennzeichnet, dass** das Oxidationsmittel unter Wasserstoffperoxid, Harnstoffperoxid, Alkalimetallbromaten, Salzen von Persäuren, wie Perboraten und Persulfaten, Persäuren und Oxidasen, wie Peroxidasen, Oxidoreductasen (2 Elektronen), wie Uricasen, und Oxygenasen (4 Elektronen), wie Laccasen, ausgewählt ist.

17. Vorrichtung mit mehreren Abteilungen mit einer ersten Abteilung, die eine Farbmittelzusammensetzung, wie sie in einem der Ansprüche 1 bis 12 definiert ist, die entweder mindestens eine Verbindung der Formel (II) oder (IIbis) oder mindestens eine Verbindung der Formel (I), (Ibis) oder (Iter) und mindestens eine Oxidationsbase und gegebenenfalls einen oder mehrer Kuppler enthält, und mit einer zweiten Abteilung, die eine oxidierende Zusammensetzung enthält.

18. Zusammensetzung zum Färben von Keratinfasern, die in einem zum Färben geeigneten kosmetischen Medium mindestens einen Farbstoff enthält, der unter den Verbindungen der folgenden Formel (I), (Ibis), (Iter), (II) oder (IIbis) oder deren tautomeren Formen und deren Additionssalzen ausgewählt ist: 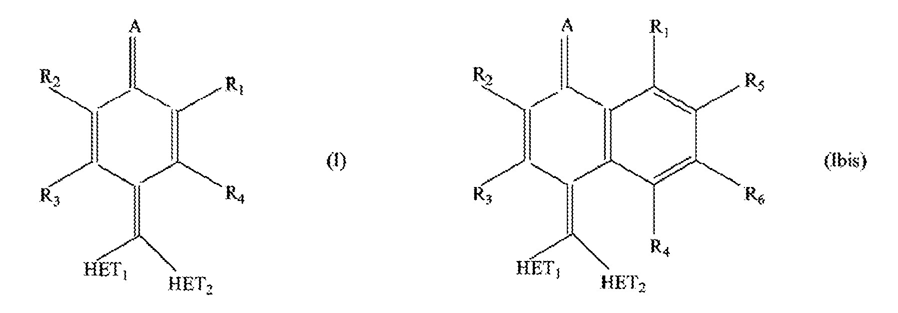 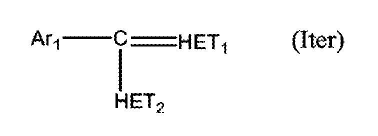 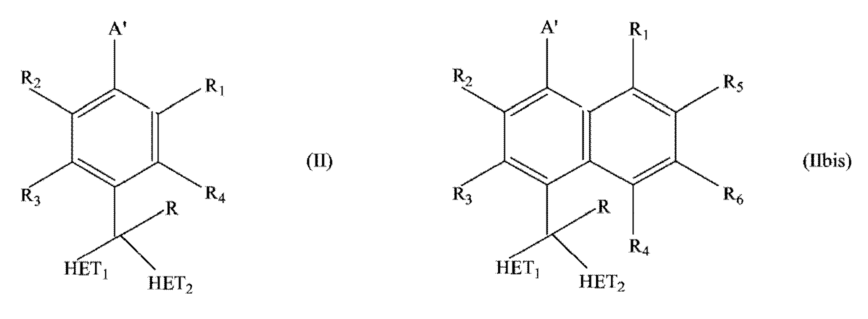 in den Formeln:
- A bedeutet O; NH; N-Alkyl, N-Hydroxyalkyl; Ammonium; N-Alkylammonium; N-(Hydroxyalkyl)ammonium; N,N-Dialkylammonium, N,N-Di(hydroxyalkyl)ammonium, N-(Hydroxyalkyl)-N-(alkyl)-ammonium, bei denen die beiden Alkylgruppen mit dem Stickstoffatom, an das sie gebunden sind, einen Ring bilden können, der durch ein oder mehrer Stickstoffatome, Sauerstoffatome oder Schwefelatome unterbrochen sein kann;
- HET₁ und HET₂ bedeuten unabhängig voneinander einen aromatischen Heterocyclus, der gegebenenfalls mit einer oder mehreren Gruppen Z' substituiert sein kann;
- Ar₁ bedeutet eine Arylgruppe, die gegebenenfalls mit einer oder mehreren Gruppen Z substituiert sein kann;
- R₁ bis R₆ und Z und Z' bedeuten unabhängig voneinander eine Gruppe, die ausgewählt sind unter einem Wasserstoffatom, Halogenatomen, wie F, Cl, Br und I, den Gruppen -NHSO₃H; Hydroxy; Alkyl; Alkoxy; Alkylthio; Amino, Monoalkylamino; Dialkylamino, bei denen die beiden Alkylgruppen gemeinsam mit dem Stickstoffatom, an das sie gebunden sind, einen Ring bilden können, der durch ein oder mehrer Stickstoffatome, Sauerstoffatome oder Schwefelatome unterbrochen sein kann; Heterocyclen und die Gruppen Nitro; Aryl; Acyl; Alkoxycarbonyl; Carboxamido; Cyano; -CO₂H; -SO₃H; -PO₃H₂; -PO₄H₂;
- A' bedeutet ein Wasserstoffatom, Hydroxy, Amino, (Hydroxyalkyl)amino, Di(hydroxyalkyl)amino, (Hydroxyalkyl)(alkyl)amino, Monoalkylamino, Dialkylamino, bei denen die beiden Alkylgruppen gemeinsam mit dem Stickstoffatom, an das sie gebunden sind, einen Ring bilden können, der durch ein oder mehrer Stickstoffatome, Sauerstoffatome oder Schwefelatome unterbrochen sein kann;
- R bedeutet ein Wasserstoffatom oder ein Halogenatom; eine Hydroxygruppe; eine Alkoxygruppe; eine Alkylthiogruppe;
wobei die Zusammensetzung einen pH-Wert von 6 bis 12 aufweist.

19. Zusammensetzung zum Färben nach Anspruch 18, **dadurch gekennzeichnet, dass** sie eine Verbindung der Formel (I), (Ibis), (Iter), (II) oder (IIbis) enthält, die unter den folgenden Verbindungen, für die die Gegenionen spezifiziert oder nicht spezifiziert sind, und deren Additionssalzen ausgewählt ist:
4-[Bis-(2-methyl-1H-indol-3-yl)-methylen]-cyclohexa-2,5-dienyliden}-dimethylammonium
1,2,2,4-Tetramethyl-6-[phenyl-(1,2,2,4-tetramethyl-1,2,3,4-tetrahydro-chinolin-6-yl)-methylen]-2,3,4,6-tetrahydrochinolinium-chlorid
1,2,2,3-Tetramethyl-5-[(1-methyl-2-phenyl-1H-indol-3-yl)-phenylmethylen]-3,5-dihydro-2H-indolinum-chlorid
9-Ethyl-3-[(9-ethyl-9H-carbazol-3-yl)-phenyl-methylen]-3H-carbazolium-chlorid
1-Ethyl-3-[(1-ethyl-2-methyl-1H-indol-3-yl)-p-tolyl-methylen]-2-methyl-3a,7a-dihydro-3H-indolium
6-{(4-Nitro-phenyl)-[1-(4-sulfo-butyl)-1,2,3,4-tetrahydro-chinolin-6-yl]-methylen}-1-(4-sulfo-butyl)-2,3,4,6-tetrahydro-chinolinium
6-{(4-Cyano-phenyl)-[1-(4-sulfo-butyl)-1,2,3,4-tetrahydro-chinolin-6-yl]-methylen}-1-(4-sulfo-butyl)-2,3,4,6-tetrahydrochinolinium
Diethyl-{4-[(2-methyl-1H-indol-3-yl)-thiophen-2-yl-methylen]-cyclohexa-2,5-dienyliden}-ammonium
1-Ethyl-3-[(1-ethyl-2-methyl-1H-indol-3-yl)-(4-methoxy-phenyl)-methylen]-2-methyl-3a,7a-dihydro-3H-indolium
N-[4-[(3-Carboxypyrazinyl)(1-ethyl-2-methyl-1H-indol-3-yl)methylen]-3-ethoxy-2,5-cyclohexadien-1-yliden]-N-ethyl-ethanaminium, inneres Salz
N-[4-[(3-Carboxypyrazinyl)(1-ethyl-2-methyl-1H-indol-3-yl)methylen]-3-ethoxy-2,5-cyclohexadien-1-yliden]-N-ethyl-ethanaminium-chlorid
3-[[4-(Dimethylamino)phenyl](2-oxo-2H-cyclohepta[b]furan-3-yl)methylen]-2,3-dihydro-2-oxo-cyclohepta[b]furylium
N-[4-[Bis(1,2,3,4,10,14b-hexahydro-2-methyldibenz[c,f]pyrazino[1,2-a]azepin-8-yl)methylen]-2,5-cyclohexadien-1-yliden]-N-methyl-methanaminium-perchlorat-diperchlorat
N-[4-[Bis(1,2,3,4,10,14b-hexahydro-2-methyldibenzo-[c,f]pyrazino[1,2-a]azepin-8-yl)methylen]-2,5-cyclohexadien-1-yliden]-N-methyl-methanaminium-perchlorat
N-[4-[Bis(1,2,3,4,10,14b-hexahydro-2-methyldibenzo-[c,f]pyrazino[1,2-a]azepin-8-yl)methylen]-2,5-cyclohexadien-1-yliden]-N-methyl-methanaminium
N-[4-[Bis(2,3-dihydro-1,5-dimethyl-3-oxo-2-phenyl-1H-pyrazol-4-yl)methylen]-2,5-cyclohexadien-1-yliden]-N-methyl-methanaminium
4-[5-[[4-(Dimethylamino)phenyl][5-(4-morpholinyl)-2-thienyl]methylen]-2(5H)-thienyliden]-morpholinium-perchlorat
4-[5-[[4-(Dimethylamino)phenyl][5-(4-morpholinyl)-2-thienyl]methylen]-2(5H)-thienyliden]-morpholinium
7-(Dimethylamino)-1-[[7-(dimethylamino)-3-(ethoxycarbonyl)-1-indolizinyl][4-(dimethylamino)phenyl]methylen]-3-(ethoxycarbonyl)-1H-indolizinium-perchlorat
7-(Dimethylamino)-1-[[7-(dimethylamino)-3-(ethoxycarbonyl)-1-indolizinyl][4-(dimethylamino)phenyl]methylen]-3-(ethoxycarbonyl)-1H-indolizinium
3-[(1,2-Dihydro-2-oxo-1-phenylcyclohepta[b]pyrrol-3-yl)[4-(dimethylamino)phenyl]methylen]-2,3-dihydro-2-oxo-1-phenyl-cyclohepta[b]pyrrolium-hexafluorphosphat
3-[(1,2-Dihydro-2-oxo-1-phenylcyclohepta[b]pyrrol-3-yl)[4-(dimethylamino)phenyl]methylen]-2,3-dihydro-2-oxo-1-phenyl-cyclohepta[b]pyrrolium
6-[[4-(Dimethylamino)phenyl](1,2,3,4-tetrahydro-1,2,2,4-tetramethyl-6-chinolinyl)methylen]-2,3,4,6-tetrahydro-1,2,2,4-tetramethyl-chinolinium-chlorid
6-[(1,2-Dihydro-1,2,2,4-tetramethyl-6-chinolinyl)[4-(dimethylamino)phenyl]methylen]-2,6-dihydro-1,2,2,4-tetramethyl-chinolinium-chlorid
9-Ethyl-3-[(9-ethyl-9H-carbazol-3-yl) [4-(methylphenylamino)phenyl]methylen]-3H-carbazolium-tetrafluorborat (1-)
3-[[4-(Dimethylamino)phenyl](9-ethyl-9H-carbazol-3-yl)methylen]-9-ethyl-3H-carbazolium-tetrafluorborat (1-)
9-Ethyl-3-[(9-ethyl-9H-carbazol-3-yl)[4-(methylphenylamino) phenyl]methylen]-3H-carbazolium-perchlorat
9-Ethyl-3-[(9-ethyl-9H-carbazol-3-yl)[4-(methylphenylamino) phenyl]methylen]-3H-carbazolium-bromid
9-Ethyl-3-[(9-ethyl-9H-carbazol-3-yl)[4-(methylphenylamino) phenyl]methylen]-3H-carbazolium-chlorid
3-[[4-(Dimethylamino)phenyl](9-ethyl-9H-carbazol-3-yl)methylen]-9-ethyl-3H-carbazolium-bromid
3-[[4-(Dimethylamino)phenyl](9-ethyl-9H-carbazol-3-yl) methylen]-9-ethyl-3H-carbazolium-chlorid
9-Ethyl-3-[(9-ethyl-9H-carbazol-3-yl)[4-(methylphenylamino) phenyl]methylen]-3H-carbazolium-iodid
3-[[4-(Dimethylamino)phenyl](9-ethyl-9H-carbazol-3-yl)methylen]-9-ethyl-3H-carbazolium-iodid
3-[[4-(Dimethylamino)phenyl](9-ethyl-9H-carbazol-3-yl)methylen]-9-ethyl-3H-carbazolium-perchlorat
4-[(2,3-Dihydro-1,5-dimethyl-3-oxo-2-phenyl-1H-pyrazol-4-yl)[4-(trimethylammonio) phenyl]methylen]-4,5-dihydro-2,3-dimethyl-5-oxo-1-phenyl-1H-pyrazolium-diperchlorat
4-[(2,3-Dihydro-1,5-dimethyl-3-oxo-2-phenyl-1H-pyrazol-4-yl)[4-(trimethylammonio)phenyl]methylen]-4,5-dihydro-2,3-dimethyl-5-oxo-1-phenyl-1H-pyrazolium
11-Ethyl-5-[(11-ethyl-3-methoxy-8-methyl-11H-benzo[a]carbazol-5-yl)(4-nitrophenyl)methylen]-3-methoxy-8-methyl-5H-benzo[a]carbazolium-tetrafluorborat
11-Ethyl-5-[(11-ethyl-3-methoxy-8-methyl-11H-benzo[a]carbazol-5-yl)(4-nitrophenyl)methylen]-3-methoxy-8-methyl-5H-benzo[a]carbazolium
11-Ethyl-5-[(11-ethyl-2-methoxy-8-methyl-11H-benzo[a]carbazol-5-yl)(4-nitrophenyl)methylen]-2-methoxy-8-methyl-5H-benzo[a]carbazolium-tetrafluorborat
11-Ethyl-5-[(11-ethyl-2-methoxy-8-methyl-11H-benzo[a]carbazol-5-yl)(4-nitrophenyl)methylen]-2-methoxy-8-methyl-5H-benzo[a]carbazolium
11-Ethyl-5-[(11-ethyl-8-methoxy-11H-benzo[a]carbazol-5-yl)(4-nitrophenyl)methylen]-8-methoxy-5H-benzo[a]carbazolium-tetrafluorborat
11-Ethyl-5-[(11-ethyl-8-methoxy-11H-benzo[a]carbazol-5-yl)(4-nitrophenyl)methylen]-8-methoxy-5H-benzo[a]carbazolium
11-Ethyl-5-[(11-ethyl-4-methoxy-11H-benzo[a]carbazol-5-yl)(4-nitrophenyl)methylen]-4-methoxy-5H-benzo[a]carbazolium-tetrafluorborat
11-Ethyl-5-[(11-ethyl-4-methoxy-11H-benzo[a]carbazol-5-yl)(4-nitrophenyl)methylen]-4-methoxy-5H-benzo[a]carbazolium
11-Ethyl-5-[(11-ethyl-3-methoxy-11H-benzo[a]carbazol-5-yl)(4-nitrophenyl)methylen]-3-methoxy-5H-benzo[a]carbazolium-tetrafluorborat
11-Ethyl-5-[(11-ethyl-3-methoxy-11H-benzo[a]carbazol-5-yl)(4-nitrophenyl)methylen]-3-methoxy-5H-benzo[a]carbazolium
11-Ethyl-5-[(11-ethyl-2-methoxy-11H-benzo[a]carbazol-5-yl)(4-nitrophenyl)methylen]-2-methoxy-5H-benzo[a]carbazolium-tetrafluorborat
11-Ethyl-5-[(11-ethyl-2-methoxy-11H-benzo[a]carbazol-5-yl)(4-nitrophenyl)methylen]-2-methoxy-5H-benzo[a]carbazolium
5-[(2-Chlor-4-nitrophenyl)(11-ethyl-11H-benzo[a]carbazol-5-yl)methylen]-11-ethyl-5H-benzo[a]carbazolium, Salz mit Trifluormethansulfonsäure(1:1)
5-[(2-Chlor-4-nitrophenyl)(11-ethyl-11H-benzo[a]carbazol-5-yl)methylen]-11-ethyl-5H-benzo[a]carbazolium
11-Ethyl-5-[(11-ethyl-11H-benzo[a]carbazol-5-yl)(4-nitrophenyl)methylen]-5H-benzo[a]carbazolium-tetrafluorborat
11-Ethyl-5-[(11-ethyl-11H-benzo[a]carbazol-5-yl)(4-nitrophenyl)-methylen]-5H-benzo[a]carbazolium
5-[[4-(Diethylamino)phenyl](11-ethyl-11H-benzo[a]carbazol-5-yl)methylen]-11-ethyl-5H-benzo[a]carbazolium-tetrafluorborat(1-)
5-[[4-(Diethylamino)phenyl](11-ethyl-11H-benzo[a]carbazol-5-yl)methylen]-11-ethyl-5H-benzo[a]carbazolium
5-[[4-(Dimethylamino)phenyl](11-ethyl-11H-benzo[a]carbazol-5-yl)methylen]-11-ethyl-5H-benzo[a]carbazolium-tetrafluorborat(1-)
5-[[4-(Dimethylamino)phenyl](11-ethyl-11H-benzo[a]carbazol-5-yl)methylen]-11-ethyl-5H-benzo[a]carbazolium
4-[(2,3-Dihydro-1,5-dimethyl-3-oxo-2-phenyl-1H-pyrazol-4-yl)[4-(dimethylamino)phenyl]methylen]-4,5-dihydro-2,3-dimethyl-5-oxo-1-phenyl-; Salz von 1H-Pyrazolium und einer Monosäure
3-[[4-[Bis(phenylmethyl)amino]phenyl](9-ethyl-9H-carbazol-3-yl)methylen]-9-ethyl-3H-carbazolium-iodid
3-[[4-(Dimethylamino)phenyl](9-methyl-2-phenyl-9H-imidazo[1,2-a]benzimidazol-3-yl)methylen]-9-methyl-2-phenyl-3H-imidazo[1,2-a]benzimidazolium-chlorid
3-[[4-(Dimethylamino)phenyl](2,9-dimethyl-9H-imidazo[1,2-a]benzimidazol-3-yl)methylen]-2,9-dimethyl-3H-imidazo[1,2-a]benzimidazolium-bromid
4-[(2,3-Dihydro-1,5-dimethyl-3-oxo-2-phenyl-1H-pyrazol-4-yl)[4-(dipropylamino)phenyl]methylen]-4,5-dihydro-2,3-dimethyl-5-oxo-1-phenyl-1H-pyrazolium
4-[[4-(Diethylamino)phenyl](2,3-dihydro-1,5-dimethyl-3-oxo-2-phenyl-11H-pyrazol-4-yl)methylen]-4,5-dihydro-2,3-dimethyl-5-oxo-1-phenyl-1H-pyrazolium
4-[(2,3-Dihydro-1,5-dimethyl-3-oxo-2-phenyl-1H-pyrazol-4-yl)(4-nitrophenyl)methylen]-4,5-dihydro-2,3-dimethyl-5-oxo-1-phenyl-1H-pyrazolium
4-[(2,3-Dihydro-1,5-dimethyl-3-oxo-2-phenyl-1H-pyrazol-4-yl)[4-(dimethylamino)phenyl]methylen]-4,5-dihydro-2,3-dimethyl-5-oxo-1-phenyl-1H-pyrazolium
4-[(4-Aminophenyl)(2,3-dihydro-1,5-dimethyl-3-oxo-2-phenyl-1H-pyrazol-4-yl)methylen]-2,3-dimethyl-5-oxo-1-phenyl-1H-pyrazolium
3-[[4-(Dimethylamino)phenyl](6-ethenyl-1,2-dimethyl-3-indolizinyl)methylen]-6-ethenyl-1,2-dimethyl-3H-indolizinium
3-[p-(Dimethylamino)-alpha-(6-ethyl-1,2-dimethyl-3-indolizinyl)benzyliden]-6-ethyl-1,2-dimethyl-3H-indolizinium-iodid
3-[p-(Dimethylamino)-alpha-(1,2-dimethyl-6-vinyl-3-indolizinyl)benzyliden]-1,2-dimethyl-6-vinyl-3H-indolizinium-perchlorat
4-(p-Amino-alpha-antipyrinylbenzyliden)-2,3-dimethyl-5-oxo-1-phenyl-2-pyrazolinium-perchlorat
4-(alpha-Antipyrinyl-p-nitrobenzyliden)-2,3-dimethyl-5-oxo-1-phenyl-2-pyrazolinium-perchlorat
4-[alpha-Antipyrinyl-p-(diethylamino)benzyliden]-2,3-dimethyl-5-oxo-1-phenyl-2-pyrazolinium-perchlorat
4-[alpha-Antipyrinyl-p-(dimethylamino)benzyliden]-2,3-dimethyl-5-oxo-1-phenyl-2-pyrazolinium-perchlorat
Bis[6-(dimethylamino)-3-methyl-1H-indol-2-yl] [4-(dimethylamino)phenyl]-methylium-perchlorat
Bis[6-(dimethylamino)-3-methyl-1H-indol-2-yl][4-(dimethylamino)phenyl]-methylium
Bis[6-(dimethylamino)-3-methyl-1H-indol-2-yl](4-nitrophenyl)-methylium-perchlorat
Bis[6-(dimethylamino)-3-methyl-1H-indol-2-yl](4-nitrophenyl)-methylium
Bis(benzo[b]thien-2-yl) [4-(dimethylamino)phenyl]-methylium-tetrafluorborat
Bis(benzo[b]thien-2-yl) [4-(dimethylamino)phenyl]-methylium [4-(Dimethylamino)phenyl]bis(1-methyl-1H-pyrrol-2-yl)-methylium-tetrafluorborat
[4-(Dimethylamino)phenyl]bis(1-methyl-1H-pyrrol-2-yl)-methylium
Diantipyrinyl-(p-nitrophenyl)methylium-perchlorat
Bis(9,10-dihydro-9,9,10-trimethyl-3-acridinyl)[4-(dimethylamino)phenyl]-methylium-chlorid
[4-(Dimethylamino)phenyl]bis(10-methyl-10H-phenothiazin-2-yl)-methylium-chlorid
4,4'-[[4-(Dimethylamino)phenyl]methylen]bis[1,2-dihydro-1,5-dimethyl-2-phenyl-3H-pyrazol-3-on-mono[tetrafluorborat(1-)]
4,4'-[[4-(Dimethylamino)phenyl]methylen]bis[1,2-dihydro-1,5-dimethyl-2-phenyl-3H-pyrazol-3-on-bis[tetrafluorborat(1-)]
4,4'-[[4-(Dimethylamino)phenyl]methylen]bis[1,2-dihydro-1,5-dimethyl-2-phenyl-, Salz von 3H-Pyrazol-3-on und einer Disäure
4,4'-[[4-(Dimethylamino)phenyl]methylen]bis[1,2-dihydro-1,5-dimethyl-2-phenyl-, Salz von 3H-Pyrazol-3-on und einer Monosäure
4,4'-[(4-Nitrophenyl)methylen]bis[1-ethyl-chinolinium-dichlorid Bis(9-Ethyl-9H-carbazol-3-yl)(4-nitrophenyl)-methylium-iodid
[4-(Dimethylamino)phenyl]bis(9-ethyl-9H-carbazol-3-yl-methylium-iodid
9H-carbazol-3-yl-[4-(dimethylamino)phenyl](6-methyl-9H-carbazol-3-yl)-methylium
2,2'-[(4-Nitrophenyl)methylen]bis[1,2,3,4-tetrahydro-1-oxo-chinolizinium
9H-Carbazol-3-yl-[4-(dimethylamino)phenyl](6-methyl-9H-carbazol-3-yl)-methylium-tetrafluorborat
6,6'-Dichlor-9,9'-dibutyl-3,3'-dicarbazolyl-p-nitrophenylmethyl-chlorid
2,2'-(p-Nitrobenzyliden)bis[1,2,3,4-tetrahydro-1-oxo-chinolizinium-diperchlorat
p-(Diethylamino)-alpha,alpha-bis(2,3-dimethyl-5-oxo-1-phenyl-3-pyrazolin-4-yl)-benzylium
alpha,alpha-Diantipyrinyl-p-(dimethylamino)-benzylium
alpha,alpha-Bis(2,3-dimethyl-5-oxo-1-phenyl-3-pyrazolin-4-yl)-p-nitro-benzylium
p-Amino-alpha,alpha-di-4-antipyrinyl-benzylium.

20. Zusammensetzung nach einem der vorhergehenden Ansprüche, **dadurch gekennzeichnet, dass** die Verbindung(en) der Formel (I), (Ibis), (Iter), (II) und (IIbis) und/oder ihr(e) Additionssalz(e) 0,0001 bis 10 Gew.-% des Gesamtgewichts der Farbmittelzusammensetzung, vorzugsweise 0,005 bis 10 Gew.-% und noch bevorzugter 0,01 bis 6 Gew.-% des Gesamtgewichts der Farbmittelzusammensetzung ausmachen.

21. Zusammensetzung nach einem der Ansprüche 18 bis 20, **dadurch gekennzeichnet, dass** die Zusammensetzung ferner einen oder mehrere Direktfarbstoff(e) enthält, der (die) von den Verbindungen der Formel (I), (Ibis), (Iter), (II) und (IIbis) verschieden ist (sind).

22. Zusammensetzung nach Anspruch 21, **dadurch gekennzeichnet, dass** der oder die Direktfarbstoff(e), der (die) von den Verbindungen der Formel (I), (Ibis), (Iter), (II) und (IIbis) verschieden ist (sind), unter den neutralen, sauren oder kationischen, nitrierten Direktfarbstoffen aus der Benzolreihe, neutralen, sauren oder kationischen direktziehenden Azofarbstoffen, neutralen, sauren oder kationischen direktziehenden Chinon-Farbstoffen, direktziehenden Azin-Farbstoffen, direktziehenden Triarylmethan-Farbstoffen, direktziehenden Indoaminfarbstoffen und direktziehenden natürlichen Farbstoffen ausgewählt ist (sind).

23. Zusammensetzung nach einem der Ansprüche 21 und 22, **dadurch gekennzeichnet, dass** der oder die Direktfarbstoff(e), der (die) von den Verbindungen der Formel (I), (Ibis), (Iter), (II) und (IIbis) verschieden ist (sind), 0,001 bis 20 Gew.-% und vorzugsweise 0,005 bis 10 Gew-% des Gesamtgewichts der Farbmittelzusammensetzung ausmachen.

24. Zusammensetzung nach einem der Ansprüche 20 bis 23, **dadurch gekennzeichnet, dass** die Zusammensetzung ferner eine oder mehrere Oxidationsbase(n) und gegebenenfalls einen oder mehrere Kuppler enthält.

25. Zusammensetzung nach Anspruch 24, **dadurch gekennzeichnet, dass** die Oxidationsbase(n) unter den p-Phenylendiaminen, Bisphenylalkylendiaminen, p-Aminophenolen, o-Aminophenolen, den heterocyclischen Basen und den Additionssalzen dieser Verbindungen ausgewählt sind.

26. Zusammensetzung nach einem der Ansprüche 24 und 25, **dadurch gekennzeichnet, dass** der oder die Kuppler unter den m-Phenylendiaminen, m-Aminophenolen, m-Dihydroxybenzolen, Naphthalinkupplern, heterocyclischen Kupplern und den Additionssalzen dieser Verbindungen ausgewählt sind.

27. Zusammensetzung nach einem der Ansprüche 24 bis 26, **dadurch gekennzeichnet, dass** die Oxidationsbase(n) und/oder der oder die Kuppler jeweils in einer Menge von 0,001 bis 10 Gew.-% und vorzugsweise 0,005 bis 6 Gew.-% des Gesamtgewichts der Farbmittelzusammensetzung enthalten sind.
